(19) Europäisches Patentamt
European Patent Office
Office européen des brevets

(11) EP 1 198 489 B1

(12) EUROPÄISCHE PATENTSCHRIFT

(45) Veröffentlichungstag und Bekanntmachung des Hinweises auf die Patenterteilung:
**28.04.2004 Patentblatt 2004/18**

(21) Anmeldenummer: **00936907.5**

(22) Anmeldetag: **21.06.2000**

(51) Int Cl.7: **C08G 65/329**, C08G 65/333, A61K 48/00, C12N 15/87, A61K 47/48

(86) Internationale Anmeldenummer:
**PCT/EP2000/005778**

(87) Internationale Veröffentlichungsnummer:
**WO 2001/000708 (04.01.2001 Gazette 2001/01)**

(54) **KOMBINATIONEN ZUR EINFÜHRUNG VON NUCLEINSÄUREN IN ZELLEN**
COMBINATIONS FOR INTRODUCING NUCLEIC ACIDS INTO CELLS
COMBINAISONS POUR L'INTRODUCTION D'ACIDES NUCLEIQUES DANS DES CELLULES

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **25.06.1999 EP 99112260**
**24.11.1999 DE 19956502**

(43) Veröffentlichungstag der Anmeldung:
**24.04.2002 Patentblatt 2002/17**

(73) Patentinhaber:
- **Plank, Christian**
  **82229 Seefeld (DE)**
- **Stemberger, Axel, Dr.**
  **D-85579 Neubiberg (DE)**
- **Scherer, Franz**
  **83661 Lenggries (DE)**

(72) Erfinder:
- **Plank, Christian**
  **82229 Seefeld (DE)**
- **Stemberger, Axel, Dr.**
  **D-85579 Neubiberg (DE)**
- **Scherer, Franz**
  **83661 Lenggries (DE)**

(74) Vertreter: **VOSSIUS & PARTNER**
**Siebertstrasse 4**
**81675 München (DE)**

(56) Entgegenhaltungen:
**WO-A-94/09056**
**WO-A-96/21036**
**WO-A-97/25067**
**WO-A-98/19710**
**US-A- 5 455 027**

- **PATENT ABSTRACTS OF JAPAN vol. 1998, no. 06, 30. April 1998 (1998-04-30) & JP 10 028583 A (HISAMITSU PHARMACEUT CO INC), 3. Februar 1998 (1998-02-03)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung bezieht sich auf das Gebiet des Gentransfers, insbesondere auf Kombinationen von einem Träger und einem Komplex bestehend aus einem Nucleinsäuremolekül und einem Copolymer.

Die Verfügbarkeit stabiler, effizienter Genvektoren ist Voraussetzung für die klinische Umsetzbarkeit gentherapeutischer Strategien. Allerdings sind die meisten bekannten Gentransfer-Vehikel bei der systemischen Anwendung . mit dem Ziel der somatischen Gentherapie noch mit Problemen behaftet.

Für effizienten Gentransfer in vivo sind grundsätzlich folgende zwei Transportprobleme zu lösen: 1) Transfer des zu transferierenden Agens (z.B. Plasmid DNA, Oligonukleotide) von der Applikationsstelle im Organismus zur Zielzelle (der extrazelluläre Aspekt) und 2) Transfer des zu transferierenden Agens von der. Zelloberfläche in das Zytoplasma bzw. den Zellkern (der zelluläre Aspekt). Eine wesentliche Voraussetzung für den rezeptorvermittelten Gentransfer ist die Kompaktierung von DNA zu Partikeln von Virusgröße und Freisetzung der DNA aus internen Vesikeln nach endozytotischer Aufnahme in die Zellen. Diese Voraussetzung wird durch Kompaktierung von DNA mit bestimmten kationischen Polymeren erfüllt, deren chemische Beschaffenheit die Freisetzung von DNA-Komplexen aus internen Vesikeln (Endosomen, Lysosomen) nach endozytotischer Aufnahme in Zellen gewährleistet (Boussif et al., 1995; Ferrari et al., 1997; Haensler & Szoka, 1993; Tang et al., 1996). Solch ein Effekt wird auch durch den Einbau von pH-abhängigen membranzerstörenden Peptiden in DNA-Komplexe erzielt (Plank et al., 1994; WO 93/07283). Bei geeigneter Zusammensetzung der DNA-Komplexe kann eine spezifische Aufnahme und effizienter Gentransfer in Zellen durch Rezeptor-Liganden-Wechselwirkung erzielt werden (Kircheis et al., 1997; Zanta et al., 1997). Besonders geeignet für den rezeptorvermittelten Gentransfer sind auch Komplexe von DNA mit kationischen Peptiden (Gottschalk et al., 1996; Wadhwa et al., 1997; Plank et al., 1999).

Die klinische Umsetzung der vielversprechenden Forschungsergebnisse, die im Gentransfer mit nichtviralen Vektoren erzielt werden, wird unter anderem dadurch erschwert, daß der extrazelluläre Aspekt des Transportproblems nur unvollständig gelöst ist. Eine der Ursachen für dieses Problem ist die chemisch-physikalische Beschaffenheit der nichtviralen Gentransfervektoren, aufgrund derer sie bei systemischer Anwendung starke Wechselwirkungen mit Blut- und Gewebekomponenten eingehen (z.B. durch Opsonisierung, das Anheften von Serumprotein), wodurch insbesondere der auf bestimmte Zielzellen ausgerichtete rezeptorvermittelte Gentransfer eingeschränkt wird. 'Es wurde gezeigt, daß die Oberflächenmodifikation von DNA-Komplexen mit Poly(ethylenglykol) deren Blutproteinbindende Eigenschaften entscheidend vermindert (Plank et al., 1996; Ogris, 1998; WO 98/59064). Eine weitere Einschränkung des Einsatzes nichtviraler Vektoren besteht in der unzureichenden Löslichkeit (bzw. Stabilität) von DNA-Komplexen in vivo. Mit den bekannten Methoden war es bisher nicht möglich, DNA in ausreichend hohen Konzentrationen für intravenöse Anwendungen (z.B. im Bereich von 1 mg/ml) mit einem Polykation zu komplexieren, weil die DNA-Komplexe unter physiologischen Salzkonzentrationen aggregieren und aus der Lösung ausfallen.

Ähnliche Probleme treten auch bei der Applikation niedermolekularer chemischer Verbindungen auf. Auf dem Gebiet der "klassischen" Arzneimittel werden biologisch abbaubare synthetische Polymere dazu verwendet, Pharmazeutika in solch einer Form zu verpacken, die erhöhte Verweildauer im Organismus gewährleistet und zur gewünschten biologischen Verfügbarkeit im Zielorgan führt ("controlled release"). Dafür wird die Oberflächenmodifikation von kolloidalen Partikeln mit Polyethylenglykol derart gestattet, daß die unerwünschte Opsonisierung unterdrückt wird. Über die Synthese und Charakterisierung biologisch abbaubarer Polymere für den Einsatz bei einer Vielzahl medizinischer Anwendungen existiert umfangreiche Literatur (Coombes et al., 1997). Je nach Substanz und Applikation werden die chemischen Bindungen im Polymerrückgrat variiert. Durch geeignete Positionierung von Ester-, Amid-, Peptidoder Urethanbindungen kann die gewünschte Labilität in physiologischem Milieu erreicht werden, wobei die Sensitivität gegenüber den Angriff von Enzymen gezielt variiert wird. Für eine schnelle und effiziente Synthese von biologisch wirksamen Substanzen haben sich kombinatorische Syntheseprinzipien bewährt (Balkenhohl et al., 1996). Durch systematische Variation weniger Parameter gelangt man zu einer großen Anzahl von Verbindungen, welche die gewünschte Grundstruktur aufweisen (Brocchini et al., 1997). Mit einem geeigneten, aussagekräftigen biologischen Selektionssystem können aus diesem Pool von Verbindungen diejenigen herausgesucht werden, welche die gewünschten Eigenschaften zeigen.

In dem US-Patent Nr. 5,455,027 werden Polymere beschrieben, die aus alternierenden Einheiten eines Polyalkylenoxids und eines funktionalisierten Alkans bestehen, wobei an die funktionelle Seitengruppe des Alkans ein pharmokologischer Wirkstoff kovalent gekoppelt ist.

WO 98/19710 beschreibt die Herstellung von synthetischen Polyelektrolytvektoren und Nukleinsäure-Trägervehikeln durch Komplexbildung. Dabei wird ein Nukleinsäure enthaltender Kern-Bestandteil, der durch Selbstorganisation eines kationischen Polymermaterials und Nukleinsäure-Materials gebildet wird, bevorzugt DNA, die in einem Expressionsvektor enthalten ist, direkt oder indirekt mit verschiedenen anderen funktionellen Molekülen oder molekularen Einheiten einschließlich hydrophiler Polymermaterialien versehen, die den Kernbestandteil mit einer Hülle und einer sterischen Abschirmung umgeben. Dadurch werden die Stabilität und Biokompatibilität des Polyelektrolytkomplexes verbessert. Die in WO 98/19710 beschriebenen Vektoren können nützlich in der Gen-Therapie sein. Zur Bindung

an den Kern-Bestandteil enthalten die hydrophilen Polymermaterialien Gruppen mit signifikanter chemischer Reaktivität. WO 98/19710 beschreibt nicht die Möglichkeit der Nutzung physikalischer Wechselwirkungen, wie z.B. elektrostatischer Wechselwirkungen, zum Anbringen einer polymeren hydrophilen Hülle zur Abschirmung und sterischen Stabilisierung des Kern-Bestandteils, der aus Polykation und Nukleinsäure durch Selbstorganisation gebildet wird.

Im Laufe der letzten Jahre haben sich bei der Anwendung nichtviraler Gentransfersysteme folgende wesentliche Erkenntnisse herauskristallisiert:

a) Komplexe aus Plasmid-DNA und kationischen Polymeren eignen sich zum Gentransfer in vitro und in vivo, wobei Komplexen mit Polymeren mit sekundären und tertiären Aminogruppen auch eine inhärente endosomolytische Aktivität zugesprochen werden kann, die zu effizientem Gentransfer führt (Boussif et al., 1995; Tang et al., 1996).

b) Verzweigte kationische Peptide eignen sich ab einer gewissen Kettenlänge des kationischen Anteils zur effizienten Bindung an DNA und zur Bildung von partikulären DNA-Komplexen (Plank et al., 1999).

c) Polykation-DNA-Komplexe gehen starke Wechselwirkungen mit Blutkomponenten ein und aktivieren das Komplementsystem (Plank et al., 1996).

d) Starke Wechselwirkungen von partikulären Strukturen mit Blutkomponenten können durch Modifikation mit Polyethylenglykol vermindert oder verhindert werden; dies gilt auch für Polykation-DNA-Komplexe (Plank et al., 1996; Ogris et al., 1999).

Der vorliegenden Erfindung lag die Aufgabe zugrunde, ein neues, verbessertes nichtvirales Gentransfersystem auf der Grundlage von Nucleinsäure-Polykation-Komplexen bereitzustellen.
Bei der Lösung der im Rahmen der vorliegenden Erfindung zugrundeliegenden Aufgabe wurde von der Überlegung ausgegangen, die Nucleinsäure bzw. Nucleinsäure-Komplexe mit einem geladenen Polymer zu umhüllen, welches die Komplexe physikalisch stabilisiert und sie vor Opsonisierung schützt.

Die vorliegende Erfindung betrifft in einem ersten Aspekt eine Kombination aus einem Träger und einem Komplex enthaltend ein oder mehrere Nucleinsäuremoleküle und ein oder mehrere geladene Copolymere der allgemeinen Formel I

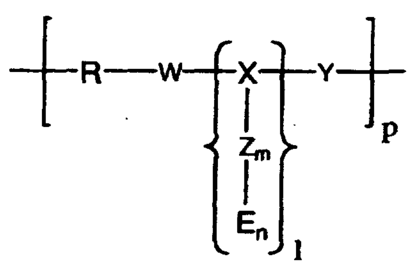

wobei R amphiphiles Polymer oder ein homo- oder hetero-bifunktionelles Derivat davon ist,
und worin X

i) eine Aminosäure oder ein Aminosäurederivat, ein Peptid oder ein Peptidderivat oder ein Spermin- oder Spermidinderivat ist; oder

ii) worin X

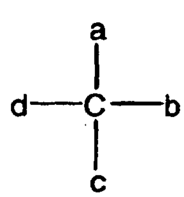

ist, wobei
a H oder, gegebenenfalls halogen- oder diaikylamino-substituiertes, $C_1$-$C_6$-Alkyl bedeutet;

und wobei

b, c und d gleiches oder verschiedenes, gegebenenfalls halogen- oder dialkylamino-substituiertes $C_1$-$C_6$-Alkylen bedeutet; oder

iii) worin X

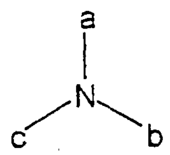

ist, wobei

a H oder, gegebenenfalls halogen- oder dialkylamino-substituiertes, $C_1$-$C_6$-Alkyl bedeutet,

und wobei

b und c gleiches oder verschiedenes, gegebenenfalls halogen- oder dialkylamino-substituiertes $C_1$-$C_6$-Alkylen bedeuten; oder

iv) worin X

eine substituierte aromatische Verbindung mit drei funktionellen Gruppierungen $W_1Y_1Z_1$ ist, wobei W, Y und Z die unten angegebenen Bedeutungen haben;

wobei

W, Y oder Z gleiche oder verschiedene Reste CO, NH, O oder S oder eine zur Reaktion mit SH, OH, NH oder $NH_2$ befähigte Linkergruppierung sind;

und wobei das Effektormolekül E

ein kationisches oder anionisches Peptid oder Peptidderivat oder ein Sperminoder Spermidinderivat oder ein Glykosaminoglycan oder ein nichtpeptidisches Oligo/Poly-kation oder -anion; worin

m und n unabhängig voneinander 0, 1 oder 2 sind; worin

p vorzugsweise 3 bis 20; und worin

I 1 bis 5, vorzugsweise 1 ist.

Für den Fall, daß | >1 ist, kann die Einheit X-$Z_m$-$E_n$ gleich oder verschieden sein.

Aromat bedeutet im Sinne der Erfindung einen monocyclischen oder bicyclischen aromatischen Kohlenwasserstoffrest mit 6 bis 10 Ringatomen, der - neben den eingangs genannten Substituenten - gegebenenfalls unabhängig mit einem oder mehreren weiteren Substituenten, vorzugsweise mit einem, zwei oder drei Substituenten ausgewählt aus der Gruppe $C_1$-$C_6$-Alkyl, -O-( $C_1$-$C_6$-Alkyl), Halogen vorzugsweise Fluor, Chlor oder Brom - Cyano, Nitro, Amino, mono-($C_1$-$C_6$-Alkyl)amino, di-($C_1$-$C_6$-Alkyl)amino substituiert sein kann. Bevorzugt ist der Phenylrest.

Aromat im Sinne der vorliegenden Erfindung kann auch für einen Heterorarylrest, d.h. einen monocyclischen oder bicyclischen aromatischen Kohlenwasserstoffrest mit 5 bis 10 Ringatomen stehen , der ein, zwei oder drei Ringatome ausgewählt aus der Gruppe N, O oder S unabhängig von einander enthält, wobei die restlichen Ringatome C bedeuten.

Alkylamino, bzw. Dialkylamino steht - sofern nicht anders angegeben für eine Aminogruppe, die mit einem oder zwei $C_1$-$C_6$-Alkylresten substituiert ist, wobei - im Fall von zwei Alkylresten - die beiden Alkylgruppen auch einen Ring bilden können. $C_1$-$C_6$-Alkyl steht im allgemeinen - sofern nicht anders angegeben - für einen verzweigten oder unverzweigten Kohlenwasserstoffrest mit 1 bis 6 Kohlenstoffatom(en), der gegebenenfalls mit einem oder mehreren Halogenatom (en) - vorzugsweise Fluor - substituiert sein kann, die untereinander gleich oder verschieden sein können. Als Beispiele seien folgende Kohlenwasserstoffreste genannt:

Methyl, Ethyl, Propyl, 1-Methylethyl (Isopropyl), n-Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2,-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 4 Kohlenstoffatomen, wie Methyl, Ethyl, Propyl, *iso*-Propyl, n-Butyl, 1-Methylpropyl, 2-Methylpropyl oder 1,1-Dimethylethyl.

Entsprechend bedeutet Alkylen eine verzweigte oder unverzweigte zweibindige Kohlenwasserstoffbrücke mit 1 bis 6

Kohlenstoffatomen, die gegebenenfalls mit einem oder mehreren Halogenatom(en) - vorzugsweise Fluor - substituiert sein kann, die untereinander gleich oder verschieden sein können.
Das amphiphile Polymer R ist bevorzugt ein Polyalkylenoxid, Polyvinylpyrollidon, Polyacrylamid, Polyvinylalkohol, oder ein Copolymer dieser Polymere.

Beispiele für geeignete Polyalkylenoxide sind Polyethylenglykole (PEG), Polypropylenglykole, Polyisopropylenglykole, Polylbutylenglykole.

Bevorzugt im Rahmen der vorliegenden Erfindung sind Polyalkylenoxide, insbesondere PEG.

Das Polyalkylenoxid kann im Copolymer als solches vorliegen, oder als Thio-, Carboxyoder Aminoderivat.

Das Polymer R weist bevorzugt ein Molekulargewicht von 500- 10 000 auf, vorzugsweise 1000 - 10 000.

Für den Fall i), in dem X eine Aminosäure ist, kann für die Synthese des Copolymeren eine Aminosäure mit drei funktionellen Gruppen eingesetzt werden, wobei zwei dieser Gruppen zur Copolymerisation mit dem Polymer und eine zur Ankoppelung des Effektorsmoleküls E befähigt sind; in diesem Fall kann Z entfallen. Bevorzugt sind die natürlichen Aminosäuren Glutaminsäure, Asparaginsäure, Lysin, Omithin und Tyrosin. Prinzipiell können statt der natürlichen Aminosäuren auch synthetische verwendet werden (z.B. entsprechende Spermin- und Spermidinderivate).

Im Fall i) kann für die Synthese auch ein Aminosäurederivat verwendet werden, das zwei funktionelle Gruppen zur Copolymerisation mit dem Polymeren enthält und durch Modifikation einer Aminosäure (Glutaminsäure, Asparaginsäure, Lysin oder Omithin) mit einer Linkergruppierung zur Ankoppelung des Effektormoleküls erhalten wurde, womit Z entfällt (m=0); Beispiele für Linkergruppierungen sind Pyridylthiomercaptoalkylcarboxylate (siehe Fig. 1) oder Maleimidoalkancarboxylate.

Im Fall i) kann X auch ein Peptid(derivat) sein. Im Fall eines ungeladenen Peptids oder Peptidderivats ist daran E direkt oder über Z gekoppelt.
Für den Fall, daß X ein positiv oder negativ geladenes Peptid oder Peptidderivat oder ein Spermin- oder Spermidinderivat ist, stellt X selbst das Effektormolekül dar (Z und E entfallen, m=n=0). Im einfachsten Fall besteht das Peptid in diesem Fall aus einer linearen Abfolge aus zwei oder mehreren identischen oder verschiedenen natürlichen oder synthetischen Aminosäuren, wobei die Aminosäuren so gewählt werden, daß das Peptid insgesamt entweder positiv oder negativ geladen ist. Alternativ kann das Peptid verzweigt sein. In diesen Fällen stellt das Peptid als solches den Effektor dar, Z und E entfatten (m = n = 0). Beispiele für einen Typ geeigneter verzweigter kationischer Peptide wurden von Plank et al., 1999, beschrieben.
Geeignete anionische Peptidderivate X weisen die allgemeine Formel (Peptid)$_n$-B-Spacer-(Xaa) auf. Das Peptid ist eine Abfolge von Aminosäuren oder Aminosäurederivaten mit insgesamt negativer Ladung. Bevorzugt besteht das Peptid aus drei bis 30 Aminosäuren, vorzugsweise besteht es ausschließlich aus Glutaminsäure- und/oder Asparaginsäureresten. n stellt die Anzahl der Verzweigungen in Abhängigkeit der in B enthaltenen funktionellen Gruppen dar. B ist ein Verzweigungsmolekül, vorzugsweise Lysin oder ein Molekül des Typs X im Fall von ii) bis iv). Der Spacer ist ein Peptid, bestehend aus 2 bis 10 Aminosäuren oder eine organische Aminocarbonsäure mit 3 bis 9 Kohlenstoffatomen im Carbonsäuregerüst, z.B. 6-Aminohexansäure. Der Spacer dient zur räumlichen Trennung des geladenen Effektormoleküls vom Polymergerüst. Xaa ist bevorzugt eine trifunktionelle Aminosäure, insbesondere Glutaminsäure oder Asparaginsäure und kann im allgemeinen eine Verbindung des Typs X, Fall i) - iv) sein.

Alternativ kann X im Fall i) ein Peptidderivat sein, wobei die Modifikation des Peptids in einer geladenen Gruppierung besteht, die von einer Aminosäure verschieden ist; Beispiele für derartige Gruppierungen sind Sulfonsäuregruppierungen oder geladene Kohlenhydratreste, wie Neuraminsäuren, oder sulfatierte Glycosaminoglycane. Die Modifikation des Peptids kann nach Standardmethoden durchgeführt werden, entweder direkt im Zuge der Peptidsynthese oder, nachträglich am fertigen Peptid.

Das Effektormolekül E kann, wie X im Fall i), ein polykationisches oder polyanionisches Peptid oder Peptidderivat oder ein Spermin- oder Spermidinderivat sein. Im einfachsten Fall stellt das Peptid auch in diesem Fall eine lineare Abfolge aus zwei oder mehreren identischen oder verschiedenen natürlichen oder synthetischen Aminosäuren dar, wobei die Aminosäuren so gewählt werden, daß das Peptid insgesamt entweder positiv oder negativ geladen ist. Alternativ kann das Peptid verzweigt sein. Beispiele für geeignete verzweigte kationische Peptide wurden von Plank et al., 1999, beschrieben. Geeignete anionische Moleküle E weisen die allgemeine Formel (Peptid)$_n$-B-Spacer-(Xbb) auf, wobei Xbb vorzugsweise eine Aminosäure mit einer reaktiven Gruppe ist, welche direkt oder über Z an X koppelbar ist.

Die Koppelung des Effektorpeptids E an Z oder direkt an X erfolgt über eine im Peptid von vornherein vorhandene oder nachträglich eingeführte reaktive Gruppe, z.B. eine Thiolgruppe (in einem Cystein oder durch Einführung einer Mercaptoalkansäuregruppe). Alternativ, in Abhängigkeit von Z, kann die Koppelung auch über bereits vorhandene oder nachträglich eingeführte Amino- oder Carbonsäuregruppen erfolgen.
Altemativ kann E, wie X im Fall i), ein Peptidderivat sein, wobei die Modifikation des Peptids in einer geladenen Gruppierung besteht, die von einer Aminosäure verschieden ist, Beispiele für derartige Gruppierungen sind Sulfonsäuregruppierungen oder geladene Kohlenhydratreste, wie Neuraminsäuren, oder sulfatierte Kohlenhydratreste. Die Koppelung an X erfolgt auch in diesem Fall direkt oder über Z.

Das Copolymer der allgemeinen Formel I

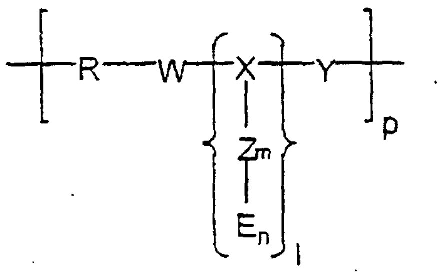

ist bevorzugt als streng alternierendes Block-Copolymer aufgebaut.

Gegebenenfalls ist das Copolymer mit einem zellulären Liganden für die Zielzelle (Rezeptorligand L) modifiziert. In diesem Fall ist die Mehrzahl der Linkerpositionen Z mit E besetzt, dazwischen ist an einzelne Positionen des Linkers Z an Stelle des kationischen bzw. anionischen Effektors E ein zellulärer Ligand gekoppelt. Alternativ liegt der Ligand an einzelne Positionen des Effektormoleküls E gekoppelt vor. Bevorzugt beträgt das Verhältnis E:L ca. 10:1 bis 4:1. Der Rezeptorligand kann biologischen Ursprungs sein (z.B. Transferrin, Antikörper, Kohlenhydratreste) oder synthetisch (z.B. RGD-Peptide, synthetische Peptide, Derivate von synthetischen Peptiden); Beispiele für geeignete Liganden sind in der WO 93/07283 angegeben.

Die erfindungsgemäßen Copolymeren können nach folgendem Verfahrensschema hergestellt werden:

Der Copolymerisationspartner X bzw. $X\text{-}Z_m\text{-}E_n$ wird, sofern er ein Peptid oder, Peptidanalog ist, nach Standardmethoden, z.B. an der festen Phase (solid phase peptide synthesis, SPPS) nach Fmoc-Protokoll synthetisiert (Fields et al., 1990). Die Aktivierung der Aminosäurederivate erfolgt dabei mit TBTU/HOBt oder mit HBTU/HOBt (Fields et al., 1991). Verwendet werden für die ionischen Aminosäurepositionen folgende Derivate in ihrer N-terminal Fmoc-geschützten Form:

(a) kationische Seitenketten: R(Pbf), K(Boc,Trt), Omithin(Boc), Caboxyspermin oder -spermidin (Boc).
(b) anionische Seitenketten: D(O-tert.Bu), E(O-tert.Bu).

Für die Verzweigungsstelle B des Moleküls $(Peptid)_n$-B-Spacer-(Xaa) oder $(Peptid)_n$-B-Spacer-(Xbb) kommt Fmoc-K(Fmoc)-OH zum Einsatz. Die Peptide werden mit TFA/DCM vom Harz gespalten.

Wenn der Polymerisationspartner X ein Peptid der allgemeinen Struktur $(Peptid)_n$-B-Spacer-(Xaa) in der nachfolgenden Copolymerisation ist, so wird an der Position Xaa Glutaminsäure oder Asparaginsäure verwendet, die an einer Carboxyl-Position mit einer Benzylschutzgruppe versehen ist. Diese wird selektiv durch Hydrogenolyse (Felix et al., 1978) entfernt. Die N-terminal gelegenen Aminosäurepositionen der Peptidkette werden mit Boc-geschützten Aminosäuren belegt, um die Schutzgruppenabspaltung nach Copolymerisation des Peptids mit PEG in einem Schritt durchführen zu können.

Ist der Polymerisationspartner X ein Aminosäurederivat, das eine Linkergruppierung enthält (z.B. 3-Mercaptopropionsäure, 6-Aminohexansäure), so kann dieses in Flüssigphase nach klassischen Methoden der Peptidchemie erhalten werden. Mercaptopropionsäure wird mit 2,2'-Dithiodipyridin umgesetzt und chromatographisch gereinigt. Das Reaktionsprodukt wird mit Carboxyl-geschützter Glutaminsäure (O-t.butyl) unter HOBt/EDC-Aktivierung umgesetzt (siehe Fig. 1). Analog wird 6-Fmoc-Aminohexansäure umgesetzt. Die Carboxylschutzgruppen werden in TFA/DCM entfernt, das resultierende Glutaminsäurederivat wird mittels chromatographischer Methoden gereinigt.

Die Herstellung der Copolymeren ist nach folgenden Prinzipien möglich und wird anhand eines PEG-Peptid-Copolymeren veranschaulicht:

**(1) poly(PEG -O-OC-) Matrix ("Polyester"):**

Die Copolymerisation der ionischen, partiell seitenkettengeschützten Peptid-Dicarbonsäuren bzw. Glutamin- oder Asparaginsäurederivate mit PEG-Macromonomeren in definierten Molmassenbereichen (MW 400-20000 in Handel erhältlich, z.B. Fluka) liefert eine Matrix auf PEG-Esterbasis. Diese stellt ein in physiologischer Umgebung hydrolyselabiles System dar (Ulbrich et al., 1985).

Die p(PEG-Peptid)-Copolymere werden dabei nach etablierten Methoden, z.B. mit Dicyclohexylcarbodiimid/DMAP, vorzugsweise in streng alternierender Folge aufgebaut (Zalipsky et al., 1984; Nathan, A., 1992). Das PEG-Macromonomer wird zusammen mit einem seitenkettengeschützten Peptid bzw. Glutamin- oder Asparaginsäurederivat in Dichlormethanlösung mit DCC / DMAP versetzt. Nach Abtrennung des entstandenen Harnstoffderivates

kann das Polymer durch Fällung mit kaltem Äther erhalten werden. Die Abspaltung der noch verbliebenen Seitenkettenschutzgruppen gelingt mit TFA in Dichlormethan (unter diesen Bedingungen ist auch die PEG-Ester-Bindung stabil (Zalipsky et al., 1984)). Das ionische Polymer wird durch Ausfällen und abschließenden Chromatographieschritt erhalten. Die Reaktionsführung ermöglicht eine Kontrolle des Polymerisationsgrades und des Ladungsanteils pro PEG-Einheit im Polymer.

**(2) poly(PEG -HN- OC-) Matrix ("Polyamid")**

Alternativ zum Polyester kann eine amidische Polymermatrix aufgebaut werden, falls, bei Verwendung eines Copolymer-DNA-Komplexes in einer gentherapeutischen Anwendung, eine zu schnelle Hydrolysierbarkeit und daher eine zu hohe Instabilität bei systemischer Applikation zu erwarten ist. in diesem Fall werden Diamino-PEG-Derivaten anstelle der der PEG-Macromonomere verwendet, die in Analogie zur oben beschriebenen Synthese mit den ionischen Peptiden bzw. Glutamin- oder Asparaginsäurederivaten copolymerisiert werden. Bei dieser Synthese wird ein hydrolysestabile's Amidgerüst erhalten. Diaminomodifizierte Polyethylenglykole sind als Grundstoffe in definierten Molmassenbereichen zwischen 500 und 20000 im Handel erhältlich (z.B. Fluka). Die verbliebenen säurelabilen Seitenkettenschutzgruppen der Peptidkomponenten werden, z.B. mit mit TFA/DCM abgespalten, und die Polymere werden mittels chromatographischer Methoden gereinigt.

Copolymere aus Glutamin- oder Asparaginsäurederivaten werden in einem weiteren Schritt mit anionischen oder kationischen Peptiden umgesetzt, die eine geeignete reaktive Gruppe enthalten. Z.B. werden Copolymere der 3-(2'-thio-pyridyl)-mercaptopropionyl-glutaminsäure mit Peptiden umgesetzt, die eine freie Cystein-Thiolgruppe enthalten. Von Copolymeren, die aus 6-Fmoc-aminohexanoylglutaminsäure hervorgegangen sind, wird unter basischen Bedingungen die Fmoc-Schutzgruppe entfernt. Das Produkt wird mit einem carboxylaktivierten, geschützten Peptid umgesetzt. Die Peptidschutzgruppen (t-Boc bzw. O-t.butyl) werden in DCM/TFA entfernt, das Endprodukt wird chromatographisch gereinigt. Alternativ kann die Aminogruppe von Ahx mit bifunktionellen Linkern derivatisiert werden und anschließend mit einem Peptid umgesetzt werden.

Die Kopplung des Liganden L kann direkt durch Aktivierung von Carboxylgruppen am Effektor E (bevorzugt bei anionischen Copolymeren) bzw. am Liganden erfolgen oder über Zwischenschaltung bifunktioneller Linker wie Succinimidyl-pyridyl-dithioproprionat (SPDP; Pierce oder Sigma) und ähnlicher Verbindungen. Die Reinigung des Reaktionsprodukts kann durch Gelfiltration und Ionenaustauschchromatographie durchgeführt werden.

Die Umsetzung dieses Copolymerisationsansatzes kann auch nach kombinatorischen Prinzipien erfolgen. Als selektierbare Variable treten dabei vor allem der Typ und das Molekulargewicht (Polymerisationsgrad) des Polymeren R, die Identität des Polymerisationspartners $X\text{-}Z_m\text{-}E_n$ bzw. des Effektormoleküls E (z.B. eine Serie von anionischen Peptiden mit einer zunehmenden Anzahl von Glutaminsäuren) und der Gesamtpolymerisationsgrad p auf.

Durch Variation von Molmassen der PEG-Macromonomere, der Art der verwendeten ionischen Spezies sowie deren Anteil am Copolymer und des Polymerisationsgrades der Polymermatrix ist ein Mehrparametersystem geschaffen, welches die schnelle Parallelkonstruktion einer homologen Reihe unterschiedlicher Copolymerer und in der Folge, nach Komplexierung mit der Nucleinsäure, verschiedener nichtviraler Vektoren ermöglicht. Das Synthesekonzept wird hierfür im Maßstab einer Zellkulturplatte (z.B. 96 wells per plate) umgesetzt. Dazu wird die chemische Synthese an den benötigten Mikromaßstab angepaßt (Reaktionsvolumina im Bereich von 500 μl). Dies ermöglicht die direkte Übertragung der parallel synthetisierten Polymere in den biologischen Assay und trägt so zu einem schnellen "screening" einer Vielzahl von Systemen und zum schnellen Auffinden geeigneter Verbindungen beitragen. Für die Durchführung des biologischen Auswahlverfahrens im Hinblick auf die bevorzugte Verwendung der erfindungsgemäßen Copolymeren für den Gentransfer werden die Copolymere z.B. mit DNA-Komplexen versetzt und dann Tests unterzogen, die eine Beurteilung der Polymereigenschaften bezüglich der beabsichtigten Anwendung (z.B. Gentransfer) zulassen. Ebensolche Auswahlverfahren können für Copolymer-umhüllte Nanopartikel angewandt werden. Solche Screening- und Selektionsverfahren können z.B. Komplementaktivierungstests im 96-Well-Plate-Format dienen (Plank et al., 1996), turbidimetrische Messungen der durch Serumalbumin oder Salz induzierten Aggregation im gleichen Format oder in vitro Gentransferstudien im gleichen Format (Plank et al., 1999) oder fluoreszenzoptische Verfahren im gleichen Format sein.

Solche Untersuchungen geben z.B. Auskunft darüber, welche Copolymere aus einem kombinatorischen synthetischen Ansatz sich dazu eignen, die Oberfläche von DNA-Komplexen so zu modifizieren, daß deren Löslichkeit ausreichend für Gentransferapplikationen in vivo ist, deren Wechselwirkung mit Blut- und. Gewebekomponenten eingeschränkt wird, sodaß deren Verweil- und Wirkdauer im Blutkreislauf ausreichend erhöht wird, um rezeptorvermittelten Gentransfer in Zielzellen zu ermöglichen.

Die Copolymere werden bevorzugt für den Transport von Nucleinsäuren in höhere eukaryotische Zellen verwendet.

Die vorliegende Erfindung betrifft somit in einem weiteren Aspekt eine Kombination aus einem Träger und Komplexen, enthaltend ein oder mehrere Nucleinsäuremoleküle und ein oder mehrere geladene Copolymere der allgemeinen Formel I.

Vorzugsweise ist das Nucleinsäuremolekül mit einem organischen Polykation oder einem kationischen Lipid kondensiert.

In einem weiteren Aspekt betrifft die Erfindung somit in Kombination mit einem Träger vorliegende Komplexe aus Nucleinsäure und einem organischen Polykation oder einem kationischen Lipid, die dadurch gekennzeichnet sind, daß sie an ihrer Oberfläche ein geladenes Copolymer der allgemeinen Formel I über ionische Wechselwirkung gebunden haben.

Bei den in die Zelle zu transportierenden Nucleinsäuren kann es sich um DNAs oder RNAs handeln, wobei hinsichtlich der Nucleotidsequenz und Größe keine Beschränkungen bestehen. Die in den erfindungsgemäßen Komplexen enthaltene Nucleinsäure wird vor allem durch den in der Zelle zu erzielenden biologischen Effekt definiert, z. B. im Falle der Anwendung im Rahmen der Gentherapie durch das zur Expression zu bringende Gen bzw. den Genabschnitt, oder durch die beabsichtigte Substitution oder Reparatur eines defekten Gens oder einer beliebigen Zielsequenz (Yoon et al., 1996; Kren et al. 1998), oder durch die Zielsequenz eines zu inhibierenden Gens (z.B. im Fall der Anwendung von AntisenseoligoriboNucleotiden oder Ribozymen). Vorzugsweise handelt es sich bei der in die Zelle zu transportierenden Nucleinsäure um Plasmid-DNA, die eine für ein therapeutisch wirksames Protein codierende Sequenz enthält. Für die Anwendung im Rahmen einer Krebstherapie codiert die Sequenz z.B. für ein oder mehrere Zytokine, wie Interleukin-2, IFN-$\alpha$, IFN-$\gamma$ TNF-$\alpha$, oder für ein Selbstmordgen, das in Kombination mit dem Substrat zur Anwendung kommt. Für die Anwendung bei der sog. genetischen Tumorvakzinierung enthalten die Komplexe DNA, codierend für ein oder mehrere Tumorantigene oder Fragmente davon, gegebenenfalls in Kombination mit DNA codierend für ein oder mehrere Zytokine. Weitere Beispiele für therapeutsich wirksame Nucleinsäuren sind in der WO 93/07283 angegeben.

Das erfindungsgemäße Copolymer hat die Eigenschaft, den Nucleinsäure-Polykation-Komplex sterisch zu stabilisieren und dessen unerwünschte Wechselwirkung mit Komponenten von Körperflüssigkeiten (z.B. mit Serumproteinen) zu vermindern oder zu unterdrücken.

Geeignete organische Polykationen für die Komplexierung von Nucleinsäure für den Transport in eukaryotische Zellen sind bekannt; aufgrund ihrer Wechselwirkung mit der negativ geladenen Nucleinsäure wird diese kompaktiert und in eine für die Aufnahme in die Zellen geeignete Form gebracht. Beispiele sind Polykationen, die für den rezeptorvermittelten Gentransfer verwendet wurden (EP 0388 758; WO 93/07283), wie homologe lineare kationische Polyaminosäuren (wie Polylysin, Polyarginin, Polyomithin) oder heterologe lineare gemischt kationisch-neutrale Polyaminosäuren (bestehend aus zwei oder mehreren kationischen und neutralen Aminosäuren), verzweigte und lineare kationische Peptide (Plank et al., 1999; Wadhwa et al. 1997), nichtpeptidische Polykationen (wie lineare oder verzweigte Polyethylenimine, Polypropylenimine), Dendrimere (sphäroidale Polykationen, die mit einem gut definierten Durchmesser und einer exakten Anzahl von endständigen Aminogruppen synthetisiert werden können; (Haensler und Szoka, 1993; Tang et al., 1996; WO 95/02397), kationische Kohlenhydrate, z.B. Chitosan (Erbacher et al. 1998). Die Polykationen können auch mit Lipiden modifiziert sein (Zhou et al. 1994; WO 97/25070).

Weitere geeignete Kationen sind kationische Lipide (Lee et al. 1997), die zum Teil im Handel erhältlich sind (z.B. Lipofectamin, Transfectam).

Im folgenden wird, wenn nicht anders angegeben, der Begriff "Polykation" stellvertretend sowohl für Polykationen als auch für kationische Lipide verwendet.

Im Rahmen der vorliegenden Erfindung bevorzugte Polykationen sind Polyethylenimine, Polylysin und Dendrimere, z. B. Polyamidoamindendrimere ("PAMAM"-Dendrimere).

Die Größe bzw. Ladung der Polykationen kann über einen weiten' Bereich schwanken; sie wird so gewählt, daß der mit Nucleinsäure gebildete Komplex bei physiologischer Salzkonzentration nicht dissoziiert, was durch den Ethidiumbromidverdrängungsassay (Ethidiumbromide Displacement Assay) auf einfache Weise bestimmt werden kann (Plank et al, 1999). In einem weiteren Schritt wird eine definierte Menge an Nucleinsäure mit steigenden Mengen des ausgewählten Polykations inkubiert, der gebildete Komplex auf die zu transfizierenden Zellen aufgebracht und die Genexpression (im allgemeinen mit Hilfe eines Reportergenkonstrukts, z.B. Luciferase) nach Standardmethoden gemessen.

Der Aufbau der Nucleinsäure-Komplexe erfolgt über elektrostatische Wechselwirkungen. Die DNA kann im Verhältnis zum Polykation im Überschuß vorliegen, so daß derartige Komplexe eine negative Oberflächenladung aufweisen; im umgekehrten Fall, wenn das die Nucleinsäure kondensierende Polykation im Überschuß vorliegt, haben die Komplexe eine positive Oberflächenladung. Bevorzugt im Rahmen der vorliegenden Erfindung liegt das Polykation im Überschuß vor.

Bevorzugt wird das Verhältnis Polykation:Nucleinsäure im Falle eines positiven Ladungsüberschusses so eingestellt, daß das Zeta-Potential etwa +20 bis +50 mV beträgt, im Falle der Verwendung bestimmter Polykationen, z.B. Polylysin, kann es auch darüber liegen.

Im Falle eines negativen Ladungsüberschusses beträgt das Zetapotential etwa -50 bis -20mV.

Die Messung des Zeta-Potentials erfolgt nach etablierten Standardmethoden, wie z.B. von Erbacher et al. 1998, beschrieben.
Das Polykation ist gegebenfalls mit einem zellulären Liganden oder Antikörper konjugiert; geeignete Liganden sind in der WO 93/07283 beschrieben. Für den zielzellgerichteten Gentransfer im Rahmen einer Tumortherapie werden Liganden oder Antikörper für tumorzellassoziierte Rezeptoren (z.B. CD87; uPA-R) bevorzugt, die in der Lage sind, den Gentransfer in Tumorzellen zu erhöhen.
Bei der Herstellung der Komplexe wird die Nucleinsäure, im allgemeinen Plasmid-DNA, mit dem Polykation (ggf. derivatisiert mit. einem Rezeptorliganden), das im Ladungsüberschuß vorliegt, inkubiert. Dabei bilden sich Partikel, die über rezeptorvermittelte Endzytose in Zellen aufgenommen werden können. Anschließend werden die Komplexe mit einem erfindungsgemäßen negativ geladenen Copolymer, vorzugsweise Polyethylenglykol-Copolymer, inkubiert. Der Effektor E im Copolymer ist bevorzugt ein polyanionisches Peptid. Alternativ wird zuerst das Copolymer mit Nucleinsäure gemischt und dann mit Polykation inkubiert, oder, als dritte Variante, zuerst mit Polykation gemischt und dann mit Nucleinsäure inkubiert.
Alternativ wird die Nucleinsäure mit einem Polykation; das im elektrostatischen Unterschuß vorliegt, inkubiert und dann ein kationisches Copolymer zugefügt. Auch hier kann die Reihenfolge der Mischungsschritte variiert werden, wie oben für anionische Copolymere beschrieben. Die relativen Anteile der einzelnen Komponenten werden so gewählt, daß der resultierende DNA-Komplex ein schwach positives, neutrales oder schwach negatives Zeta-Potential aufweist (+10 mV bis -10 mV).

Im Fall der Verwendung positiv geladener Copolymerer können diese als alleinige Nucleinsäure-bindende und -kondensierende polykationische Moleküle verwendet werden; der Anteil eines Polykations oder kationischen Lipids kann somit entfallen. Die relativen Anteile der einzelnen Komponenten werden auch in diesem Fall so gewählt, daß der resultierende DNA-Komplex ein schwach positives, neutrales oder schwach negatives Zeta-Potential aufweist (+10 mV bis -10 mV).

In den Komplexen sind gegebenenfalls Polykation und/oder Copolymer mit gleichen oder verschiedenen zellulären Liganden modifiziert.

Die erfindungsgemäßen Nucleinsäure-Komplexe, die durch das elektrostatisch gebundene Copolymer der allgemeinen Formel I in ihrer Größe stabilisiert und somit vor Aggregation geschützt sind, haben den Vorteil, in Lösung über längere Zeiträume (Wochen) lagerbar zu sein. Darüberhinaus haben sie den Vorteil, auf Grund der Schutzwirkung des gebundenen Copolymers eine verminderte bzw. keine Wechselwirkung mit Komponenten von Körperflüssigkeiten (z.B. mit Serumproteinen) einzugehen.

Die Erfindung betrifft in einem weiteren Aspekt eine pharmazeutische Zusammensetzung, enthaltend eine Kombination aus einem Träger und einem Komplex enthaltend eine therapeutisch wirksame Nucleinsäure, das erfindungsgemäße Copolymer und gegebenenfalls ein organisches Polykation oder kationisches Lipid.

Die erfindungsgemäße pharmazeutische Zusammensetzung liegt bevorzugt als Lyophilisat vor, gegebenenfalls unter Zusatz von Zucker, wie Saccharose oder Dextrose, in einer Menge, die in der gebrauchsfertigen Lösung eine physiologische Konzentration ergibt. Die Zusammensetzung kann auch in Form eines Kryokonzentrats vorliegen. Die erfindungsgemäße Zusammensetzung kann auch tiefgefroren (kryokonserviert) oder als gekühlte Lösung vorliegen.

In einem weiteren Aspekt dienen die erfindungsgemäßen Kombinationen mit positiv oder negativ geladenen Copolymeren dazu, kolloidale Partikel ("Nanopartikel"), wie sie für die Applikation klassischer Arzneimittel entwickelt werden, sterisch zu stabilisieren sowie deren unerwünschte Wechselwirkung mit Komponenten von Körperflüssigkeiten (z.B. mit Serumproteinen) zu vermindern oder zu unterdrücken. Weiters können die erfindungsgemäßen mit Rezeptorliganden modifizierten Copolymere dazu verwendet werden, solche Nanopartikel an ihrer Oberfläche mit Rezeptorliganden zu versehen, um Arzneimittel mit erhöhter Spezifität an Zielzellen zu transferieren ("Drug Targeting").

In einem weiteren Aspekt betrifft die vorliegende Erfindung eine Kombination aus einem Träger und einem Komplex enthaltend ein oder mehrere Nucleinsäuremoleküle und ein oder mehrere erfindungsgemäße Copolymere. Für die bevorzugten Ausführungsformen der Copolymere sowie der Nucleinsäuremoleküle gilt dabei das oben Gesagte. Unter einem Träger wird in diesem Zusammenhang ein Körper oder eine Substanz verstanden, der bzw. die mit zu transformierenden Zellen in vivo oder in vitro in Kontakt gebracht werden kann und der bzw. die den Komplex aus Nucleinsäure(n) und Copolymere(n) trägt. Vorzugsweise handelt es sich bei dem Träger um ein kohärentes zusammenhängendes Material, d.h. um einen Feststoff, besonders bevorzugt um einen plastischen oder verformbaren Feststoff, wie z.B. ein Gel, einen Schwamm, eine Folie, ein Pulver, ein Granulat oder eine Faszie. Der Träger kann aus einem biologisch nicht resorbierbaren Material oder auch aus einem biologisch resorbierbaren Material bestehen.
Bei dem Träger kann es sich auch um einen Träger handeln, der hergestellt wird durch die Quervemetzung der erfindungsgemäßen Copolymere, vorzugsweise in Gegenwart von Nucleinsäuremolekülen. So besteht zum Beispiel die Möglichkeit des Einschlusses von bekannten Genvektoren (nackte DNA, nackte RNA, Lipoplexe, Polyplexe), sowie von Oligonucleotiden und Ribozymen, gegebenenfalls chemisch modifiziert, in quervernetze erfindungsgemäße Polymere. Die Quervernetzung erfolgt dabei z.B. in situ in Gegenwart des Genvektors, DNA, Oligonucleotids etc. durch Zugabe eines die Quervenetzung auslösenden Agens in wässrigem oder organischem Lösungsmittel. Die Natur des

quervemetzenden Agens ist abhängig von der Copolymerstruktur. So kann z.B. das in Fig. 2 gezeigte Polymerrückgrat durch Zugabe von Dithiolen wie z.B. Cystein-Cystein oder nicht-aminosäureartigen Dithiolen quervemetzt werden. Quervernetzung von carboxylsäurehaltigen Copolymeren kann durch Zugabe von beliebigen Diaminen unter Carboxylsäureaktivierung erfolgen (z.B. Überführung der Carboxylsäure in einen aktivierten Ester in situ) (Nathan et al., Macromolecules 25 (1992), 4476-4484). Ein Polymerrückgrat mit primären oder sekundären Aminen kann z.B. durch Zugabe einer aktivierten Dicarbonsäure erfolgen. Nach der Quervernetzung kann das Präparat zu einem Film getrocknet werden.

Ein Beispiel für ein biologisch nicht resorbierbares Material ist Silikon (z.B. für Katheter). Möglich ist aber auch die Verwendung von anderen biologisch nicht resorbierbaren Materialien, die als Implantate in den Körper eingebracht werden können und/oder bereits Einsatz gefunden haben, z.B. in der plastischen Chirurgie. Beispiele hierfür sind PTFE (z.B. für Gefäßprothesen), Polyurethan (z.B. für Katheter), metallische Materialien (z.B. medizinische Stähle, Titatlegierung für Endoprothesen; Geflechte aus Metall zur Verwendung als Gefäßstützen ("stents")).

Vorzugsweise ist der Träger ein biologisch resorbierbares Material. Beispiele hierfür sind aus Thrombin oder Fibrinogen hergestellte Fibrinkleber, Chitin, Oxycellulose, Gelatine, Polyethylenglycolcarbonate, aliphatische Polyester, wie z.B. Polymilchsäuren, Polyglycolsäuren und die hiervon abgeleiteten Aminosäureverbindungen, wie Polyamide und Polyurethane oder Polyether sowie die entsprechenden Mischpolymerisate. Ferner kann jedes andere biologisch abbaubare Polymer als Träger verwendet werden, insbesondere sogenannte "self curing adhesives" auf der Basis von Hydrogelen. Insbesondere sind als biologisch resorbierbare Materialien alle Materialien geeignet, die im Körper enzymatisch und/oder durch hydrolytische Prozesse abgebaut werden können. Hierzu zählen auch bioresorbierbares chemisch definiertes Calciumsulfat, Triclaciumphosphat, Hydroxyapatit, Polyanhydride, Träger aus gereinigten Proteinen oder aus teilgereinigter extrazellulärer Matrix. Besonders bevorzugt ist der Träger Kollagen, besonders bevorzugt eine Kollagenmatrix hergestellt aus Knorpel- und Hautkollagen, wie sie z.B. von Sigma oder Collagen Corporation vertrieben wird. Beispiele für die Herstellung einer Kollagenmatrix sind z.B. beschrieben in den US-Patenten 4,394,370 und 4,975,527.

Ganz besonders bevorzugt ist der Träger aus Kollagen und besonders bevorzugt ein Kollagenschwamm. Ganz allgemein binden negativ geladene Polysaccharide wie Glucosaminoglykane über ionische Wechselwirkungen an Kollagen. Die Bindung kann zu positiv geladenen Aminosäuren in den Kollagenfibrillen (Lysin, Hydroxylysin und Arginin) erfolgen oder sogar zu negativ geladenen Aminosäuren, vermittelt durch divalente Kationen wie Calcium. Weiterhin können die ionischen Bindungseigenschaften von Kollagen durch Vorbehandlung mit Säure oder Lauge und anschließendes Gefriertrocknen gezielt beeinflußt werden. Mittels dieser in der Kollagenchemie bekannten Techniken ist es z.B. möglich, Kollagenmaterialien mit Suspensionen erfindungsgemäßer Komplexe zu tränken, um eine ionische Bindung zwischen Kollagen als Trägermaterial und den DNA-Komplexen herzustellen.

Positiv geladene Aminosäuren sind im Kollagen nicht in kurzen kationischen Abschnitten konzentriert. Solche Strukturmerkmale des Trägers sind aber für die effiziente Bindung von DNA erforderlich. Um eine festere Bindung an das Trägermatsrial zu erzielen, kann dieses ferner mit kationischen, DNA-bindenden Substanzen wie Peptiden (Plank et al., Human Gene Therapy 10 (1999), 319-333) oder Polyethylenimin (PEI) derivatisiert werden. Zu diesem Zweck wird der Kollagenschwamm z.B. mit dem bifunktionellen Kopplungsreagenz Succinimidyl-pyridyldithiopropionat (SPDP) modifiziert. Polyethylenimin wird mit Iminothiolan derivatisiert, was zur Einführung von Thiolgruppen führt. Das zu koppelnde kationische Peptid trägt am C-Terminus ein Cystein. Die Thiolgruppen reagieren mit dem SPDP-derivatisierten Kollagenschwamm unter Ausbildung von Disulfidbrücken. Die so gewonnenen Schwamm-Derivate sollten DNA fest binden, und die Freisetzung der DNA ist mit starker zeitlicher Verzögerung zu erwarten.

Zur Herstellung einer erfindungsgemäßen Kombination kann beispielsweise das trockene Kollagenmaterial mit DNA/Copolymer-Komplexen in 5 % Glucose inkubiert werden. Die Schwämme werden dann gefriergetrocknet.

Generell kann eine erfindungsgemäße Kombination hergestellt werden, indem ein entsprechender Träger mit dem Komplex aus Nucleinsäure und Copolymer in Kontakt gebracht wird, so daß der Träger den Komplex aufnimmt bzw. so bindet, daß er ihn wieder freisetzen kann. Entsprechende Methoden sind dem Fachmann bekannt (Bonadio et al. (1999). Nat Med 5(7): 753-759. Shea, L. D. et al. (1999). Nat Biotechnol 17(6): 551-554). In den Beispielen ist beispielhaft die Herstellung einer Kombination aus Kollagenschwamm als Träger und einem Nucleinsäure/Copolymer-Komplex beschrieben.

Die erfindungsgemäßen Kombinationen können für den Transfer von Nucleinsäuren in Zellen, vorzugsweise in Zellen höherer Eukaryonten, bevorzugt von Vertebraten, insbesondere von Säugern, sowohl in vitro als auch in vivo verwendet werden.

Im Zusammenhang mit der in vivo Anwendung kommt es insbesondere in Betracht, daß die Kombination direkt als implantat eingebracht wird, z.B. subcutan oder aber als Beschichtung z.B. auf einem Katheter, Gelenkersatz bzw. einer Endoprothese (z.B. zur Verbesserung der Gewebeintegration). Weitere Verwendungsmöglichkeiten betreffen Wundauflagen, generell die Abdeckung großflächiger Hautdefekte wie z.B. bei Verbrennungen oder Decubitalgeschwüren, sowie als Trägermaterial für die modernen Verfahren des Tissue Engineerings (Mooney, D. J. and Mikos, A. G. (1999). Sci Am 280(4): 60-65). Des weiteren kommt eine Verarbeitung der beschichteten Materialien in Form

von Pulvem in Betracht, die mit gängigen Gewebeklebstoffsystemen gezielt in den Organismus verbracht und dort fixiert werden und als Depotform ihre Wirkung (Transfektion) entfalten.

Ferner betrifft die vorliegende Erfindung auch ein Arzneimittel enthaltend eine erfindungsgemäße Kombination gegebenenfalls in Verbindung mit pharmazeutisch verträglichen Zusatzstoffen.

Gegenstand der Erfindung ist ebenfalls ein Kit enthaltend einen wie oben definierten Träger, sowie ein erfindungsgemäßes Copolymer oder einen Komplex aus einem erfindungsgemäßen Copolymer und einem Nucleinsäuremolekül.

**Fig.1:** Herstellung der Copolymergerüste aus 3-(2'-thio-pyridyl)-mercaptopropionyl-glutaminsäure und O,O'-Bis (2-aminoethyl)poly(ethylenglykol) 6000 oder O,O'-Bis(2-aminoethyl)poly(ethylenglykol) 3400

**Fig. 2:** Ankoppelung von geladenen Peptiden an das Copolymergerüst

**Fig. 3:** Herstellung des Copolymergerüsts aus dem geschützten Peptid $E4E^{PROT}$ und O,O'-Bis(2-aminoethyl)poly (ethylenglykol) 6000

**Fig. 4:** Komplementaktivierungstests

**Fig. 5:** Erythrozyten-Lysetest

**Fig. 6:** Elektronenmikroskopische Aufnahmen von PEI-DNA-Komplexen (N/P = 8) in Gegenwart des Copolymers P3YE5C

**Fig. 7:** Zetapotential von PEI- und DOTAP/Cholesterin-DNA-Komplexen in Abhängigkeit von der Menge zugesetzten Copolymers P3YE5C bzw. P6YE5C

**Fig. 8:** Herstellung DNA/Polykation/Copolymer-Komplexen

**Fig. 9:** Gentransfer in K562-Zellen mit PEI(25 kD)-DNA-Komplexen in Gegenwart und Abwesenheit des Copolymers P3YE5C

**Fig. 10:** Transfektion der Brustkrebszellinie MDA-MB435S mit Polylysin-DNA-Komplexen in Gegenwart und Abwesenheit des Hüllpolymers P3INF7

**Fig. 11:** Lipofection in NIH3T3-Zellen in Gegenwart und Abwesenheit des Copolymers P3YE5C

**Fig. 12:** Transfektion von HepG2-Zellen mit DOTAP/Cholesterin-DNA und PEI-DNA in Gegenwart und Abwesenheit von P6YE5C

**Fig. 13:** Intravenöser Gentransfer in vivo mittels DNA/Polykation-Komplexen mit Copolymerhülle

**Fig. 14:** Freisetzung radioaktiv markierter DNA aus vektorbeladenen Kollagenschwämmen. Die Schwämme wurden wie in Beispiel 17 beschrieben präpariert. Im Falle nackter DNA binden etwa 50 % der aufgebrachten Menge aktiv, während die andere Hälfte sofort freigesetzt wird. Die weitere Freisetzungskinetik folgt in etwa einem linearen Verlauf. Auf Schwämme aufgebrachte Genvektoren sind zu 90 % fest gebunden und werden über einen längeren Zeitraum nach exponentiellem Verlauf freigesetzt. Kationisch derivatisierte Schwämme ("PEI-SPDP" und "Peptid-SPDP") binden nackte DNA effizient und zeigen eine ähnliche Freisetzungskinetik wie vektorbeladene Schwämme.

**Fig. 15:** Gentransfer in NIH-3T3 Mausfibroblasten durch vektorbeladene Kollagenschwämme. Die Kollagenschwämme wurden wie in Beispiel 16 beschrieben präpariert (nackte DNA, PEI-DNA; DOTAP-Cholesterin DNA nach Variante) und wie in Beispiel 18 beschrieben zum Gentransfer eingesetzt. Im Falle von DOTAP-Cholesterin-Schwämmen wurden die Präparate einerseits einer adhärenten Lage von Zellen zugegeben (links), andererseits wurden frisch trypsinierte Zellen auf die Schwämme aufgebracht (rechts). Der weitere Versuchsverlauf war jeweils identisch: Die Reportergenexpression wurde über verschiedene Zeiträume hin untersucht und dauert insbesondere in Zellen, die an/in den Schwämmen wachsen, lange an.

**BEISPIEL 1: Herstellung von geladenen Copolymeren der allgemeinen Formel I**

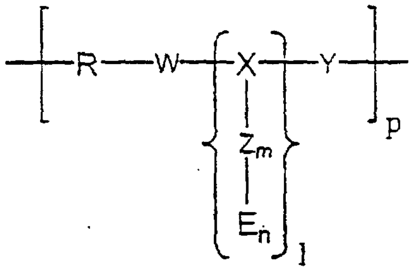

1.1. Herstellung der Copolymergerüste aus 3-(2'-thio-pyridyl)-mercaptopropionylglutaminsäure und O.O'-Bis(2-aminoethyl)poly(ethylenglykol) 6000 oder O.O'-Bis(2-aminoethyl)poly(ethylenglykol) 3400 (Diamino-PEG-3400: Fluka)

In diesem Fall ist in der allgemeinen Formel I:

W=Y=NH;

X=3-mercaptopropionyl-glutaminsäure, das heißt, ein Aminosäurederivat gemäß Fall i), das durch Ankoppelung der Linkergruppierung 3-(2'-Thiopyridyl)-mercaptopropionsäure an Glutaminsäure erhalten wurde; daher entfällt Z(m=0).

a) Umsetzung von 3-Mercaptopropionsäure mit 2,2'-Dithiodipyridin (1):
1 g DTDP (Fluka) wurde in 4 ml absolutem Ethanol (Merck) gelöst. Nach Zugabe von 100 µl Triethylamin (Aldrich) wurden 87 µl (1mmol) 3-Mercaptopropionsäure zugesetzt. Nach 1 h wurde das Reaktionsgemisch in Aliquots mittels RP-HPLC gereinigt: Präparative C18-Säule (Vydac, 218TP1022), Flußrate 25 ml/min, 0.1 % Trifluoressigsäure, 0-40 % Acetonitril in 24 min, 40-100 % Acetonitril in 5 min, 100 % Acetonitril in 5 min. Der Produktpeak eluierte bei ca. 20 % Acetonitril. Die Produktfraktionen wurden vereinigt und gefriergetrocknet.
In einer Abwandlung dieser Synthese wird vor der Reinigung mittels RP-HPLC überschüssiges DTDP durch langsame Zugabe von Wasser unter Rühren ausgefällt. Der Niederschlag wird zweimal in Ethanol aufgenommen und erneut durch Zugabe von Wasser ausgefällt. Die vereinigten wässrigen Phasen werden wie oben mittels RP-HPLC gereinigt.

b) Synthese von 3-(2'-thio-pyridyl)- mercaptopropionyl-glutaminsäure (2b):
Das in a) erhaltene Produkt 1 (s.Fig.1; 0.5 mmol) wurde in 25 ml Dichlormethan gelöst. Unter Rühren und Eiskühlung wurden in einem 50 ml Polypropylenröhrchen (Fa. Peske) der Reihe nach je ein mmol Glutaminsäure-di-t-butylester (Glu(OtBu)OtBu, Bachem), 1-Hydroxybenzotriazol (Aldrich), N-Ethyl-N'-(dimethylaminopropyl)-carbodiimid (Aldrich) und Diisopropylethylamin (Aldrich) zugesetzt. Nach 48 h Reaktion wurde das Gemisch am Rotationsverdampfer bis auf einen öligen Rückstand eingeengt, der in 20 ml Ethylacetat aufgenommen wurde. Diese Lösung wurde je zweimal mit 0.5 M Salzsäure, gesättigter Natriumhydrogencarbonatlösung und gesättigter Natriumchloridlösung ausgeschüttelt. Die organische Phase wurde am Rotationsverdampfer bis auf einen öligen Rückstand eingeengt und über Nacht am Hochvakuum getrocknet (Produkt 2a; s. Fig. 1). Produkt 2a wurde ohne weitere Aufreinigung zur Entfernung der t-Butylschutzgruppen in 30 ml Dichlormethan:Trifluoressigsäure (2:1) aufgenommen und zwei Stunden bei Raumtemperatur gerührt. Am Rotationsverdampfer wurde bis auf einen öligen Rückstand eingeengt, der mit eiskaltem Ether gewaschen wurde. Nach Trocknung am Hochvakuum wurde das Produkt in 100 mM Hepes pH 7.4 gelöst und in Aliquots mittels RP-HPLC (gleiche Bedingungen wie für Produkt 1) gereinigt. Die Produktfraktionen wurden vereinigt. Produkt 2b (s. Fig.1) wurde in einer Ausbeute von 270 µmol erhalten (27 % über alle Stufen). Theoretisches Molekulargewicht: 344.05. Gefunden: 345,0 ($MH^+$).

c1) Copolymerisation von Pyridyl-(2-dithiopropionyl)-glutaminsäure (2b) mit O,O'-Bis(2-aminoethyl)poly(ethylenglykol) 6000 (Diamino-PEG-6000; Fluka)
Produkt 2b wurde in 3 ml Dimethylformamid (Fluka) gelöst und mit Dichlormethan auf 20 ml aufgefüllt. 5 ml

dieser Lösung (67.5 µmol) wurden der Reihe nach mit 506 mg Diamino-PEG-6000 (84 µmol, entspricht 1.25 Äquivalenten; Fluka), 30 mg Dicyclohexylcarbodiimid (135 µmol, 2 Äquivalente, 135 µl einer 1 M Lösung in DMF) und 2 mg Dimethylaminopyridin (0.25 Äquivalente, 1 M Lösung in DMF) versetzt. Nach 2 h wurden 10 µl für einen Ninhydrintest entfernt, der nur noch schwache Blaufärbung zeigte. Rohprodukt 3 (s. Fig. 1) wurde nach Kühlung auf -20°C durch Fällung aus dem Reaktionsgemisch mit t-Butylmethylether unter Rühren erhalten. Das Produkt wurde im Vakuum getrocknet. Aliquots wurden in Wasser aufgenommen, und nach Entfernung von unlöslichem Rückstand durch Filtration (Ultra-Free MC, Millipore) wurde mittels Gelfiltration gereinigt. Dazu wurde eine XK 16/40-Säule (Pharmacia) mit Superdex 75 (Pharmacia) nach den Vorgaben des Herstellers gefüllt. Aliquots von je 20 mg Rohprodukt 3 wurden bei einer Flußrate von 1 ml/min mit 20 mM Hepes pH 7.3 als Eluens gereinigt. Die Hauptfraktion eluierte bei einem scheinbaren Molekulargewicht von 40.000 Da nach höhermolekularen, deutlich abgetrennten Vorfraktionen, die getrennt gesammelt wurden.

c2) Copolymerisation von Pyridyl-(2-dithiopropionyl)-glutaminsäure (2b) mit O,O'-Bis(2-aminoethyl)poly(ethylenglykol) 3400 (Diamino-PEG-3400; Fluka) (Produkt 4; s. Fig.1)
Produkt 4 wurde nach gleichem Ansatz und gleicher Aufreinigung erhalten wie Produkt 3. Als Hauptfraktion (54 % der Produktfraktionen) nach Gelfiltration wurde ein Produkt erhalten, das bei einem scheinbaren Molekulargewicht von 22.800 Da eluierte (Nebenfraktioneh sind ein Produkt mit 64 kD, 14 % der Gesamtmenge, und ein Produkt mit 46 kD, 32 % der Gesamtmenge).
Das Reaktionsschema der Syntheseschritte a) bis c), die das Copolymergerüst ergeben, ist in Fig. 1 dargestellt: 3-Mercaptopropionsäure wird mit 2,2'-Dithiodipyridin umgesetzt, das Produkt (1) wird an carboxylgeschützte Glutaminsäure gekoppelt (Produkt 2a). Nach Abspaltung der t-Butyl-Schutzgruppen erhält man 3-(2'-thio-pyridyl)-mercaptopropionyl-glutaminsäure (2b), die unter DCC-Aktivierung mit O,O'-Bis(2-aminoethyl)poly(ethylenglykol) 6000 oder mit O,O'-Bis(2-aminoethyl)poly(ethylenglykol) 3400 copolymerisiert wird. Erhalten werden die Produkte 3 bzw. 4.

1.2. Peptidsynthese

Die Peptide wurden nach dem FastMoc™-Protokoll auf einem Applied Biosystems 431 A Peptidsynthesizer hergestellt.

i) Peptid YE5C (Sequenz [Ac-YEEEEE]$_2$-ahx-C) wurde unter Verwendung von 330 mg cysteinbeladenem Chlortritylharz (0.5 mmol/g; Bachem) mit den Schutzgruppen Trityl- (Cys), di-Fmoc (Lys) und O-t-Butyl- (Gtu) hergestellt. Es wurde je 1 mmol der geschützten Aminosäuren eingesetzt. Nach dem Verzweigungspunkt (Lys) wurden durchgehend Doppelkopplungen durchgeführt. Die Acetylierung der N-Termini wurde am Peptidharz mit 2 mmol Acetanhydrid in 2 ml N-Methylpyrrolidon in Gegenwart von 2 mmol Diisopropylethylamin durchgeführt. Das Peptid wurde als Rohprodukt nach Spaltung vom Harz (500 µl Wasser, 500 µl Thioanisol, 250 µl Ethandithiol in 10 ml Trifluoressigsäure) und Fällung mit Diethylether gewonnen. Das Rohprodukt wurde in 100 mM HEPES pH 7.9 gelöst und mittels Perfusionschromatographie gereinigt (Poros 20 HQ, Boehringer Mannheim, gefüllt in eine 4 x 100 mm PEEK-Säule. 0 - 0.5 M NaCl in 8 min, Flußrate 10 ml/min). Der Extinktionskoeffizient des Peptids in 50 mM Natriumphosphatpuffer in 6 M Guanidiniumhydrochlorid beträgt bei 280 nm 2560 $M^{-1}cm^{-1}$ (Gill und von Hippel 1989).

ii) Peptid INF7 (Sequenz GLFEAIEGFIENGWEGMIDGWYGC) wurde nach der gleichen Prozedur an 500 mg Chlortritylharz (0.5 mmol/g) synthetisiert, wie für YE5C beschrieben vom Harz gespalten und mit Diethylether gefällt. Das Rohprodukt wurde im Vakuum getrocknet. Aliquots von je 20 mg wurden in 500 µl 1 M. Triethylammoniumhydrogencarbonat-Puffer pH 8 gelöst und durch Gelfiltration gereinigt (Sephadex G-10 von Pharmacia gefüllt in eine HR 10/30-Säule von Pharmacia. Flußrate 1 ml/min. Als Eluent dienten 20 mM HEPES pH 7.3 / 150 mM NaCl oder 100 mM TEAB oder 100 mM Ammoniumhydrogencarbonat). Extinktionskoeffizienten: 278 nm 12600; 279 nm 12665; 280 nm 12660 $M^{-1}cm^{-1}$.

iii) Peptid SFO29-ahx (Sequenz $K_2$K-ahx-C) wurde auf analoge Weise synthetisiert (500 mg Fmoc-Cys(Trt)-Chlortritylharz von Bachem; 0.5 mmol/g) und nach Standardmethoden gereinigt (Sephadex G10 mit 0.1 % TFA als Eluens; Reverse Phase HPLC, 0.1 % TFA - Acetonitril-Gradient). Das Lysin am Verzweigungspunkt war alpha, epsilon-di-Fmoc-L-Lysin, die folgenden Lysine alpha-Fmoc-epsilon-Boc-L-Lysin.

iv) Peptid E4E (Sequenz [EEEE]$_2$KGGE) wurde analog synthetisiert. Ansatzgröße 0.25 mmol Fmoc-Glu(OBzl)-Chlortritylharz. Die Belegung des Harzes erfolgte durch Suspendieren entsprechender Mengen O-Chlorotritylchloridharz (Alexis) in absolutiertem Dichlormethan und Versetzen mit je 2 eq. FmocGlu(OBzI)OH und Diisopropylethylamin. Nach mehrstündigem Schütteln wurde abfiltriert und mehrfach mit Dimethylformamid, Methanol, Isopropanol, Dichlormethan und Diethylether gewaschen. Zum Einsatz kommt ein modifiziertes Fmoc-Protokoll. Die N-terminale Aminosäure trägt eine Boc-Schutzgruppe, um aus der Festphasensynthese ein vollgeschütztes basenstabiles Peptidderivat der Sequenz $(E(Boc)[E(tBu)]_3)_2KGGE(OBzl)OH$ ($E4E^{PROT}$) zu erhalten.
Die Abspaltung vom Harz erfolgte mit Dichlormethan / Essigsäure / Trifluorethanol 8:1:1 bei Raumtemperatur. Die Benzylester-Schutzgruppe der C-terminalen Glutaminsäure wurde selektiv mit $H_2$ / Palladium auf Aktivkohle nach Standardvorschrift abgespalten.
Die Peptidmassen wurden mittels Elektrospray Massenspektroskopie bestimmt und.damit die Identität der Peptide bestätigt.

1.3. Ankoppelung der Peptide an das Copolymergerüst (4) bzw. (5)

Die Lösungen von je 1,2 Äquivalenten C-terminal cysteinhaltigem Peptid und Copolymergerüst, erhalten in 1.1 (bezogen auf die Thiopyridyl-Gruppen am Polymer), in 20 mM Hepes, pH 7.4 werden vereinigt und 15 Stunden bei Raumtemperatur gerührt oder geschüttelt.
Zur Bestimmung der einzusetzenden Äquivalente werden die verfügbaren Thiopyridyl-Bindungsstellen durch Versetzen einer verdünnten Lösung des Polymers mit 2-Mercaptoethanol und anschließender Messung der Extinktion des freigesetzten 2-Thiopyridons bei einer Wellenlänge von 342 nm, die Konzentration der freien Thiolfunktionen des cysteinhaltigen Peptides mit Ellman-Reagenz bei einer Wellenlänge von 412 nm nach Lambert-Beer bestimmt.
Nach Ablauf der Reaktion, deren Vollständigkeit durch die Absorption des freigesetzten Thiopyridons bei 342 nm bestimmt wurde, wurde eingeengt und das Produkt mittels Gelfiltration (Superdex 75-Material, Pharmacia) fraktioniert.

1.3.1 Herstellung des Copolymer P3YE5C

Es wurde das verzweigte Peptid YE5C, Sequenz $(YEEEEE)_2K(ahx)C$, verwendet, das über eine Disulfidbrükke des Cystein-Thiols mit der 3-Mercaptopropionyl-glutaminsäure-Gruppierung verknüpft ist.

a) Copolymer P3YE5C wurde aus Fraktion 3 (22.800 Da) des Produktes (4) und gereinigtem Peptid hergestellt. Als Produkt wurde eine Verbindung mit einem scheinbaren Molekulargewicht von 35.000 Da erhalten. Unter Berücksichtigung des Molekulargewichts des Peptides und des Ausgangspolymers bedeutet dies einen Polymerisationsgrad p = 6 (6 sich wiederholende Einheiten).
b) Copolymer P6YE5C wurde aus Fraktion 3 (40.200 Da) des Produktes (3) und gereinigtem Peptid hergestellt. Als Produkt wurde eine Verbindung mit scheinbarem Molekulargewicht 55.800 Da erhalten. Der Polymerisationsgrad ist ca. 7.

1.3.2 Herstellung des Copolymer P3INF7

Es wurde das endosomolytische Peptid INF7 verwendet, das über eine Disulfidbrücke des Cystein-Thiols mit der 3-Mercapto-propionyl-glutaminsäure-Gruppierung verknüpft ist.

a) Copolymer P3INF7 wurde aus Fraktion 3 (22.800 Da) des Produktes (4) und gereinigtem Influenzapeptid hergestellt.
b) Copolymer P6INF7 wurde aus Fraktion 3 (40.200 Da) des Produktes (3) und gereinigtem Influenzapeptid INF7 hergestellt.

1.3.3 Herstellung eines Rezeptorligand-modifizierten ("lactosylierten") Copolymer

Es wurden ein Teil des lactosylierten Pepitds SFO29-ahx und 9 Teile des verzweigten Peptids YE5C, die über eine Disulfidbrücke des Cystein-Thiols mit der 3-Mercaptopropionyl-glutaminsäure-Gruppierung verknüpft sind, verwendet. Jeweils 3.32 µmol Copolymer (4) bzw (5) (bezogen auf die enthaltenen Thiopyridyl-Gruppen) gelöst in 1 ml 20 mM HEPES pH 7.4 wurden mit einer Mischung von 500 nmol lactosyliertem SFO29-ahx und 4,48 µmol Peptid YE5C in 1.1 ml HEPES-Puffer Versetzt. Dies entspricht einem 1.5-fachen Überschuß an freien Thiolgruppen der Peptide über die zu Verfügung stehenden Thiopyridylgruppen, und der Anteil des lactosylierten Peptids an Gesamtpeptid ist 10 %. Die Reaktion verlief über Nacht vollständig. Die Produkte wurden wie beschrieben mittels Gelfiltration (Superdex 75) gereinigt.
Das Reaktionsschema zur Ankoppelung der geladenen Peptide an das Copolymergerüst gemäß 1.3 ist in Fig. 2 dargestellt: An Produkt 3 bzw. 4 werden Peptide mit freien Thiolgruppen gekoppelt, z.B. das Peptid INF7 (links) oder das Peptid YE5C. Es werden die Produkte P3INF7 (aus O,O'-Bis(2-aminoethyl)poly(ethylenglykol) 3400), P6INF7 (aus O,O'-Bis(2-aminoethyl)poly(ethylenglykol) 6000) und analog P3YE5C und P6YE5C erhalten.

**BEISPIEL 2: Herstellung des Copoylymergerüsts aus Fmoc-6-Aminohexanoylglutaminsäure und O,O'-Bis (2-aminoethyl)poly(ethylenglykol) 6000 (Diamino-PEG-6000; Fluka) oder O,O'-Bis(2-aminoethyl)poly(ethylenglykol) 3400 (Diamino-PEG-3400; Fluka)**

In diesem Fall gilt für die allgemeine Formel I:
W=Y=NH; X=Fmoc-6-Aminohexanoyl-glutaminsäure.

Das heißt, X ist gemäß i) ein Aminosäurederivat, das durch Ankoppelung von Fmoc-6-Aminohexansäure an Glutaminsäure erhalten wurde. Für die Ankoppelung des Effektormoleküls E kann Z entfallen oder ein bifunktioneller Linker wie SPDP oder EMCS sein.
Ein zur Ankoppelung an dieses Polymergerüst geeigneter Effektor E kann ein Peptid des Typs $E4E^{PROT}$ (Z entfällt) oder des Typs YE5C sein, wobei dieses über das Cystein-Thiol mit dem Linkermolekül Z (z.B. SPDP oder EMCS) reagiert.

a) Synthese des Dipeptids Fmoc-6-Aminohexansäure- GluOH (6):
1 g Fmoc-geschützte 6-Aminohexansäure (2.82 mmol), 1.2 eq. Glu(OtBu)OtBu und 1.2 eq. 1-Hydroxybenzotriazol werden in 200 ml Dichlormethan gelöst. Die Mischung wurde nach Kühlen auf 0°C mit 1.2 eq. N-Ethyl-N'-(dimethylaminopropyl)-carbodiimid und 1.7 ml Diisopropylethylamin versetzt (pH = 8). Nach einer Stunde bei O°C wurde noch 18 Std bei Raumtemperatur gerührt. Das Lösungsmittel wurde nun vollständig abdestilliert, der Rückstand mit Ethylacetat aufgenommen und mit 0.5 N Salzsäure, gesättigter Natriumhydrogencarbonat-Lösung und gesättigter Natriumchlorid-Lösung extrahiert. Nach Abziehen des Lösungsmittels wurde Fmoc-6-Aminohexansäure-Giu (OtBu)OtBu (5) nach Gefriertocknung erhalten.
Di-t-butyl-geschützes Derivat (5) wurde in 30 ml Dichlormethan/Trifluoressigsäure 2:1 gelöst und eine Stunde bei Raumtemperatur gerührt. Nach vollständiger Reaktion (Reaktionskontrolle mit reversed phase-HPLC) wurde das Lösungsmittel auf ca. 5% des Ausgangsvolumen reduziert und das Produkt (6) durch Präzipitation aus Diethylether erhalten. Abschließende Reinigung erfolgte durch RP-HPLC mit einem Acetonitril/ Wasser/ 0.1%TFA Gradienten.

b) Copolymerisation von Fmoc-6-Aminohexansäure-GluOH (6) mit O,O'-Bis(2-aminoethyl)poly(ethylenglykol) 3400' (Diamino-PEG-3400, Fluka), Produkt (7):
10 mg (6), 1.5 eq. O,O'-Bis(2-aminoethyl)poly(ethylenglykol) 3400', 2 eq. Dicyclohexylcarbodiimid und 0.25 eq. 4-(Dimethylamino)-pyridin werden in 5 ml Dichlormethan gelöst. Nach 30 minütigem Rühren bei Raumtemperatur wurde die Lösung nach Aufkonzentrieren filtriert und das Lösungsmittel danach vollständig abdestilliert. Der Rückstand wurde in 500 µl Wasser suspendiert und gefriergetrocknet.
Nach Abspaltung der Fmoc-Schutzgruppe (20% Piperidin in Dimethylformamid oder Dichlormethan) vom Polymer kann das Copolymer unter Verwendung gängiger Peptid-Kupplungschemie mit beliebigen Peptiden mit freiem C-Terminus conjugiert werden.

**BEISPIEL 3: Herstellung des Copolymergerüsts aus dem geschützten Peptid $E4E^{PROT}$ und O,O'-Bis(2-aminoethyl)poly(ethylenglykol) 6000 (Diamino-PEG-6000; Fluka oder O,O'-Bis(2-aminoethyl)poly(ethylenglykol) 3400 (Diamino-PEG-3400; Fluka)**

In diesem Fall ist in der allgemeinen Formel I
W=Y=NH; X = das verzweigte Peptid $E4E^{PROT}$.

In diesem Beispiel stellt ein polyanionisches Peptid X gemäß i) selbst den Effektor dar; daher entfallen Z und E (m = n = 0)
Copolymerisation von $E4E^{PROT}$ mit O,O'-Bis(2-aminoethyl)-poly-(ethylenglykol) 6000 (Diamino-PEG-6000, Fluka) (8);

50 µmol $E4E^{PROT}$, 1.5 eq. O,O'-Bis(2-aminoethyl)-poly(ethylenglykol) 6000, 2 eq. Dicyclohexylcarbodiimid und 0.25 eq. 4-(Dimethylamino-)pyridin wurden in 10 ml Dichlormethan gelöst. Nach vierstündigem Rühren bei 4°C wurde die Lösung nach Aufkonzentrieren filtriert und das Lösungsmittel vollständig abdestilliert. Der Rückstand wurde in 500 µl Wasser suspendiert und gefriergetrocknet.

Zur Abspaltung der verbliebenen säurelabilen Seitenkettenschutzgruppen wurde wie in der Literatur beschrieben mit Trifluoressigsäure unter Zusatz von bis zu 5% Scavenger (bevorzugt Ethandithiol, Triethylsilan, Thioanisol) versetzt und zwei Stunden bei Raumtemperatur gerührt. Die Isolierung des Rohproduktes erfolgte durch Präzipitation aus Diethylether. Die endgültige Reinigung erfolgte wie oben beschrieben mittels Gelfiltration (Superdex75, Pharmacia).

Fig. 3: zeigt das Reaktionsschema: Die Benzylschutzgruppe an Carboxylat 1 der C-terminalen Glutaminsäure des voligeschützten Peptids $E4E^{PROT}$ wird selektiv mit $H_2$/Palladium auf Aktivkohle abgespalten. Das Produkt wird

unter DCC-Aktivierung mit O,O'-Bis(2-aminoethyl)poly(ethylenglykol) 6000 oder mit O,O'-Bis(2-aminoethyl)poly(ethylenglykol) 3400 copolymerisiert. Im letzten Schritt werden die Schutzgruppen der N-terminal gelegenen Glutaminsäuren mit TFA in DCM abgespalten.

**BEISPIEL 4: Komplementaktivierungsstudien**

Der Test wurde im wesentlichen wie in Plank et al., 1996, beschrieben durchgeführt.

a) Polylysin-DNA-Komplexe mit und ohne Copolymer P6INF7:
Polylysin (durchschn. Kettenlänge 170; Sigma) - DNA wurde als Stock-Lösung hergestellt, indem 64 µg pCMVLuc in 800 µl HBS zu 256 µg pL in 800 µl HBS gegeben wurden und durch Pipettieren gemixt wurden, dies entspricht einem berechneten Ladungsverhältnis von 6.3. Von dieser Suspension von Polyplexen wurden als Positivkontrolle je 50 µl in Spalte 1 A-F einer 96-Kalottenplatte gegeben und mit 100 µl $GVB^{2+}$-Puffer versetzt. Alle anderen Vertiefungen enthielten 50 µl $GVB^{2+}$-Puffer. 100 µl wurden von Spalte 1 in Spalte 2 transferiert, gemixt usw. wie in Plank et al. 1996 beschrieben.

Desweiteren wurden je 350 µl der Polylysin-DNA Stock-Lösung versetzt mit 35, 70 und 105 nmol (bezogen auf Anteil INF7) des Polymers P6INF7 und nach 15 min Inkubation mit $GVB^{2+}$-Puffer auf 1050 µl aufgefüllt. Je 150 µl der resultierenden Suspension wurden auf die Reihen A bis F der Spalte 1 einer 96-Kalottenplatte aufgefüllt. Eine 1.5-fache Verdünnungsreihe in $GVB^{2+}$-Puffer und der weitere Komplementaktivierungstest wurden wie oben und in Plank et al. 1996 ausgeführt.

Die Endkonzentrationen der Komponenten in Spalte 1 sind für DNA 2/3 µg, für pL 8/3 µg und für das Polymer 0, 5, 10, 15 nmol (bezogen auf INF7) pro 200 µl Gesamtvolumen.

b) Komplementaktivierung durch PEI-DNA-Komplexe mit und ohne Copolymerhülle:
Der Test wurde wie beschrieben durchgeführt.
PEI (25 kD, Aldrich) - DNA-Komplexe wurden durch Vereinigung gleicher Volumina einer DNA-Lösung (80 µg/ml in 20 mM HEPES pH 7.4) und einer PEI-Lösung (83,4 µg/ml in 20 mM HEPES pH 7.4) hergestellt. Die DNA-Komplexe wurden zur Entfernung von überschüssigem, ungebundenem PEI drei mal 15 min bei 350 x g in Centricon-100-Filterröhrchen (Millipore) zentrifugiert, wobei zwischen Zentrifugationen jeweils mit in 20 mM HEPES pH 7.4 auf das ursprüngliche Volumen aufgefüllt wurde. Nach dem letzten Zentrifugationsschritt wurde eine Stocklösung an DNA-Komplex erhalten, die einer DNA-Konzentration von 300 µg/ml entsprach. 182 µl dieser Lösung wurden mit 20 mM HEPES pH 7.4 auf 2520 µl verdünnt. Je 610 µl dieser Lösung (entsprechend je 13,2 µg DNA) wurden zu Lösungen von P6YE5C in je 277,6 µl 20 mM HEPES pH 7.4 pipettiert. Die resultierenden Lösungen wurden mit 5 M NaCl auf eine Salzkonzentration von 150 mM gebracht. Je 150 µl der resultierenden Lösungen wurden in Spalte 1, A bis F, einer 96-Well-Platte transferiert. Die Verdünnungsreihe in $GVB^{2+}$-Puffer wurde wie beschrieben durchgeführt (Plank et al. 1996).

Ebenso wurden je 610 µl PEI-DNA-Komplex höherer Konzentration (86 ng DNA pro µl) mit je 277,6 µl von Lösungen des Polymers P3YE5C inkubiert. Die Lösungen enthielten 0, 1, 2 ,3 Ladungsäquivalente bezüglich der eingesetzten DNA an Peptid YE5C. Nach 15 min wurden je 27,45 µl 5 M NaCl zugefügt (resultierendes Gesamtvolumen 915 µl). Je 150 µl der resultierenden Lösung wurden in Spalte 1, Reihen A bis F einer 96-Kalottenplatte transferiert (dies entspricht jeweils 8.6 µg DNA und 9 µg PEI). Die Verdünnungsreihe in $GVB^{2+}$ und der weitere Versuchsvorgang wurde wie oben für pL-DNA beschrieben durchgeführt.

Das Ergebnis des Komplementaktivierungstests ist in Fig. 4 dargestellt:

A) Komplementaktivierung durch Polylysin-DNA-Komplexe in Anwesenheit und Abwesenheit des Copolymers P6INF7. Der CH50-Wert bezeichnet eine bestimmte Serumverdünnung, die zur Lyse von 50 % der Schaferythrozyten in diesem Versuchsaufbau führt. Der Wert $CH50_{max}$ bezeichnet jenen CH50-Wert, der mit unbehandeltem Humanserum erhalten wird. Im beschriebenen Versuchsaufbau wurde Humanserum mit Genvektoren inkubiert. Die CH50-Werte, die mit so behandeltem Serum erhalten werden, sind niedriger als $CH50_{max}$, wenn die Vektoren die Komplementkaskade aktivieren. Die Daten sind in Prozent von $CH50_{max}$ gezeigt. Die starke Komplementaktivierung, die mit Polylysin-DNA-Komplexen beobachtet wurde, kann durch Hüllpolymer P6INF7 gänzlich unterbunden werden.

B) Das Peptid INF7 selbst, in freier Form oder polymergebunden, ist ein schwacher Komplement-Aktivator. Eingebaut in einen Polylysin-DNA-Komplex verschwindet diese Komplementaktivierung.

C) Komplementaktivierung durch PEI-DNA-Komplexe (N/P = 8) in Gegenwart des Copolymers P3YE5C. Der ungeschützte DNA-Komplex ist ein starker Aktivator des Komplementsystems. Das Copolymer P3YE5C vermindert die Komplementaktivierung in Abhängigkeit von der zugesetzten Menge an Copolymer, führt jedoch im untersuchten Bereich nicht zu vollständigem Schutz.

D) Dagegen schützt Copolymer P6YE5C schon in kleinen zugesetzten Mengen vollständig vor Komplementaktivierung.

**BEISPIEL 5: Erythrozyten-Lyse-Test**

Der durchgeführte Test dient zur Untersuchung der Fähigkeit von Peptiden, natürliche Membranen pH-abhängig zu lysieren.

Die in diesem Beispiel verwendeten Erythrozyten wurden wie folgt erhalten: 10 ml frisches Blut wurde von Freiwilligen entnommen und sofort in 10 ml Alsevers Lösung (Whaley 1985; Plank et al., 1996) aufgenommen. Aliquots von je 3 ml wurden 3 mal mit dem entsprechenden Puffer (je 40 ml Citrat bzw. HBS) gewaschen (nach Pufferzugabe und Schütteln Zentrifugation bei 2500 x g und Verwerfen des Überstandes). Die Konzentration der Erythrozyten wurde mit einem "Extinktionskoeffizienten" von 2.394 x $10^{-8}$ ml/Zellen bei 541 nm bestimmt. Zur Herleitung des Extinktionskoeffizienten wurde die Zellzahl in einem Aliquot mit einer Neubauerkammer bestimmt und nachfolgend die Extinktion dieser Lösung nach Zugabe von 1 µl 1 % Triton X-100 bei 541 nm bestimmt.

In einer 96-Kalotten-Platte wurden in Spalte 1. Aliquots von INF7 bzw. copolymergekoppelten INF7 (P3INF7) in 150 µl 10 mM Natriumcitrat pH 5 / 150 mM NaCl bzw. in HBS-Puffer vorgelegt (üblicherweise 45 µmol Peptid). In allen anderen Vertiefungen wurden je 50 µl Puffer (Citrat bzw. HBS) vorgelegt. 100 µl wurden mit einer Mehrkanalpipette von Spalte 1 in Spalte 2 transferiert, durch Pipettieren gemixt, 100 µl wurden von Spalte 2 in Spalte 3 transferiert usw. Die übrigen 100 µl aus Spalte 11 wurden verworfen, in Spalte 12 befand sich nur Puffer. Die resultierende 1.5-fache Verdünnungsreihe wurde mit je 50 µl Puffer (Citrat bzw. HBS) auf 100 µl aufgefüllt. Daraufhin wurden je 3 x $10^6$ humane Erythrozyten zugegeben, die Platten mit Parafilm abgedeckt und bei 400 rpm in einem Schüttelinkubator (Series 25 Incubator Shaker; New Brunswick Scientific Co.; NJ, USA) bei 37°C für 1 h geschüttelt. Daraufhin wurden die Platten bei 2500 x g zentrifugiert, je 150 µl Überstand in eine Flachboden-96-Kalottenplatte transferiert und freigesetztes Hämoglobin bei 410 nm mit einem ELISA-Plate-Reader gemessen. 100 % Lyse wurde durch Zugabe von 1 µl 1 % Triton X-100 in einzelne Vertiefungen aus Spalte 12 (vor Transferieren in die Flachbodenplatte) bestimmt, 0 % Lyse wurde aus unbehandelten Proben aus Spalte 12 bestimmt.

Das Ergebnis des Erythrozyten-Lyse-Tests ist in Fig. 5 dargestellt: Peptid INF7 zeigte hohe pH-spezifische Aktivität. Aus der Synthese des Copolymers P3INF7 wurden nach chromatographischer Auftrennung (Superdex 75, Pharmacia) vier Fraktionen (abnehmendes Molekulargewicht von 1 bis 4) gewonnen. Von diesen zeigten Fraktionen 2 und 3 gegenüber freiem Peptid INF7 höhere Lyseaktivität. In allen Fällen war die Aktivität streng pH-abhängig, d.h. keine Lyse bei neutralem pH (nicht gezeigt).

**BEISPIEL 6: Größenbestimmung von DNA-Komplexen mittels dynamischer Lichtstreuung und Elektronenmikroskopie**

Herstellung von PEI-DNA Polyplexen und Aufbringen der Polymerhülle: Je 40 µg DNA (pCMVLuc) in 333 µl 20 mM HEPES pH 7.4 wurden zu 41.7 µg PEI (25 kD, Aldrich) in 333 µl HEPES pH 7.4 pipettiert und gemixt. Nach 10 - 15 min Inkubation wurden 0, 0.5, 1, 1.5, 2 oder 3 Ladungsäquivalente (bezüglich der Ladung der eingesetzten DNA) an Polymeren P3YE5C bzw. P6YE5C in je 333 µl HEPES zugesetzt (bzw. 0, 1, 2, 3 und 5 Äquivalente in einem zweiten Experiment). Dies entspricht einer Menge bezogen auf das Peptid YE5C von 0, 152, 303, 455, 606 oder 909 pmol an Polymer pro µg DNA. DOTAP/Cholesterin-DNA-Komplexe wurden aus DOTAP/Cholesterin (1:1 mol/mol)-Liposomen in 330 µl 20 mM HEPES pH 7.4 und DNA im selben Volumen bei einem Ladungsverhältnis von 5 hergestellt. Die Lipoplexe wurden mit 0, 1, 2, 3 und 5 Äquivalenten des Copolymers P3YE5C in 330 µl Puffer inkubiert. Die endgültige DNA-Konzentration des Komplexes war 10 µg/ml.

Die Größe der DNA-Komplexe wurde einerseits durch dynamische Lichtstreuung (Zetamaster 3000, Malvem Instruments) sofort nach Polymerzugabe und dann zu verschiedenen Zeitpunkten über mehrere Stunden hinweg bestimmt, andererseits durch Elektronenmikroskopie wie in Erbacher et al., 1998, und Erbacher et al., 1999, beschrieben.

Fig. 6 zeigt die elektronenmikroskopischen Aufnahmen von PEI-DNA-Komplexen (N/P = 8) in Gegenwart des Copolymers P3YE5C.

A) In Gegenwart eines Ladungsäquivalents (bezüglich der Ladungen der eingesetzten DNA) des Copolymers. Die Partikelgröße beträgt 20 bis 30 nm.

B) In Gegenwart von zwei Ladungsäquivalenten des Copolymers. Die Mehrzahl der Partikel weist Größen um 20

nm auf. Dies sind monomolekulare DNA-Komplexe, d.h. ein Plasmidmolekül verpackt in ein Partikel.

C) In Gegenwart von 1,5 Ladungsäquivalenten des Copolymers nach Zugabe von BSA zu einer Endkonzentration von 1 mg/ml und inkubation über Nacht. Copolymergeschützte DNA-Komplexe bleiben stabil und aggregieren nicht, im Gegensatz zu ungeschützten PEI-DNA-Komplexen, die unter diesen Bedingungen sofort präzipitieren (nicht gezeigt).

**<u>BEISPIEL 7:</u> Bestimmung des Zetapotentials von DNA-Komplexen**

An den gleichen Proben wie in Beispiel 6 wurden die Zetapotentiale mit dem Gerät von Malvem bestimmt, wobei die Parameter Brechungsindex, Viskosität und Dielektrizitätskonstante auf die Werte von deionisiertem Wasser eingestellt wurden, was nur näherungsweise gelten kann.

Fig. 7 zeigt das Zetapotential von PEI- und DOTAP/Cholesterin-DNA-Komplexen in Abhängigkeit von der Menge zugesetzten Copolymers P3YE5C. Das Zetapotential als Maß für die Oberflächenladung der Komplexe nimmt von stark positiv über neutral zu leicht negativ mit steigender Menge zugesetzten Copolymers ab. Dies zeigt, daß das Copolymer an die DNA-Komplexe bindet und deren elektrostatische Ladung ausgleicht bzw. abschirmt.

**<u>BEISPIEL 8:</u> Herstellung von DNA-Komplexen und Transfektionen**

Für die in den nachfolgenden Beispielen durchgeführten Zellkultur- und Transfektionsexperimente wurden, wenn nicht anders angegeben, die folgenden Materialien und Methoden verwendet:

a) Gentransfer in Zellkultur in einer 96-Well Platte
Adhärente Zellen werden am Tag vor der Transfektion in Flachboden-Platten zu 20.000 - 30.000 Zellen pro Vertiefung ausgesät (je nach Teilungsrate der Zellen. Während der Transfektion sollte die Konfluenz etwa 70 - 80 % sein).
Vor der Transfektion wird Medium abgesaugt. Zur Transfektion gibt man 150 µl Medium auf die Zellen und gibt dann 50 µl DNA-Komplexe zu.

b) Zusammensetzung der DNA-Komplexe: Vorzugsweise 1 µg DNA/well Endkonzentration; Berechnung für 1.2-fache Menge; 20 µl Volumen pro Komponente (DNA, PEI, Polymer). Schließlich werden 50 µl DNA-Komplex für die Transfektion verwendet. Puffer: 20 mM HEPES pH 7.4/ 150 mM NaCl = HBS. Die verwendeten Volumina an Puffer bleiben gleich.
Für den Fall, daß man DNA-Komplexe für 96 Einzelversuche benötigt, führt man die Berechung zweckmäßig so durch, als würde man 100 Einzelversuche durchführen: z.B.
DNA: 1 µg x 100 x 1.2 = 120 µg in 20 x 100 µl HBS = 2 ml Gesamtvolumen. Polyethylenimin (PEI): Um ein N/P-Verhältnis von 8 zu erzielen berechnet man gemäß der Formel

$$N/P = \frac{(\mu gPEI)}{43} \times \frac{330}{(\mu gDNA)}$$

$$8 = \frac{(\mu gPEI)}{43} \times \frac{330}{(120)},$$

daß man 125.09 µg PEI benötigt und zwar in 20 x 100 µl = 2 ml HBS.
Hüllpolymer: Will man z.B. für die angegebene Menge DNA und PEI Hüllpolymer in einer Menge von 2 Ladungsäquivaltenten (bezüglich DNA) einsetzen so berechnet sich nach der Formel

$$\mu l\ (\text{Hüllpol.}) = 1000 \times \frac{(\mu gDNA)}{330} \times \frac{Ladungsäqu.}{c(\text{Hüllpol. } [\mu mol/ml])}$$

das benötigte Volumen an Hüllpolymer bei einer Konzentration c von 11.1 µmol / ml als

$$\mu l\ (\text{Hüllpol.}) = 1000 \times \frac{120}{330} \times \frac{2}{11.1} = 65.5\ \mu l,$$

die ebenfalls auf 2 ml mit HBS aufgefüllt werden.
Dies ist ein Beispiel für 100 Versuche zu je 1 µg DNA. Üblicherweise werden etwa je 5 Versuche durchgeführt und

z.B. N/P-Verhältnisse von 4, 5, 6, 7, 8 mit jeweils 0, 1, 2, 3 Ladungsäquivalenten Hüllpolymer untersucht.

c) Mischen der DNA-Komplexe:
Nach Herstellung der benötigten Vorverdünnungen wird DNA unter Vortexen zu PEI gegeben. Nach 15 min wird wiederum unter Vortexen Hüllpolymer zum vorgefertigten PEI-DNA-Komplex gegeben. Nach weiteren 30 min werden je 50 µl DNA-Komplexe zu den Zellen gegeben, die sich in je 150 µl Medium befinden. Die verwendeten Gefäße richten sich nach dem berechneten Gesamtvolumen. Im obigen Fall wird PEI zweckmäßig in einem 14 ml Polypropylen-Röhrchen (z.B. Falcon 2059) vorgelegt, die anderen beiden Komponenten in 6 ml-Röhrchen (z.B. Faicon 2063). Für Einzelversuche im 96-Well-Plate kann man die Komponenten auch in einer 96-Well-Platte anmischen. Wenn das endgültige Gesamtvolumen der DNA-Komplexe 1 - 1.5 ml beträgt, eignen sich Eppendorf-Tubes. Zum Mischen kann statt Vortexen mit der Mikropipette auf- und abpipettiert werden.

Umrechnung auf 3 cm-Schalen (6-well plate):
Für 3 cm-Schalen (6-well plate) werden zweckmäßig Mengen von 2 bis 5 µg DNA, Volumen je Komponente etwa je 100 µl eingesetzt, wobei die Berechnung analog zu oben erfolgt. Im 12-well plate werden zweckmäßig Mengen von ca. 1 µg DNA je Versuch eingesetzt.

Fig. 8 zeigt schematisch die Formulierung von DNA-Kompiexen: Bevorzugt wird zuerst Polykation mit Plasmid-DNA inkubiert, was zu einem positiv geladenen DNA-Komplex führt (z.B. PEI, N/P = 8). Dann wird negativ geladenes Copolymer zugesetzt, das elektrostatisch an den vorgeformten Komplex bindet. Das Copolymer kann mit Rezeptorliganden modifiziert sein, symbolisiert durch Sterne (rechts).

d) Luciferin-Substratpuffer
Als Luciferin-Substratpuffer wurde eine Mischung von 60 mM Dithiothreitol, 10 mM Magnesiumsulfat, 1 mM ATP, 30 µM D (-)-Luciferin, in 25 mM Glycyl-Glycin-Puffer pH 7.8, verwendet.

e) Proteinbestimmung in Zellysaten
Den Proteingehalt der Lysate bestimmte man mit dem Bio-Rad Protein Assay (Fa. Bio-Ra): Zu 10 µl (bzw. 5 µl) des Lysats wurden 150 µl (bzw. 155 µl) dest. Wasser und 40 µl Bio-Rad Protein Assay Farbstoff-Konzentrat in eine Vertiefung einer durchsichtigen 96-Kalotten-Platte (Typ "flat bottom", Fa. Nunc, Dänemark) gegeben und bei 630 nm mit dem Absorbance-Reader "Biolumin 690" und dem Computer-Programm "Xperiment" (beide Fa. Molecular Dynamics, USA) die Absorption gemessen. Als Eichkurve wurden Konzentrationen von 50, 33.3, 22.3, 15, 9.9, 6.6, 4.4, 2.9, 2.0, 1.3, 0.9 und 0 ng BSA / µl vermessen. Bovines Serum Albumin (BSA) wurde als Bio-Rad Protein Assay Standard II gekauft. Somit konnte insgesamt eine Angabe in pg Luciferase pro mg Protein gemacht werden.

**<u>BEISPIEL 9:</u> Gentransfer in K562-Zellen mit PEI(25 kD)-DNA-Komplexen in Gegenwart und Abwesenheit des Copolymers P3YE5C**

K562-Zellen (ATCC CCL 243) wurden in RPMI-1640-Medium unter Zusatz von 10 % FCS, 100 units/ml Penicillin, 100 µg/ml Streptomycin und 2 mM Glutamin bei 37°C in einer Atmosphäre von 5 % $CO_2$ kultiviert. Die Zellen wurden am Abend vor der Transfektion mit Desferoxamin zu einer Endkonzentration von 10 µM versetzt. Unmittelbar vor der Transfektion wurde das Medium gewechselt. Je 50.000 Zellen in je 160 µl Medium wurden in die Vertiefungen einer 96-Well-Platte ausgebracht.
Transferrin-PEI (hTf-PEI 25 kD) wurde im wesentlichen wie von Kircheis et al. beschrieben (Kircheis et al., 1997) durch reduktive Aminierung hergestellt. Es wurde ein Produkt erhalten, das durchschnittlich 1.7 Transferrinmoleküle pro PEI-Molekül gekoppelt hat.
In einem Vorversuch wurde eine Zusammensetzung von hTf-PEI-Polyplexen bestimmt, die hohe Transfektion ergibt und bei der ein deutlicher Einfluß des Rezeptorliganden feststellbar ist.
32.4 µg hTf-PEI (Mengenangabe bezieht sich auf hTf) in 600 µl HBS wurden mit 36 µg PEI (25kD) in 600 µl HBS vereinigt. Zu dieser Mischung wurden 40 µg DNA (pCMVLuc) in 600 µl HBS pipettiert und gemischt. Je 270 µl der resultierenden Lösung wurden nach 15 min zu je 90 µl von Lösungen des Polymers P3YE5C in HBS bzw. HBS alleine gegeben. Diese Lösungen enthielten Polymermengen, die 0/0.5/1/1.5/2/3 Ladungsäquivalenten bezüglich der Ladung der eingesetzten DNA enthielten. Gleichermaßen wurden DNA-Komplexe ohne hTf mit der äquivalenten Menge PEI hergestellt (40 µg DNA + 42 µg PEI + Hüllpolymer). Je 60 µl der resultierenden Mischungen (entspricht einer Menge von je 1 µg DNA / well) wurden in je 5 Vertiefungen einer Rundboden-96-Well-Platte vorgelegt und 50.000 K562 in je 160 µl RPMI-Medium wurden zugegeben. Nach 24 h wurden die Zellen durch Zentrifugation sedimentiert, der Überstand durch Aspiration entfernt und je 100 µl Lysepuffer (250 mM Tris pH 7.8; 0.1 % Triton X-100) wurden zugegeben. Nach 15 min Inkubation wurde durch Pipettieren gemischt und je 10 µl Probe wurden zum Luciferasetest im 96-well-plate-Format in eine schwarze Platte (Costar) transferiert und mit je 100 µl Luciferin-Substrat-Puffer versetzt. Die Messung der resultierenden Lichtemission erfolgte mittels Microplate Scintillation & Luminescence counter "Top Count" (Fa. Canberra-Packard, Dreieich). Die Meßzeit betrug 12 Sekunden, die Meßverzögerung war 10 min. und Hintergrund-Werte wurden automatisch abgezogen. Als Standard wurden je 100, 50, 25, 12.6, 6.25, 3.13, 1.57, 0.78, 0.39, 0.2, 0.1, 0.05, 0.025, 0.013, 0.007 und 0 ng Luciferase (Boehringer Mannheim) in je 10 µl Lysepuffer (= 2-fache Verdünnungsserie) unter gleichen Bedingungen gemessen und daraus eine Eichkurve erstellt.

Fig. 9 zeigt das Ergebnis der Gentransferexperimente mit PEI-DNA-Komplexen (N/P = 8) in K562-Zellen in Gegenwart und Abwesenheit von Transferrin als Rezeptorligand unter Zusatz des Copolymers P3YE5C. Das Copolymer interferiert nicht mit Gentransfer und steigert diesen sogar, wenn ein Rezeptorligand im DNA-Komplex vorhanden ist.

Gezeigt ist die Expression des Reportergens Luciferase bezogen auf die Menge Gesamtprotein im Zellextrakt (Mittelwerte und Standardabweichungen aus Triplikaten).

**<u>BEISPIEL 10</u>: Transfektion der Brustkrebszellinie MDA-MB435S mit Polylysin-DNA-Komplexen in Gegenwart und Abwesenheit des Hüllpolymers P3INF7**

MDA-MB435S Zellen ( humane Brustkrebs-Zellinie) wurden in DMEM-Medium. unter Zusatz von 10 % FCS, 100 units/ml Penicillin, 100 µg/ml Streptomycin und 2 mM Glutamine bei 37°C in einer Atmosphäre von 5 % $CO_2$ kultiviert. Am Abend vor der Transfektion wurden die Zellen zu 20.000 Zellen pro Vertiefung in 96-Well-Platten (Flachboden) ausgebracht.
Die DNA-Komplexe wurden wie folgt hergestellt:
Berechnung für 1 Well: Zu erzielende Menge ist 1 µg DNA pro Well, 4 µg pL170 in einem Gesamtvolumen von 60 µl HBS. Diese Mengen wurden mit 1.2 multipliziert. Die DNA-Komplexe wurden wie in folgender Tabelle angegeben gemixt, wobei zuerst DNA zu Polylysin gegeben wurde und dies wiederum nach 15 min zu Polymer P3INF7 bzw. zum Puffer. Die Versuche wurden in Triplikaten ausgeführt. Je 60 µl DNA-Komplexe wurden zu Zellen, bedeckt von 150 µl Medium, gegeben. Nach 4 h wurde Medium gewechselt, nach 24 h wurde nach Waschen mit PBS und Zugabe von 100 µl Lysepuffer der Luciferasetest und der Proteintest wie in Beispiel 9 beschrieben durchgeführt.

| Nr. | P3INF7 | HBS µl | pL170 µl = µg | HBS | 7.2µg DNA in HBS(µl) |
|---|---|---|---|---|---|
| 1 | 144,6 (5 nmol) | 71,4 | 28,8 | 79,2 | 108 |
| 2 | 289,2 (10 nmol) | - | 28,8 | 42,6 | 71,4 |
| 3 | - | - | 28,8 | 187,2 | 216 |

Fig. 10 zeigt das Ergebnis der Gentransferexperimente mit Polylysin-DNA-Komplexen in die humane Brustkrebszellinie MDA-MB435S in Gegenwart und Abwesenheit des Copolymers P3INF7. In Abwesenheit des Copolymers tritt keine meßbare Reportergenexpression auf. Die pH-abhängig membranzerstörende und somit endosomolytische Aktivität des Copolymers bewirkt effizienten Gentransfer. 5 nmol bzw. 10 nmol P3INF7 beziehen sich auf die Menge des eingesetzten, polymergebundenen Peptids INF7.

**<u>BEISPIEL 11</u>: Lipofection in Gegenwart von Hüllpolymeren (Fig. 11)**

NIH3T3-Zellen (ATCC CRL 1658) wurden in DMEM-Medium unter Zusatz von 10 % FCS, 100 units/ml Penicillin, 100 µg/ml Streptomycin und 2 mM Glutamine bei 37°C in einer Atmosphäre von 5 % $CO_2$ kultiviert.
Am Abend vor der Transfektion in 6-Well-Plates in einer Dichte von 500.000 Zellen pro Vertiefung ausgebracht.

Herstellung von DNA-Komplexen:
16 µg DNA in 240 µl 20 mM HEPES pH 7.4 wurden mit einer Lösung von 242 nmol DOTAP/Cholesterin-Liposomen in 240 µl des gleichen Puffers versetzt. Dies ergibt ein Ladungsverhältnis ($^+/_-$) von 5. 210 µl der resultierenden Lösung wurden zu 105 µl einer Lösung pipettiert, die 6,36 nmol des Polymers P3YE5C enthielt (bezogen auf den Peptidanteil YE5C; dies entspricht 3 DNA-Ladungsäquivalenten). Für den Kontrollversuch wurden 210 µl DOTAP/Cholesterin-DNA zu 105 µl 20 mM HEPES pH 7.4 pipettiert. Je 90 µl der resultierenden DNA-Komplexe wurden den Zellen zugegeben, die sich in 800 µl frischem Medium befanden; dies entspricht 2 µg DNA pro Vertiefung. Die Versuche wurden in Triplikaten ausgeführt.

Gleichermaßen wurde der Versuch mit Lipofectamine™ anstatt DOTAP/Cholesterin durchgeführt. In diesem Falle wurde eine Menge von Lipofectamin (DOSPA) eingesetzt, die zu einem Ladungsverhältnis von 7 ($^+/_-$) führt.

30 min nach Zugabe der DNA-Komplexe wurden den Zellen je 1 ml frisches Medium zugefügt, nach 3 h weitere 2 ml. Das Medium wurde nicht gewechselt. 22 h nach Komplex-Zugabe wurden die Zellen mit PBS gewaschen und in 500 µl Lysepuffer lysiert. Aliquots des Zellysats wurden im Luciferasetest und zur Proteinbestimmung eingesetzt.

Fig. 11 zeigt das Ergebnis der Lipofection in NIH3T3-Zellen in Gegenwart und Abwesenheit des Copolymers P3YE5C. Weder die Transfektion mit DOTAP/Cholesterin-DNA noch jene mit Lipofectamin wird signifikant erniedrigt (3 Ladungsäquivalente des Copolymers; DOTAP/Cholesterin-DNA hat bei dieser' Zusammensetzung ein neutrales Zetapotential, s. Fig. 7).

**BEISPIEL 12: Transfektion von HepG2-Zellen mit DOTAP/Cholesterin-DNA und PEI-DNA in Gegenwart und Abwesenheit von P6YE5C**

HepG2-Zellen (ATCC HB 8065) wurden in DMEM-Medium unter Zusatz von 10 % FCS, 100 units/ml Penicillin, 100 µg/ml Streptomycin und 2 mM Glutamine bei 37°C in einer Atmosphäre von 5 % $CO_2$ kultiviert.
Zwei Tage vor der Transfektion wurden die Zellen in 6-Well-Plates in einer Dichte von 500.000 Zellen pro Vertiefung ausgebracht. Die Transfektion mit DOTAP/Cholesterin erfolgte exakt wie oben für NIH3T3-Zellen beschrieben, wobei diesmal Polymer P6YE5C verwendet wurde. Weiters wurden 7 µg DNA in 105 µl HEPES-Puffer zu 7,3 µg PEI 25 kD gelöst im gleichen Volumen pipettiert. Nach 15 min Inkubation wurde diese Lösung zu 105 µl einer Lösung des Polymers P6YE5C pipettiert, die 3 DNA-Ladungsäquivalente an YE5C enthielt. Je 90 µl dieser Lösung wurden den Zellen zugegeben. Die Versuche wurden in Triplikaten ausgeführt.
Fig. 12 zeigt den Gentransfer in HepG2-Zellen in Gegenwart und Abwesenheit des Copolymers P6YE5C. Transfektion durch DOTAP/Cholesterin-DNA wird nicht signifikant inhibiert. Die Transfektion durch PEI-DNA-Komplexe wird erniedrigt (3 Ladunsäquivalente des Copolymers).

**BEISPIEL 13: Intravenöser Gentransfer in vivo**

a) Kontrolle (PEI-DNA, N/P = 8):
150 µg DNA (pCLuc) in 337.5 µl 20 mM HEPES pH 7.4 wurden zu 156.4 µl PEI (25 kD, Aldrich) im gleichen Volumen HEPES-Puffer pipettiert. Nach 15 min wurden 75 µl 50 % Glucose zugegeben. Von dieser Lösung wurden je 100 µl in die Schwanzvene von Mäusen injiziert (entsprechend einer Dosis von 20 µg DNA pro Tier).

b) Kontrolle (DOTAP/Cholesterin-DNA; Ladungsverhältnis $^{+}/_{-}$ = 5):
DOTAP-Cholesterin-Liposomen wurden nach Standardvorschrift hergestellt (Barron et al., 1998). In diesem Fall wurden Liposomen mit einem molaren Verhältnis DOTAP zu Cholesterin von 1:1 hergestellt und einer Endkonzentration von DOTAP von 5 mM in 5 % Glucose. 130 µg DNA in 91.1 µl 20 mM HEPES pH 7.4 wurden zu 393.5 µl Liposomensuspension gegeben. Nach 15 min wurden 65 µl 50 % Glucose zugegeben. Von dieser Lösung wurden je 100 µl in die Schwanzvene von Mäusen injiziert (entsprechend einer Dosis von 20 µg DNA pro Tier).

c) PEI-DNA (N/P = 8) mit Copolymerhülle:
150 µg DNA in 2475 µl wurden zu 156.4 µg PEI (25 kD) im gleichen Volumen unter Vortexen zugegeben. Nach 15 min wurden 3 Ladungsäquivalente (bezüglich der Ladungen der eingesetzten DNA-Menge) an Polymer P3YE5C in 2475 µl HEPES-Puffer unter Vortexen zugegeben. Nach weiteren 30 min wurden die DNA-Komplexe durch Zentrifugation in Centricon 30-Röhrchen auf eine DNA-Konzentration von 454 µg/ml aufkonzentriert. Diese Lösung wurde dann unter Zugabe von 50 % Glucose und 20 mM HEPES pH 7.4 auf eine Endkonzentration von 200 µg DNA pro ml und 5 % Glucose gebracht. Von dieser Lösung wurden je 100 µl in die Schwanzvene von Mäusen injiziert (entsprechend einer Dosis von 20 µg DNA pro Tier).

d) DOTAP/Cholesterin-DNA (5:1) mit Copolymerhülle: 393.9 µl Liposomensuspension wurden direkt zu einer Lösung von 130 µg DNA in 65.3 µl Wasser pipettiert. Nach 15 min wurden 3 Ladungsäquivalente P3YE5C in 216.9 µl HEPES-Puffer zugegeben und nach weiteren 30 min 75 µl 5 % Glucose. Von dieser Lösung wurden je 115.5 µl in die Schwanzvene von Mäusen injiziert (entsprechend einer Dosis von 20 µg DNA pro Tier).

Fig. 13 zeigt das Ergebnis der in vivo Gentransferexperimente: PEI-DNA- bzw. DOTAP/Cholesterin-DNA-Komplexe mit oder ohne gebundenes Copolymer P3YE5C (3 Ladungsäquivalente) wurden in die Schwanzvene von Mäusen injiziert (n = 6). Die Tiere wurden 24 h nach Injektion geopfert, und die Reportergenexpression in Organen wurde bestimmt. Die höchste Aktivität wurde jeweils an der Injektionsstelle gemessen, mit PEI-DNA-Copolymer trat signifikante Reportergenexpression in Lunge und Herz auf, während Gentransfer in die Lunge durch DOTAP/Cholesterin-DNA bei Anwendung des Copolymers inhibiert wurde.

**BEISPIEL 14: Sterische Stabilisierung von PEI-DNA-Komplexen**

PEI-DNA-Komplexe wurden exakt wie in Beispiel 6 beschrieben hergestellt (PEI-DNA, N/P = 8, 0/1,5/3 Ladungsäquivalente Copolymer P3YE5C bzw. P6YE5C). Die Größe der Komplexe wurde mittels dynamischer Lichtstreuung bestimmt und betrug 20 bis 30 nm. Daraufhin wurde 5 M NaCl zu einer Endkonzentration von 150 mM zugegeben. PEI-DNA ohne Copolymer aggregierte sofort (nach 5 min war eine Population von Partikeln >500 nm zu messen; nach 15 min war die Mehrzahl der Partikel >1000 nm; über Nacht fielen die Komplexe aus der Lösung aus). In Gegenwart von P3YE5C bzw. P6YE5C (1,5 oder 3 Ladungsäquivalente) blieb die Partikelgröße zumindest über 3 Tage stabil.

Ebenso führte die Zugabe von BSA zu einer Endkonzentration von 1 mg/ml zu sofortiger Präzipitation von PEI-DNA. In Gegenwart von P3YE5C bzw. P6YE5C (1,5 Ladungsäquivalente und mehr) bleibt die Partikelgröße zumindest über 24 h konstant (siehe auch Fig. 6c).

**BEISPIEL 15: Herstellung von Kollagenschwämmen, die mit COPROGs (copolymer-protected gene vectors) beladen sind**

Je 500 µl einer Plasmid-DNA-Lösung (codierend für Luciferase unter der Kontrolle des CMV-Promotors; Konzentration 0.5 mg/ml in Wasser) wurden je 500 µl einer Polyethylenimin-Lösung (25 kD; Aldrich; 521 µg/ml in Wasser) mit einer Mikropipette zugegeben und rasch durch Aufziehen und Ausblasen der Pipette gemischt. Die resultierende Vektorsuspension wurde 500 µl einer wässrigen Lösung des PROCOP ("protective copolymer") P6YE5C zugegeben und durch rasches Pipettieren gemischt. Die PROCOP-Lösung enthielt je zwei Ladungsäquivalente P6YE5C. Die Ladungsäquivalente beziehen sich auf den Quotienten der (negativen) Ladung im PROCOP und der negativen Ladung der DNA. Die Menge in nmol an einzusetzendem PROCOP berechnet sich nach der Formel

$$PROCOP(nmol) = \frac{DNA(\mu g)}{330} \times CE$$

Die Menge in Mikrolitern an einzusetzendem PROCOP berechnet sich nach

$$PROCOP(\mu l) = \frac{DNA(\mu g)}{330} \times \frac{CE}{c_{PROCOP}(mM)}$$

wobei CE die Ladungsäquivalente ("charge equivalents") an PROCOP bedeutet und $c_{PROCOP}$ die Konzentration des Copolymers ist. Die Konzentration des Polymers wird anhand der Konzentration der (negativen) Ladungen des (anionischen) Peptids im Polymer angegeben, welche wiederum durch photometrische Bestimmung der Peptid konzentration basierend auf der Extinktion des Tyrosins im Peptid bestimmt wird. Die resultierenden wässrigen Vektorsuspensionen wurden vereinigt. Je 3 ml Vektorsuspension wurden pro 4.5 x 5 cm Tachotop-Schwamm mit einer Mikropipette aufgeträufelt (der kommerziell erhältliche Schwamm wurde vorher unter der Sterilbank auf diese Größe zurechtgeschnitten, gewogen und in sterilen Glaspetrischalen vorgelegt). Nach zwei bis drei Stunden Inkubation bei Raumtemperatur wurden die Petrischalen in einem Lyophilisator (Hetosicc CD4, Fa. Heto) kurzzeitig einem Vakuum ausgesetzt, gefolgt von plötzlicher Belüftung der Vakuumkammer ("vacuum loading"). Dies führt dazu, dass die Luftblasen im Schwamm beseitigt werden und sich der Schwamm zur Gänze mit Flüssigkeit füllt. Nach insgesamt 4 Stunden Inkubation wurden die Schwämme in den Petrischalen im Lyophilisator ohne vorheriges Gefrieren über Nacht getrocknet. Die Schwämme wurden dann bis zur Implantation in Versuchstiere in den mit Parafilm verschlossenen Petrischalen bei 4°C gelagert.

**BEISPIEL 16: Herstellung, von Kollagenschwämmen, die mit herkömmlichen Genvektoren beladen sind**

(a) Beladung mit nackter Plasmid-DNA
Auf einen 4.5 x 5 cm Tachotop-Schwamm wurden unter sterilen. Bedingungen 500 µg Plasmid-DNA (pCMVLuc), gelöst in 5 ml 5 % Glucose, aufpipettiert. Dies entspricht ca. 20 µg DNA pro $cm^2$. Nach 24 h Inkubation bei 4 °C wurde der Schwamm gefriergetrocknet (Lyophilisator Hetosicc CD4, Fa. Heto, Vakuum < 10 µbar) und steril in Stücke von ca. 1,5 x 1,5 cm geschnitten. Ein solches Schwammstück entspricht also einer Beladung von ca. 45 µg DNA.
Solche Präparate wurden wie in Beispiel 19 zum Gentransfer in vitro eingesetzt. Fig. 15 zeigt eine von Anbeginn niedrige Reportergenexpression, die nach kurzer Zeit nicht mehr nachweisbar ist.

(b) Polyethylenimin / DNA Schwämme

Vorbehandlung von PEI und Bildung von DNA-Komplexen:

PEI (25 kD Molekulargewicht) wurde in sterilem Aqua Dest. oder in HBS-Puffer gelöst und durch Zugabe von 80 µl konzentrierter Salzsäure pro 100 mg PEI neutralisiert. Diese Lösung wurde mittels Centrion 30 Concentrators (Fa. Amicon-Millipore) oder mittels Dialyse (Ausschlußgrenze 12-14 kD) von niedermolekularen Komponenten befreit. Die Konzentration der Lösung wurde mittels Ninhydrintest, bei dem die primären Amine quantifiziert werden, bestimmt.

Zur Bildung von DNA-Komplexen wurden gleiche Volumina von Lösungen von pDNA und PEI vereinigt. Unter Schütteln wurde DNA der PEI-Lösung zugesetzt. Die Menge von PEI wurde so gewählt, daß sich Stickstoff-zu-Phosphat-Verhältnisse (N/P-Verhältnis) von 8:1 bzw. 10:1 ergaben. Dieses Verhältnis ist das molare Verhältnis von Stickstoff-Atomen im PEI zu den Phosphaten (= negative Ladungen) der Nucleotide der DNA.

Berechnung:

$$N/P = \frac{(\mu g PEI)}{43} \times \frac{330}{(\mu g DNA)}$$

(330 = mittleres MW eines Nucleotids; 43 = MW der sich wiederholenden Grundeinheit von PEI unter Berücksichtigung der primären Amine).

Beladung der Schwämme mit PEI / DNA-Komplexen:

Auf 4.5 x 5 cm große Tachotop- oder Resorba-Schwämme wurden 5 ml PEI / DNA-Komplex-Lösungen mit 250 µg, 375 µg oder 500 µg DNA und N / P-Verhältnissen von 8 oder 10 aufpipettiert. Die 250 µg DNA pro 4.5 x 5 cm entsprechen 10 µg DNA pro $cm^2$, die 375 µg DNA auf 4.5 x 5 cm entsprechen 15 µg DNA pro $cm^2$ und die 500 µg DNA auf 4.5 x 5 cm entsprechen 20 µg DNA pro $cm^2$. Nach 24 h Inkubation bei 4 °C wurden die Präparate gefriergetrocknet und steril in ca. 1,5 x 1,5 cm Stücke zerschnitten (entsprechend 22,5 µg, 34 µg oder 45 µg DNA). Solche Präparate wurden wie in Beispiel 19 zum Gentransfer in vitro eingesetzt. Fig. 15 zeigt hohe Genexpression. Die Genexpression wurde über mehrere Wochen verfolgt. Dabei wurde eine Zunahme der Expression am Schwamm beobachtet (nicht gezeigt).

(c) Liposomen / DNA-Schwämme

Herstellen von kationischen Liposomen aus DOTAP-Pulver:

In einem silanisierten 15 ml Reagensglas mit Schraubverschluß wurde eine 5 mM DOTAP / Chloroform-Lösung hergestellt. Mit einem Rotationsverdampfer (Rotavapor-R, Firma Büchi, Schweiz) wurde das Chloroform abgedampft, so daß sich an der. Röhrchen-Innenwand ein gleichmäßiger Lipidfilm bildete. Der Rotationsverdampfer wurde mit Argon-Gas belüftet, um Sauerstoff auszuschließen. Das Röhrchen wurde über Nacht in das Vakuum des Lyophilisators gestellt. Der Lipidfilm wurde daraufhin mit 15 ml einer 5 % Glucoselösung rehydratisiert, wobei zuerst etwa 30 Sekunden gevortext wurde und dann 30 min mit Ultraschall (Sonifikator: Sonorex RK 510 H, Fa. Bandelin) behandelt wurde, wobei eine stabile Liposomensuspension entstand.

Herstellung von DOTAP-Lipoplexen:

Für einen 4.5 x 5 cm Schwamm werden 222 µg DNA benötigt, um 20 µg DNA auf 1,5 x 1,5 cm zu erhalten. Das Ladungsverhältnis ($^+/_-$) sollte 5:1 sein, wobei die positiven Ladungen vom DOTAP und die negativen Ladungen von der DNA kommen. 222 µg DNA entsprechen 0,67 µmol negative Ladungen. In einem Polystyrol-Röhrchen wurden 3,35 µmol DOTAP-Liposomen mit 5 % Glucose-Lösung auf ein Volumen von 2,5 ml aufgefüllt. Dazu wurden 222 µg DNA, ebenfalls in 2,5 ml 5 % Glucose-Lösung, unter leichtem Schütteln gegeben.

Applikation von DOTAP-Lipoplexen auf den Schwamm:

5 ml der oben hergestellten Liposomen / DNA-Lösung wurden unter sterilen Bedingungen gleichmäßig auf einen 4.5 x 5 cm Tachotop-Schwamm pipettiert. Nach 24 h Inkubation bei 4 °C wurde der Schwamm lyophilisiert und anschließend in 1,5 x 1,5 cm Stücke geschnitten.

(d) DNA / DOTAP-Schwämme

Auf einen 4.5 x 5 cm Tachotop-Schwamm wurden 500 µg DNA (pCMVLuc) in 5 ml 5 % Glucose-Lösung steril aufpipettiert, 24 h bei 4 °C inkubiert und dann lyophilisiert. Dies entspricht 20 µg DNA pro 1 x 1 cm bzw. 45 µg DNA pro 1,5 x 1,5 cm.

In einem Vorversuch wurde durch Aufbringen einer 0,01 % Methylviolett-Chloroform-Lösung auf einen Kollagenschwamm gezeigt, daß die gesamte Schwammstruktur von der Lösung gleichmäßig benetzt wurde. Daraus wurde geschlossen, daß dies auch für eine Lipidlösung in Chloroform möglich sein sollte. Das angestrebte Ladungsverhältnis sollte 5 sein. Für 500 µg DNA ergibt das eine Menge von 7,6 µmol DOTAP. Dementsprechend wurden 5 ml einer 1 mg/ml DOTAP-Chloroformlösung auf den Schwamm aufgebracht. Anschließend wurde der Schwamm bei -20 °C inkubiert dann über Nacht bei Raumtemperatur (damit das Chloroform verdampfen konnte)

und steril in ca. 1,5 x 1,5 cm Stücke geschnitten.

(e) DOTAP / DNA-Schwämme

Das angestrebte Ladungsverhältnis war wiederum 5:1. Auf einen 4.5 x 5 cm Tachotop-, Tissu Vlies- bzw. Resorba-Schwamm wurden 5 ml einer 1 mg DOTAP / ml-Lösung in Chlorofrom aufpipettiert, ca. 1 h bei -20°C inkubiert und bei Raumtemperatur das Chloroform über Nacht verdampft. Pro 4.5 x 5 cm Schwamm wurden 500 µg DNA (pCMVLuc) in 5 ml 5 % Glucose-Lösung aufpipettiert, 24 h bei 4 °C inkubiert und anschließend lyophilisiert. Dies entspricht 20 µg DNA pro $cm^2$.

(f) DOTAP-Cholesterin / DNA-Schwämme

Das angestrebte Ladungsverhältnis ($^+/_-$) war wieder 5:1, die angestrebte Belegung mit DNA 20 µg pro $cm^2$. Es wurden also 5 mg DOTAP und 2,95 mg Cholesterin (das sind je 305 nmol) in je 2,5 ml Chloroform gelöst und anschließend vereinigt. Diese Lösung wurde auf einen 4.5 x 5 cm Tachotop-Schwamm aufpipettiert, 1 h bei -20 °C inkubiert und das Chloroform bei Raumtemperatur verdampft. 500 µg DNA (pCMVLuc) in 5ml 5 % Glucose-Lösung wurden auf den 4.5 x 5 cm-Schwamm pipettiert, 24 h bei 4 °C inkubiert und lyophilisiert. Der Schwamm wurde dann in 1,5 x 1,5 cm-Stücke geschnitten.

Variante:

Es wurden 180 ml einer Lösung von DOTAP:Cholesterin = 1:0.9 von einer Konzentration von 2 mM Gesamtlipid in Chloroform hergestellt. Tachotop-Schwämme (Nycomed) wurden halbiert (= 4.5 x 5 cm) und in je 30 ml dieser Lösung in einem 50 ml Polypropylenröhrchen mit Schraubverschluß eingelegt und insgesamt 2 Stunden am Schüttelinkubator inkubiert. Zwischendurch wurden die Röhrchen mit geöffnetem Deckel kurzzeitig im Lyophilisator leicht evakuiert ("vacuum loading"), sodass sich die Schwämme gänzlich mit der Chloroformlösung durchtränkten. Die Schwämme wurden schließlich aus dem Chloroformbad in Glas-Petrischalen gelegt und im Vakuum über Nacht getrocknet. 500 µg DNA (pCMVLuc) in 5ml 5 % Glucose-Lösung wurden pro 4.5 x 5 cm-Schwamm aufgeträufelt, 4 h bei Raumtemperatur inkubiert und lyophilisiert. Der Schwamm wurde dann in 1,5 x 1,5 cm-Stücke geschnitten. Solche Präparate wurden wie in Beispiel 19 zum Gentransfer in vitro eingesetzt.

Dabei wird anfänglich hohe Expression von Zellen in der Kulturschale beobachtet, die schnell abnimmt, während die Expression am Schwamm gleichbleibt und dann lange erhalten bleibt. Fig. 15

(g) DNA / PEI-SH-SPDP-Schwämme

(i) Kovalente Bindung von PEI an Schwämme:

0,5 ml einer 15,5 mM SPDP-Lösung in abs. Ethanol wurden zu 2 ml 0,1M HEPES pH = 7,9 gegeben, gemischt, auf 4.5 x 5 cm Tachotop-Schwämme pipettiert und über Nacht bei 37°C inkubiert. Die Amino-Gruppen von Lysinen im Kollagen reagieren dabei in einer nucleophilen SubstitutionsReaktion mit der Carboxylgruppe des aktivierten Esters im SPDP.

Mit Aqua dest. wurde von den Schwämmen (in 14 ml Falcon-Röhrchen, Fa. Becton Dickinson, USA) das ungebundene SPDP quantitativ ausgewaschen, bis im Überstand zwischen 200-400 nm photometrisch keine Absorption mehr zu messen war. Anschließend wurden die Schwämme lyophilisiert und in ca. 1,5 x 1,5 cm Stücke geschnitten. Die Schwammstücke wurden gewogen (mit einer MC 1 research Waage, Fa. Sartorius, Göttingen). Zur Bestimmung der gekoppelten Menge SPDP wurde ein Schwammstückchen mit 2 ml HBS und 3 µl β-Mercaptoethahol inkubiert. Das dabei freiwerdende Thiopyridon wurde photometrisch bei 342 nm bestimmt (ε = 8080 l / mol). Die Belegung berechnet sich nach:

$$Belegung_{(nmol\ /\ mg)} = \frac{E_{342} \times Vol_{(ml)} \times 10^6}{\varepsilon_{(l\ /\ mol)} \times Gewicht_{(mg)}}$$

Durchschnittlich war die Belegung etwa 20 nmol SPDP / mg Kollagen. Es wurden aber auch Schwämme mit 0,45 nmol SPDP / mg Kollagen präpariert.

(ii) Derivatisierung von PEI mit Iminothiolan (Traut's Reagens).

Um PEI über eine Disulfidbindung ans SPDP und somit an den Schwamm kovalent binden zu können, muß in PEI eine Thiolgruppe eingeführt werden. Dies erfolgte durch Kopplung von 2-Iminothiolan an PEI.

PEI wurde unter Argonspülung mit einem zweifachen molaren Überschuß Iminothiolan versetzt. Es wurde 1/15 Volumen 1 M HEPES pH= 7,9 zugegeben und anschließend erfolgte die Reaktion bei Raumtemperatur für ca. 20 min. Überschüssiges Reagens wurde durch mehrmalige Zentrifugation in Centricon 30-Röhrchen abgetrennt. Die freien Thiolgruppen am PEI wurden mittels Elman-Reagens bestimmt.

(iii) Kopplung des PEI-Iminothiolanderivats an SPDP-Kollagenschwämme Zu den SPDP-Schwammstücken wurde ein 5-10 facher Überschuß (bezüglich des Verhältnisses der freien Thiolgruppen am PEI zu den Thiopyridylgruppen am Schwamm) PEI gegeben. Nach 7 Tagen bei Raumtemperatur war die Reaktion beendet. Dies wurde durch photometrische Bestimmung der Extinktion bei 342 rim festgestellt. 100 % des SPDP am Schwamm hatten mit PEI-SH reagiert.
Die Menge gekoppelten Polyethylenimins berechnet sich zu:

$$Belegung_{(nmol\ /\ mg)} = \frac{E_{342} \times Vol_{(ml)} \times 10^6}{\varepsilon_{(l\ /\ mol)} \times Gewicht_{(mg)}}$$

Die Schwämme wurden solange in Wasser gewaschen, bis im Überstand bei 342 nm keine Extinktion mehr zu messen war. Dann wurden sie gefriergetrocknet.

(iv) Applikation von DNA auf PEI-SH-SPDP-Schwämme
20 µg DNA (pCMVLuc) in 500 µl 5 % Glucose-Lösung wurden je 1,5 x 1,5 cm Schwammstück aufpipettiert, 24 h bei 4°C inkubiert und dann lyophilisiert.

(v) DNA / Peptid-SPDP-Schwämme
Schwämme wurden wie beschrieben mit SPDP beladen. Eine durchschnittliche Belegung von 20 nmol SPDP / mg Kollagen wurde erzielt. Es wurden aber auch Schwämme mit 12,8 nmol SPDP / mg Kollagen hergestellt. Peptid SFO7-SH mit der Sequenz $(KKKK)_2KGGC$ wurde in zweifachem molarem Überschuß über die SPDP-Gruppen in 300 µl 0,1 M HEPES pH = 7,9 auf den Schwamm aufgebracht. Die Reaktion erfolgte in einem 14 ml Falcon-Röhrchen, wobei der Luftraum des Röhrchens kurz mit Argon gespült wurde. Nach 2 Tagen bei Raumtemperatur war die Reaktion zu 60 % abgeschlossen. Dies stellte man durch Bestimmung der Extinktion bei 342 nm des Überstandes fest (Bestimmung freigesetzten Thiopyridons). Die Berechnung der Menge gebundenen Peptids erfolgte wie für PEI beschrieben.
Die Peptid-SPDP-Schwämme wurden noch solange mit Aqua dest. gewaschen, bis im Waschüberstand keine Extinktion bei 280 nm mehr meßbar war. Anschließend wurden sie gefriergetrocknet.

(vi) Aufbringen von DNA auf Peptid-SPDP-Schwämme
20 µg DNA (pCMVLuc) in 500 µl 5 % Glucose-Lösung wurden je 1,5 x 1,5 cm Schwammstück aufpipettiert, 24 h bei 4 °C inkubiert und dann lyophilisiert.

**BEISPIEL 17: Subcutane Implantation in Wistar Ratten und Bestimmung der Reportergenexpression**

(a) Versuchstiere
Als Versuchstiere werden 7 zwei Monate alte, männliche Wistar-Ratten (Charles-River Deutschland GmbH, Sulzfeld) mit Körpergewicht von 300-400g verwendet.
Die Ratten leben gruppenweise in Makrolon-Typ-4-Käfigen mit einer maximalen Belegdichte von 5 Tieren. Den Tieren stehen Alleinfutterpellets für Ratten und Mäuse "Altromin 1324, Haltungsdiät für Ratten und Mäuse" der Fa. Altromin, Lage/Lippe, Deutschland, und Wasser aus Nippeltränken ad libitum zur Verfügung. Die Haltung erfolgt auf entkeimtem und entstaubtem Weichholzgranulat (Fa. Altromin), das zweimal wöchentlich gewechselt wird. Die Tiere sind gemäß den Vorgaben zur Versuchstierhaltung in speziellen Tierhaltungsräumen einer konventionellen Tierhaltung bei einer Raumtemperatur von 20-25°C mit gleichbleibendem Luftwechsel untergebracht. Die relative Luftfeuchte beträgt 60 % -70 %. Beleuchtung: jeweils 12 Stunden dauernde Hell-Dunkel-Phase. Die Lichtintensität beträgt 50-100 lux. Die Tiere befinden sich mindestens 2 Wochen vor Versuchsbeginn im institutseigenen Tierstall und werden vor der OP nicht nüchtern gesetzt.

(b) Schwammimplantation

(i) Materialien:

- Narkosegerät (MDS Matrx-Narkosegerät) mit Isofluran (Abbot GmbH, Wiesbaden, Deutschland):
  Es handelt sich um ein Kreissystem mit Ventilator, der verbrauchte Luft weg- und neue zuführt. Die Vorteile sind eine Dauerinhalation bei chirurgischer Toleranz ohne Einsatz von injektionsnarkotika und eine gute

Steuerbarkeit der Anästhesietiefe.
Es ist keine Prämedikation notwendig und das Tier wacht nach der Narkose innerhalb von wenigen Minuten auf.

- durchsichtige Ganzkörperkammer aus Plexiglas mit Deckel
- Kopfkammer
- Heizkissen (auf Stufe 2 - 38°C)
- grüne Abdecktücher für den OP-Tisch bzw. für die Ratte
- Schermaschine
- Hautdesinfiziens (Cutasept ® F, Fa. Bode Chemie, Hamburg, Deutschland)
- Bepanthen®Roche Augensalbe (Fa. Hoffmann-La Roche AG, Grenzach-Wyhlen, Deutschland)
- wasserfester Folienstift zur Kennzeichnung der Ratten
- sterile Einmalhandschuhe
- steriles OP-Besteck bestehend aus:

  1 anatomische Pinzette
  1 chirurgische Pinzette
  1 Lexer-Schere mit spitzem auf stumpfen Schenkel
  1 gebogene Metzenbaum-Schere (spitz/spitz)
  1 NadelhalterGaze-Tupfer

- Nahtmaterial: monofiler, blauer, 45 cm langer, 4/0er Prolene® Faden mit spitzer, angeschweißter Nadel
- sterile 15er Einmalskalpelle
- 14 numerierte und abgewogene Schwämme für eine Versuchsgruppe (7 Tiere)

(ii) Operationsdurchführung:
Die Tiere werden ca. 15 Minuten vor OP-Beginn in den OP-Bereich verbracht, um ihnen eine Adaptation an die neue Umgebung zu ermöglichen. Die an das Narkosegerät angeschlossene Ganzkörperkammer wird etwa 2 Minuten vor Narkoseeinleitung mit 350 $cm^3$/min. Sauerstoff und 4 % Isofluran-Anteil vorgeflutet, damit eine entsprechende Konzentration erreicht ist, um anschließend bei der Ratte eine möglichst schnelle und somit schonendere (kurzes Exzitationsstadium) Narkoseeinleitung zu ermöglichen. Die Ratte wird in die Ganzkörperkammer gesetzt und die initiale Konzentration der Inhalationsgase wird solange aufrecht erhalten, bis - nach 1-2 Minuten - ein Verlust der Stellreflexe (Ratte bleibt auf dem Rücken liegen) eintritt und somit das Narkosestadium III, 1-2 erreicht ist. Die Ratte wird aus der Kammer herausgenommen, in Ventrallage gebracht und mit der Kopfkammer versehen. Befindet sich das Tier im Narkosestadium III,2, dem Stadium der chirurgischen Toleranz (der Zwischenzehenreflex sollte hier negativ sein), wird die Isofluranzufuhr auf 1,5 % reduziert. Auf beide Augen wird eine fettende Augensalbe aufgetragen, um, aufgrund der Aufhebung des Palpepralreflexes, eine Austrocknung der Cornea zu verhindern. In der Regio lumbalis (in der Dorsalregion zwischen letzter Rippe und Hintergliedmaße) wird mit der Schermaschine großzügig ein ca. 7 cm breiter und 2 cm langer Bereich ausrasiert; anschließende Reinigung und Desinfektion dieser Hautstellen mit einem mit Cutasept besprühten Gazetupfer. Die Haut wird rechts ca. 2 cm von der Medianen entfernt mit der chirurgischen Pinzette erfaßt und mit einem Skalpell in dorsoventraler Richtung auf einer Länge von etwa 1 cm inzidiert; mit einer Metzenbaum-Schere wird der Hautschnitt stumpf erweitert und das Subkutangewebe ca. 3 cm nach cranial vorsichtig unterminiert. Der craniale Wundrand wird mit der chirurgischen Pinzette aufgehalten und der vorbereitete Schwamm wird mit der anatomischen Pinzette soweit wie möglich nach cranial in das getunnelte Gewebe vorgeschoben. Der Hautschnitt wird mit einem U-Heft verschlossen. Der gleiche Vorgang wird auf der linken Seite wiederholt (s. 5.-7.). Die Isofluranzufuhr wird beendet, während die $O_2$-Perfusion noch erhalten bleibt. Nach Rückkehr des Schluckreflexes werden dem Tier per os ca. 0,1 ml Novalgin® (Wirkstoff: Metamizol-Natrium, Fa. Hoechst AG, Frankfurt, Deutschland) als nichsteroidales Analgetikum verabreicht. Die Ratte wird bis zum Wiedererlangen des vollen Bewußtseins in einen Einzelkäfig verbracht und nach etwa einer Stunde in ihren Käfig zurückgegeben.

(c) Schwammentnahme

(i) Materialien:

- Narkosegerät (MDS Matrx-Narkosegerät) mit Isofluran (Abbot GmbH, Wiesbaden, Deutschland)
- durchsichtige Ganzkörperkammer aus Plexiglas mit Deckel
- Kopfkammer

- sterile Einmalhandschuhe
- steriles OP-Besteck (s.o.)
- 1000 ml isotonische NaCl-Infusionslösung (Fa. Delta-Pharma GmbH, Pfullingen, Deutschland), die mit 50000 I.E. Heparin (2 x 5 ml Injektionslösung Heparin-Na zu je 25000 I.E. der Fa. ratiopharm® GmbH, Ulm/Donautal, Deutschland) versetzt ist
- Infusionsschlauch
- Butterfly-Kanüle 19 G

Die Tiere werden vor der Schwammentnahme perfundiert, um ein möglichst blutarmes Gewebe zu erhalten, damit für die spätere Luciferase-Bestimmung die Anzahl der potentiellen Störfaktoren (z.B. Hämoglobin) möglichst gering gehalten wird. 2 ml fassende Homogenisierröhrchen mit Schraubdeckel (disposable/conical 2,0 ml screw cap tube with cap, VWR scientific products, West Chester, USA), die bis zur 0,3 ml Markierung mit großen Homogenisierkügelchen (Zirconia Beads, 2,5 mm Dia, Biospec Products, Inc., Bartlesville, USA) und je 750 µl Lyse-Puffer für Tierversuche (10 ml 5 x Reporter Lysis Buffer (Promega Corporation, Madison, USA)+ 40 ml dd $H_2O$ + 1 Tablette Protease-Inhibitor(Complete™, Boehringer Mannheim GmbH, Deutschland) befüllt sind (zur Aufnahme der entnommenen Schwämme).

(ii) Durchführung:
Die Ratte wird, wie unter Schwammimplantation 1.-2. beschrieben, vorbereitet und in Narkose gelegt. Das Tier wird in Dorsallage verbracht. Die Bauchhöhle wird durch einen medianen präumbilical beginnenden und bis zum Manubrium sterni reichenden Scherenschlag eröffnet. Entlastungsschnitte rechts und links entlang der letzten Rippe. Die Vena cava caudalis wird freigelegt und eine Butterfly-Kanüle caudal der Einmündung der Venae renales gelegt. Die Infusionslösung wird angeschlossen; nach Durchfluß von ca. 5 ml wird die Vena cava caudalis caudal der Punktionsstelle mit einem Skalpell eröffnet. Das Tier wird solange mit 100 - 150 ml der Infusionslösung perfundiert bzw. entblutet, bis eine deutliche Entfärbung der Leber erkennbar ist. Die Ratte wird in Ventrallage verbracht. Inzision der Haut in der Medianen mit einem Skalpell, die beginnend von der Lendengegend bis ca. 7 cm nach cranial reicht; Entlastungsschnitte rechts und links caudal der Implantationswunden. Die Schwämme werden mit der Schere bzw. dem Skalpell weitgehend freipräpariert und mit umliegendem Gewebe (Bindegewebe und ein ca. 1 cm großes Muskelstück des Musculus longissimus dorsi) entnommen. Jeder entnommene Schwamm (mit umliegendem Gewebe) wird in 1 x PBS-Puffer gewaschen; Schwamm und Gewebe werden nun getrennt in die vorbereiteten und beschrifteten Homogenisierröhrchen gegeben. Die gefüllten Röhrchen werden dann auf Eis gestellt und möglichst sofort weiterverarbeitet.

(iii) Probenaufbereitung:
Die ständig auf Eis zu haltenden Proben werden im Mini-beadbeater® (Fa. Biospec Products, Inc., Bartlesville, USA) 3 x 20 Sekunden homogenisiert. Anschließende Zentrifugation bei 14000 Umdrehungen, 10 Minuten und 4°C.
Luciferasetest:
pro Röhrchen werden 20 µl aus dem Überstand entnommen und in verschiedene wells einer Costar® 96-well-plate (opaque Plate-solid black 96 well, Corning Costar Corporation, Cambridge, USA) einpipettiert. Pro well werden 100 µl Luciferase-Puffer (Promega Luciferase Assay System, Promega Corporation, Madison, USA) zugegeben und bei 'count delay' von einer Minute über 12 sec gemessen.

Die Resultate der vorangehend beschriebenen in vivo Versuche sind in der folgenden Tabelle dargestellt.

| PEI-DNA N/P = 8 + 2 Äquiv. P6YE5C | | Nackte DNA | | DOTAP/Cholesterin-DNA Ladungsverh. 5:1 | |
|---|---|---|---|---|---|
| Linker Schwamm | Rechter Schwamm | Linker Schwamm | Rechter Schwamm | Linker Schwamm | Rechter Schwamm |
| 214.30 | 80.44 | 0 | 0 | 0 | 0 |
| 212.17 | 90.53 | 0 | 0 | 0 | 0 |
| 40.69 | 45.67 | 0 | 0 | 0 | 0 |
| 169.50 | 91.69 | 0 | 0 | 0 | 0 |
| 18.90 | 16.51 | 0 | | 0 0 | 0 |
| 475.44 | 72.68 | 0 | 0 | 0 | 0 |

(fortgesetzt)

| PEI-DNA N/P = 8 + 2 Äquiv. P6YE5C | | Nackte DNA | | DOTAP/Cholesterin-DNA Ladungsverh. 5:1 | |
|---|---|---|---|---|---|
| Linker Schwamm | Rechter Schwamm | Linker Schwamm | Rechter Schwamm | Linker Schwamm | Rechter Schwamm |
| 0.00 | 0.00 | 0 | 0 | 0 | 0 |

Die Tabelle zeigt den Gentransfer in vivo bei subcutaner Implantation von Schwammpräparaten. Die Schwämme wurden wie in Beispiel 15 bzw. 16 beschrieben präpariert und wie in Beispiel 16 beschrieben subcutan in Wistar-Ratten implantiert. Die Genexpression wurde zunächst nach drei Tagen bestimmt. Lediglich Kollagenschwämme, die mit PEI-DNA Vektoren beladen sind, die mit einem erfindungsgemäßen Copolymer umhüllt sind, führen in diesem Versuchsaufbau zu detektierbarer Reportergenexpression (Zahlenangaben in fg Luciferase / mg Protein).

**BEISPIEL 17: Freisetzung radioaktiv markierter DNA aus diversen Kollagenschwamm-Vektor-Präparaten**

(a) Radioaktive Markierung von Plasmid-DNA durch Nick Translation

Es wurde der Nick Translation Kit der Firma Amersham (# N5500) verwendet. Pro Markierungsansatz wurde 1 µg DNA (pCMVβGal) eingesetzt. Das Protokoll des Herstellers wurde dahingehend verändert, daß die Reaktionszeit 15 min bei 15 °C, statt wie die für lineare DNA angegebenen 2 h bei 15 °C, betrug. Als Nucleosidiriphosphat wurde [α-$^{32}$P] dATP mit einer spezifischen Aktivität von 3000 Ci / mmol (Fa. Amersham, Freiburg) verwendet. Die Abtrennung des nichteingebauten [α-$^{32}$P] dATP erfolgte mit den Säulen "Nuc Trap Probe Purification Columns" und der mit Plexiglas geschützten Fixierungs-Apparatur "Push Column Beta Shield Device" (beides Fa. Stratagene, Heidelberg) nach dem Prinzip der Gelfiltration nach Vorschrift des Herstellers. Das resultierende Plasmid wurde mittels Agarosegelelektrophorese überprüft (1 % Agarosegel, 100 V, 35 min, Ethidiumbromid-Färbung). Es wurde gemischt mit unmarkiertem Plasmid aufgetragen und nach Elektrophorese und Geltrocknung im UV-Licht sowie durch Autoradiographie sichtbar gemacht. Dies erlaubt eine Größen- und Anteilsabschätzung der beim Nick-Labeling entstehenden Plasmidfragmente. Um die radioaktiv markierte DNA von Enzymen zu reinigen, verwendete man das "Promega Wizard ™ PCR Preps DNA Purification System" (Fa. Promega, USA) bei geringfügiger Modifikation des Herstellerprotokolls betreffend der apparativen Ausrüstung.

(b) Präparation von chemisch modifizierten Schwämmen mit radioaktiv markierter DNA

(i) DOTAP / DNA-Tachotop-Schwämme

Es wurden ca. 1,5 x 1,5 cm Tachotop-Schwämme zurechtgeschnitten und gewogen. Das durchschnittliche Gewicht betrug 5 mg. Dann wurden 450 .. µl einer 1 mg DOTAP / ml Chloroform -Lösung aufpipettiert, 1 h bei -20 °C inkubiert, das Chloroform bei Raumtemperatur verdampft und die Schwämme erneut gewogen. Diese DOTAP-Schwämme wurden in Vertiefungen von 6-Kalotten-Platten gelegt und ein Gemisch von 20 µg (einmal auch 40 µg) unmarkiertes Plasmid und 10 µl bzw. 30 µl Produkt der Radioaktivmarkierung pro 5 mg Schwamm in einem Gesamtvolumen von 200 µl 5 % Glucose-Lösung aufpipettiert, 2-24 h bei 4 °C inkubiert und lyophilisiert.

(ii) DNA-Tachotop-Schwämme

Methode 1: Es wurden ca. 1,5 x 1,5 cm Tachotop-Schwämme zurechtgeschnitten und gewogen. Die Schwämme wurden in Vertiefungen von 6-Kalotten-Platten gelegt und 20 µg unmarkierte Plasmid-DNA / 5 mg Schwamm und 10 bzw. 30 µl radioaktiv markierte DNA (in insgesamt 200 µl 5 % Glucose-Lösung) aufpipettiert, 2-24 h bei 4 °C inkubiert und lyophilisiert.

Methode 2:

Auf einen 4.5 x 5 cm Tachotop-Schwamm wurden 500 µg unmarkierte Plasmid-DNA und 122,1 µl radioaktiv markierte DNA (in insgesamt 2 ml 5 % Glucose-Lösung) aufpipettiert, 12 h bei 4 °C inkubiert und lyophilisiert. Der Schwamm wurde dann in 1,5 x 1,5 cm-Stücke geschnitten und jedes Stück abgewogen. Um festzustellen, wieviel der eingesetzten DNA beim Gefriertrocknungsschritt der Schwammpräparation in der Zellkulturschale verblieben war, wurden der Dekkel und der Boden mit je 2 ml 10 x SDS versetzt und Aliquote von je 40 µl

vermessen.

(iii) DOTAP / Cholesterin / DNA-Tachotop-Schwämme
Das angestrebte Ladungsverhältnis ($^{+}/_{-}$) war wieder 5:1, die angestrebte Belegung mit DNA 20 µg pro $cm^2$. Es wurden also 5 mg DOTAP und 2,95 mg Cholesterin (das sind je 305 nmol) in je 2,5 ml Chloroform gelöst und anschließend vereinigt. Diese Lösung wurde auf einen 4.5 x 5 cm Tachotop-Schwamm aufpipettiert, 1 h bei -20 °C inkubiert und das Chloroform bei Raumtemperatur verdampft. 500 µg unmarkierte Plasmid-DNA und 122,1 µl radioaktiv markierte DNA (in insgesamt 2ml 5 % Glucose-Lösung) wurden auf den 4.5 x 5 cm-Schwamm pipettiert, 24 h bei 4 °C inkubiert und lyophilisiert. Der Schwamm wurde dann in 1,5 x 1,5 cm-Stücke geschnitten und jedes Stück abgewogen. Um festzustellen, wieviel der eingesetzten DNA beim Gefriertrocknungsschritt der Schwammpräparation in der Zellkulturschale verblieben war, wurden der Deckel und der Boden mit je 2 ml 10 x SDS versetzt und Aliquote von je 40 µl vermessen.

(iv) Polyethylenimin / DNA-Tachotop-Schwämme
Auf einen 4.5 x 5 cm großen Tachotop-Schwamm wurden 2 ml PEI / DNA-Komplex-Lösungen (mit 500 µg unmarkierter Plasmid-DNA und 122,1 µl radioaktiv markierter DNA bei einem N / P-Verhältniß von 6) aufpipettiert. Die 500 µg DNA pro 4.5 x 5 cm entsprechen 20 µg DNA pro $cm^2$. Nach 24 h Inkubation bei 4 °C wurden die Präparate gefriergetrocknet und in ca. 1,5 x 1,5 cm Stücke zerschnitten und jedes Stück abgewogen. Um festzustellen, wieviel der eingesetzten DNA beim Gefriertrocknungsschritt der Schwammpräparation in der Zellkulturschale verblieben war, wurden der Dekkel und der Boden mit je 2 ml 10 x SDS versetzt und Aliquote von je 40 µl vermessen.

(v) DNA-Peptid-SPDP-Schwämme wurden wie in Beispiel 16 beschrieben hergestellt, mit der Ausnahme, daß die DNA-Komponente radioaktiv markierte DNA enthielt, wie oben beschrieben.

(vi) Copolymer-geschützte Polyethylenimin/DNA-Tachotop-Schwämme Derartige Schwämme wurden wie in Beispiel 15 beschrieben hergestellt mit der Ausnahme, daß die Plasmid-DNA-Lösung zusätzlich radioaktiv markierte DNA enthält.

(c) Nachweis der zeitabhängigen Freisetzung von radioaktiv markierter DNA aus den Schwämmen
Die verschiedenen Schwammpräparate wurden in silanisierten Glasröhrchen (16 x 100 mm Kulturröhrchen mit Schraubkappe aus AR-Glas, Fa. Brand, Deutschland) mit 1 ml PBS versetzt, kurz bei 3000 rpm zentrifugiert (Zentrifuge: Megafuge 2.0 R, Fa. Heraeus, München) und dann bei 37 °C in einem Wasserbadschüttier bei 80 oder 120 rpm geschüttelt. Nach 1 h, 1 Tag, 3 Tagen und weiterhin alle 3 Tage wurde die Menge an radioaktiver DNA im Überstand bestimmt. Dazu wurden die Röhrchen bei 3000 rpm zentrifugiert, kurz geschüttelt, 40 µl Überstand entnommen und durch 40 µl PBS ersetzt. Die Proben wurden mit 160 µl Microscint 20 high efficiency LSC-cocktail (Fa. Packard, USA) in einer Vertiefung einer weißen 96-Kalotten-Opaque-Platte (Typ "flat bottom, non-treated", Fa. Costar, USA) versetzt und mittels Top Count-Gerät (Canberra-Packard, USA) bei automatischer Korrektur für die Halbwertszeit vermessen. Die Meßzeit betrug 5 min:, die Meßverzögerung 10 min., und es wurde aus 3 Messungen der Mittelwert gebildet. Als Referenz wurden 2 µl der markierten Plasmid-DNA vermessen. Die gemessene Konzentration an DNA (cpm/ml) wurde bezüglich der bereits entnommenen Proben korrigiert (bereits entnommene Mengen wurden aufsummiert und zum gemessenen Wert addiert). Um festzustellen, wieviel der eingesetzten DNA beim Gefriertrocknungsschritt der Schwammpräparation in den Zellkulturschalen verblieben war, wurden diese mit 500 µl PBS versetzt, von denen Aliquote von 40 µl vermessen wurden. Am Ende einer Meßreihe (z.B. nach 30 Tagen Inkubation) wurden die Schwämme mit einer 1 % SDS-Lösung behandelt, um festzustellen, ob 100 % der eingesetzten Dosis wiedergewonnen werden könnten. Dazu wurden die Schwämme in frische Falcon Röhrchen transferiert, 1ml 1 % SDS zupipettiert, 1 Tag inkubiert und mehrmals stark geschüttelt. Dann wurden von diesem Überstand 40 µl entnommen, mit 160 µl Microscint 20 in einer Vertiefung einer weißen 96-Kalotten Opaque Platte gegeben und die Radioaktivität (cpm) im "Top Count" gemessen.
Die Ergebnisse sind in Fig. 14 dargestellt.
Mit nackter DNA beladene Schwämme setzen innerhalb einer Stunde ca. 50% der eingesetzten Dosis frei, gefolgt von einer in etwa linearen protrahierten Freisetzung: Dagegen zeigen vektorbeladene Schwämme geringere anfängliche Freisetzung von nicht mehr als 5% gefolgt von einer lange anhaltenden geringen Freisetzung pro Zeiteinheit. Dies bedeutet effiziente Bindung der untersuchten Vektoren an die Kollagenmatrix.

(d) Agarosegelelektrophorese zur Charakterisierung freigesetzter DNA
Nach 5 bzw. 30 Tagen Schütteln der DOTAP / DNA-Schwämme in 1 ml PBS wurden je 20 µl vom Überstand entnommen und auf einem kleinen Ethidium-Bromid gefärbten 1 % Agarose-Gel für 35 min. bei 100 V laufen

gelassen. Zur Kontrolle wurden 1 µg unmarkierte Plasmid-DNA und Liposomen / DNA-Komplexe (Ladungsverhältnis 5:1) aufgetragen. Das Gel wurde im UV-Licht fotografiert, dann getrocknet und dann auf einem Röntgenfilm exponiert.

**BEISPIEL 19: Transfektion von NIH 3T3-Zellen durch/auf vektorbeladenen Kollagenschwämmen In vitro**

In Zellkulturplatten (6-Loch-Platten der Fa. TPP) werden pro Vertiefung ca. 50.000 bis 400.000 trypsinisierte NIH 3T3 Mäusefibroblasten (adhärent) in 4 ml DMEM-Medium (Dulbecco's Modified Eagles Medium) unter Zusatz von Antibiotika (500 Einheiten Penicillin, 50 mg Streptomycin/500 ml) sowie 10 %igem fötalem Kälberserum sowie 1,028 g/1 N-Acetyl-L-Alanyl-L-Glutamin ausgesät und ein bis zwei Tage in einer Atmosphäre (Luft) mit 5 % Kohlendioxid bei 37°C inkubiert. In einer angemessenen Anzahl von Vertiefungen werden ein bis zwei Tage nach dem.Aussäen je ein nach Beispiel 15 bzw. 16 präparierter Kollagenschwamm (1,5 x 1,5 cm) gelegt, und ca. drei Tage bei 37°C in einer Atmosphäre mit 5 % Kohlendioxid inkubiert. Die erste Messung der Lüciferase-Expression erfolgt in einem Zeitraum von ein bis drei Tagen. Dazu werden die Vertiefungen mit den adhärenten Zellen nach Entnahme des Kollagenschwamms dreimal mit einer Phosphat-Pufferlösung (PBS) gewaschen und anschließend mit 500 µl Lyse-Puffer (0,1 % Triton in 250 mM Tris, pH = 7,8) behandelt. Anschließend wird die Luciferase-Aktivität gemessen, wie unten angegeben.
Um die protrahierende Wirkung nachzuweisen, werden die entnommenen Kollagenschwämme erneut in frische Vertiefungen mit ausgesäten Zellen gelegt und ca. drei Tage bei 37°C in einer Atmosphäre mit 5% Kohlendioxid inkubiert Danach werden die Kollagenschwämme aus den Vertiefungen genommen, die adhärenten Zellen in gleicher Weise gewaschen und mit Lyse-Puffer behandelt, wie vorstehend angegeben, worauf die Luciferase-Aktivität der Zellen gemessen wird, wie unten angegeben. Dieses Verfahren wird beliebig oft wiederholt, je nachdem wieviel Einansätze von vornherein eingesetzt wurden. Damit kann über einen Zeitraum von jedenfalls 6 Wochen oder länger verfolgt werden, in welchem Ausmaß die nach A) präparierten Kollagenschwämme zur Transfektion fähig sind, d.h., zur Expression der Luciferase-Aktivität in den Zellen führen.
Luciferasetest:
Bewachsene Kollagenschwämme wurden aus den Gewebekulturschalen entfernt und mit PBS gewaschen, ebenso wurden die Zellen in den Gewebekulturschalen mit PBS gewaschen. Dabei eventuell von den Schwämmen abgewaschene Zellen werden aus der Waschlösung abzentrifugiert und separat auf Luciferaseexpression untersucht. Die dabei gewonnenen Werte werden der Luziferaseexpression am Schwamm zugerechnet. Zellen auf Schwämmen wurden durch Zugabe von 1 ml Lysepuffer lysiert, Zellen in den Vertiefungen durch Zugabe von 500 µl Lysepuffer. Je 10 - 50 µl Zellysat wurden in einer schwarzen 96-Kalotten-Platte mit je 100 µl Luciferin-Substrat-Puffer versetzt. Die Messung der resultierenden Lichtemission erfolgte mittels Microplate Scintillation & Luminescence counter "Top Count" (Fa. Canberra-Packard, Dreieich). Die Meßzeit betrug 12 Sekunden, die Meßverzögerung war 10 min. und Hintergrund-Werte wurden automatisch abgezogen. Als Standard wurden je 100, 50, 25, 12.6, 6.25, 3.13, 1.57, 0.78, 0.39, 0.2, 0.1, 0.05, 0.025, 0.013, 0.007 und 0 ng Luciferase in je 50 µl Lysepuffer (= 2-fache Verdünnungsserie) unter gleichen Bedingungen gemessen und daraus eine Eichkurve erstellt.
Puffer:

(a) Lysepuffer
0.1 % Triton X-100 in 250 mM Tris pH 7.8
Luciferin-Substratpuffer
60 mM Dithiothreitol, 10 mM Magnesiumsulfat, 1 mM ATP, 30 µM D-Luciferin, in 25 mM Glycyl-Glycin-Puffer pH 7.8.

(b) HEPES-buffered saline (HBS)
20 mM HEPES, pH 7.3; 150 mM Natriumchlorid

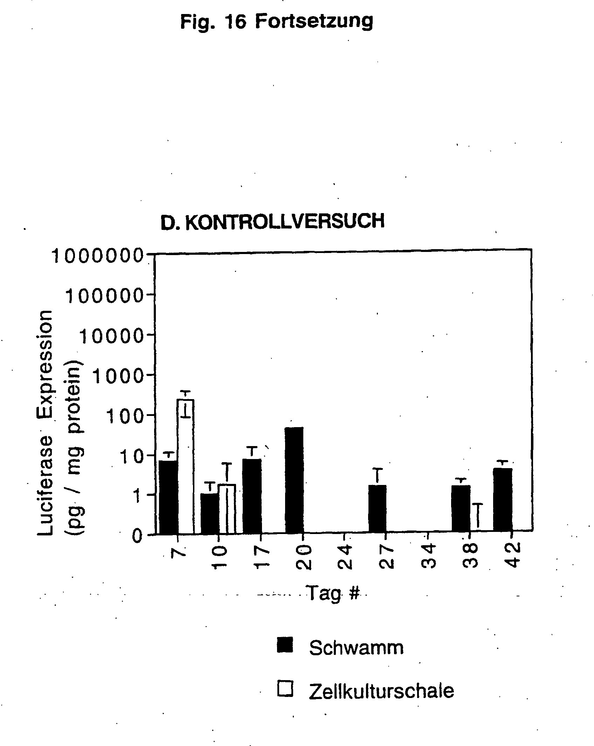

Proteinbestimmung in Zellysaten:
Den Proteingehalt der Lysate bestimmte man mit dem Bio-Rad Protein Assay (Fa. Bio-Rad, München): Zu 10 µl (bzw. 5 µl) des Lysats wurden 150 µl (bzw. 155 µl) dest. Wasser und 40 µl Bio-Rad Protein Assay Farbstoff-Konzentrat in eine Vertiefung einer durchsichtigen 96-Kalotten-Platte (Typ "flat bottom", Fa. Nunc, Dänemark) gegeben und bei 630 nm mit dem Absorbance-Reader "Biolumin 690" und dem Computer-Programm "Xperiment" (beide Fa. Molecular Dynamics, USA) die Absorption gemessen. Als Eichkurve wurden Konzentrationen von 50, 33.3, 22.3, 15, 9.9, 6.6, 4.4, 2.9, 2.0, 1.3, 0.9 und 0 ng BSA / µl vermessen. Bovines Serum Albumin (BSA) wurde als Bio-Rad Protein Assay Standard II gekauft. Somit konnte insgesamt eine Angabe in pg Luciferase pro mg Protein gemacht werden.
Die Ergebnisse dieser in vitro Versuche sind in Fig. 15 dargestellt.
Die Ergebnisse einer Fortführung der Versuche sind in der Figur 16 A für PEI-DNA gezeigt und in der Figur

16 C für nackte DNA. Ein analoges Experiment für Schwämme, die mit einem Copolymer-geschützten Vektor beladen, sind, ist in Figur 16 B gezeigt. Figur 16 D zeigt die Ergebnisse eines Kontrollexperiments. Hierfür wurden NIH3T3-Fibroblasten bei einer Dichte von 450.000 Zellen pro Vertiefung in 6-Loch-Platten am Tage vor der Transfektion (also an Tag 1) ausgelegt. Direkt vor der Transfektion wurde das Medium durch 1.5 ml frisches Medium ersetzt. Die DNA-Komplexe wurden in einem Gesamtvolumen von 500 µl zugegeben (Tag 2) gefolgt von vierstündiger Inkubation und Medienwechsel. An Tag 3 wurde jeweils ein unbehandeltes 1.5 x 1.5 cm grosses Kollagenschwammstück in jede Vertiefung zugegeben. An Tag 6 wurden neue Zellen in frischen 6-Loch-Platten ausgelegt (450.000 Zellen pro Vertiefung). An Tag 7 wurden alle bis auf 3 Schwämme in diese Platten umgesetzt. Drei Schwämme und die Vertiefungen, aus denen sämtliche Schwämme stammten, wurden dem Luciferasetest unterzogen. An Tag 10 wurden sämtliche Schwämme bis auf drei in leere Vertiefungen übersiedelt und in je 2 ml Medium weiter inkubiert. Drei Schwämme und sämtliche Vertiefungen, aus denen die Schwämme übersiedelt wurden, wurden dem Luciferasetest unterzogen. Zu den in Abbildung 16D dargestellten weiteren Zeitpunkten wurden je drei Schwämme und Zellen, die sich am Boden der Vertiefungen abgesetzt hatten, auf Luciferaseexpression analysiert.

Figur 16 D zeigt, dass die Luciferaseexpression anfänglich hoch ist, dann jedoch sehr schnell abnimmt, bzw. nicht mehr messbar ist. Das bedeutet, dass in den anderen Fällen (Fig. 15 und 16 A-C) die signifikant hohe Luciferaseexpression darauf zurückzuführen ist, dass die Zellen kontinuierlich von immobilisierten Vektoren neu transfiziert werden. Es handelt sich also nicht um eine wie immer geartete Selektion von anfänglich transfizierten Zellen am Kollagenschwamm. Wäre dies so, müsste im Kontrollexperiment die Luciferaseexpression ähnlich hoch bleiben, wie in den anderen Versuchen.

Literatur

Balkenhohl, F., von dem Bussche-Hünnefeld, C., Lansky, A. and Zechel, C. (1996). Angew. Chem. 108: 2436-2488.

Barron, L. G., Meyer, K. B. and Szoka, F. C. J. (1998). Hum. Gene Ther. 10(9(3)): 315-23.

Boussif, O., Lezoualch, F., Zanta, M. A., Mergny, M. D., Scherman, D., Demeneix, B., & Behr, J. P. (1995) Proc. Natl. Acad. Sci. USA 92, 7297-7301.

Brocchini, S., James, K., Tangpasuthadol, V. and Kohn, J. (1997). J. Am. Chem. Soc. 119(19): 4553-4554.

Coombes, A. G. A., Tasker, S., Lindblad, M., Holmgren, J., Hoste, K., Toncheva, V., Schacht, E., Davies, M. C., Illum, L. and Davis, S. S. (1997). Biomaterials 18(17): 1153-1161.

Erbacher, P., Remy, J.-S. and Behr, J.-P. (1999). Gene Ther. 6(1):138-45

Erbacher, P., Zou, S. M., Bettinger, T., Steffan, A. M. and Remy, J. S., (1998). Pharm. Res. 15(9): 1332-1339.

Felix, A., M., Heimer, E., P., Lambros, T., J., Tzongraki, C., Meienhofer, J. (1978). J. Org. Chem. 43, 4194-4196.

Ferrari, S., Moro, E., Pettenazzo, A., Behr, J. P., Zacchello, F., & Scarpa, M. (1997) Gene Ther. 4, 1100-1106.

Fields, G., B., Noble, R., L. (1990). Int. J. Peptide Protein Res. 35, 161-214.

Fields, G., B., et al., (1991). Pept. Res. 4, 88.
Gill S.C., von Hippel P.H. *Anal. Biochem.* 1989; 182: 319-326

Gottschalk, S., Sparrow, J. T., Hauer, J., Mims, M. P., Leland, F. E., Woo, S. L. C., & Smith, L. C. (1996) Gene Ther. 3, 448-457.

Haensler, J. and Szoka, F. C. (1993). Bioconj. Chem. 4(5): 372-379.

Kircheis, R., Kichier, A., Wallner, G., Kursa, M., Ogris, M., Felzmann, T., Buchberger, M. and Wagner, E. (1997). Gene Therapy 4(5): 409-418.

Kren, B. T., Bandyopadhyay, P. and Steer, C. J. (1998). Nature Med. 4(3): 285-290.

Lee; R. J. and Huang, L. (1997). Crit. Rev. Ther. Drug Carrier Syst. 1997 14(2)(2): 173-206.

Nathan, A., Bolikal, D., Vyavahare, N., Zalipsky, S., Kohn, J. (1992) Macromolecules 25, 4476-4484.

Nathan, A., Zalipsky, S., Ertel, S. I., Agathos, S. N., Yarmush, M. L., Kohn, J. (1993). Bioconj. Chem. 4, 54-62.

Ogris, M., Brunner, S., Schuller, S., Kircheis, R. and Wagner, E. (1999). Gene Ther. 6(4): 595-605.

Plank, C., Mechtler, K., Szoka, F. C. and Wagner, E. (1996). Hum. Gene Ther. 7(12): 1437-1446.

Plank, C., Oberhauser, B., Mechtler, K., Koch, C., & Wagner, E. (1994) J. Biol. Chem. 269, 12918-12924.

Plank, C., Tang, M., Wolfe, A. and Szoka, F. C. (1999). Hum. Gene Ther. 10(2): 319-333.

Tang, M. X., Redemann, C. T. and Szoka, F. C. (1996). Bioconj. Chem. 7(6): 703-714. Ulbrich, K., Strohalm, J., Kopecek, J. (1985). Macromol. Chem. 187, 1131-1144.

Wadhwa, M. S., Collard, W. T., Adami, R. C., McKenzie, D. L. and Rice, K. G. (1997). Bioconj. Chem. 8(1 ): 81-88.

Whaley, K. E. (1985). Churchill Livingstone, Edinburg, London, Melboume and New York 1985.

Yoon, K., Colestrauss, A. and Kmiec, E. B. (1996). Proc. Natl. Acad. Sci. USA 93(5): 2071-2076.

Zalipsky, S., Gilon, C., Zilkha, A. (1984). J. Makromol. Sci.-Chem. A21, 839-845.

Zanta, M. A., Boussif, O., Adib, A., & Behr, J. P. (1997) Bioconj. Chem. 8, 839-844

Zhou, X. H. and Huang, L. (1994). Biochim. Et Biophys. Acta-Biomembr. 1189(2): 195-203.

**Patentansprüche**

**1.** Kombination aus einem Träger und einem Komplex enthaltend ein oder mehrere Nucleinsäuremoleküle und ein oder mehrere geladene Copolymere der allgemeinen Formel I

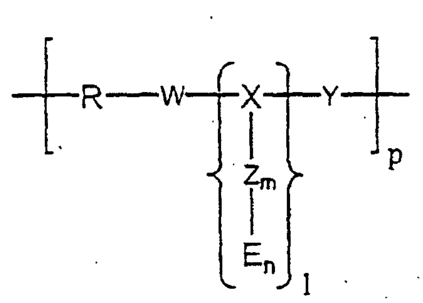

wobei R amphiphiles Polymer oder ein homo- oder hetero-bifunktionelles Derivat davon ist,
und worin X

i) eine Aminosäure oder ein Aminosäurederivat, ein Peptid oder ein Peptidderivat oder ein Spermin- oder Spermidinderivat ist; oder

ii) worin X

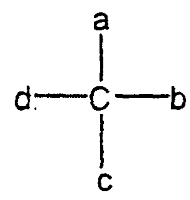

ist,

wobei

a H oder, gegebenenfalls halogen- oder dialkylamino-substituiertes, $C_1$-$C_6$-Alkyl bedeutet;

und wobei

b, c und d gleiches oder verschiedenes, gegebenfalls halogen- oder dialkylamino-substituiertes $C_1$-$C_6$-Alkylen bedeutet;

oder

iii) worin X

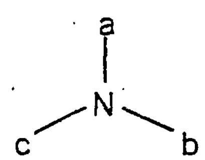

ist,

wobei

a H oder, gegebenenfalls halogen- oder dialkylamino-substituiertes, $C_1$-$C_6$-Alkyl bedeutet, und wobei

b und c gleiches oder verschiedenes, gegebenenfalls halogen- oder dialkylamino-substituiertes $C_1$-$C_6$-Alkylen bedeuten; oder

iv) worin X

eine substituierte aromatische Verbindung mit drei funktionellen Gruppierungen $W_1Y_1Z_1$ ist, wobei W, Y und Z die unten angegebenen Bedeutungen haben;

wobei

W, Y oder Z gleiche oder verschiedene Reste CO, NH, O oder S oder eine zur Reaktion mit SH, OH, NH oder $NH_2$ befähigte Linkergruppierung sind;

und wobei das Effektormolekül E

ein kationisches oder anionisches Peptid oder Peptidderivat oder ein Sperminoder Spermidinderivat oder ein Glykosaminoglycan oder ein nichtpeptidisches Oligo/Poly-kation oder -anion; worin

m und n unabhängig voneinander 0, 1 oder 2 sind; worin

p vorzugsweise 3 bis 20; und worin

l 1 bis 5, vorzugsweise 1 ist.

**2.** Kombination nach Anspruch 1, worin das amphiphile Polymer des Copolymers ein Polyalkylenoxid ist.

**3.** Kombination nach Anspruch 2, worin das amphiphile Polymer des Copolymers ein Polyalkylenglykol ist.

**4.** Kombination nach einem der Ansprüche 1 bis 3, worin X oder E in dem Copolymer ein geladenes Peptid oder Peptidderivat ist.

**5.** Kombination nach einem der Ansprüche 1 bis 4, wobei an das Copolymer ein Ligand für eine höhere eukaryotische Zelle gekoppelt ist.

**6.** Kombination nach einem der Ansprüche 1 bis 5, wobei das (die) Nucleinsäuremolekül(e) kondensiert ist (sind) mit organischen Polykation- oder kationischen Lipidmolekülen, und der so entstandene Komplex an seiner Oberfläche

ein oder mehrere geladene Copolymere der allgemeinen Formel I über ionische Wechselwirkung gebunden hat.

**7.** Kombination nach einem der Ansprüche 1 bis 6, enthaltend ein therapeutisch wirksames Nucleinsäuremolekül.

**8.** Kombination nach einem der Ansprüche 1 bis 7, wobei der Träger aus einem biologisch nicht resorbierbaren Material besteht.

**9.** Kombination nach einem der Ansprüche 1 bis 7, wobei der Träger aus einem biologisch resorbierbaren Material besteht.

**10.** Kombination nach Anspruch 9, wobei das biologisch resorbierbare Material Kollagen ist.

**11.** Kombination nach Anspruch 10, wobei der Träger ein Kollagenschwamm ist.

**12.** Kombination nach einem der Ansprüche 1 bis 7, wobei der Träger ein Träger ist, der durch Quervemetzung eines wie in Anspruch 1 definierten Copolymers erhältlich ist.

**13.** Verwendung einer Kombination nach einem der Ansprüche 1 bis 12 für den Transfer einer Nucleinsäure in Zellen.

**14.** Arzneimittel enthaltend eine Kombination nach einem der Ansprüche 1 bis 12.

**15.** Kit enthaltend einen Träger und ein Copolymer oder einen Komplex wie in Anspruch 1 definiert.

## Claims

**1.** A combination of a carrier and a complex containing one or more nucleic acid molecules and one or more charged copolymers of the general formula I

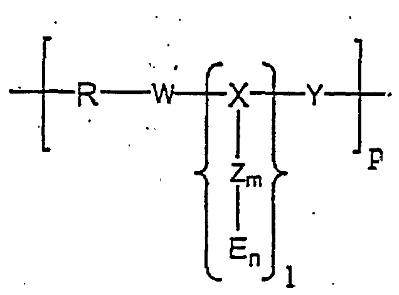

wherein R is an amphiphilic polymer or a homo- or hetero-bifunctional derivative thereof,
and wherein X

i) is an amino acid or an amino acid derivative, a peptide or a peptide derivative or a spermine or spermidine derivative; or

ii) wherein X is

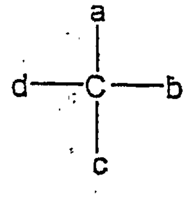

wherein

a is H or, optionally halogen- or dialkylamino-substituted, $C_1$-$C_6$ alkyl;
and wherein
b, c and d are the same or different, optionally halogen- or dialkylamino-substituted, $C_1$-$C_6$ alkylene; or

iii) wherein X is

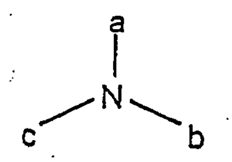

wherein
a is H or, optionally halogen or dialkylamino substituted, $C_1$-$C_6$ alkyl,
and wherein
b and c are the same or different, optionally halogen- or dialkylamino-substituted, $C_1$-$C_6$ alkylene; or

iv) wherein X
is a substituted aromatic compound with three functional groupings $W_1Y_1Z_1$, wherein W, Y and Z have the meanings mentioned below;
wherein
W, Y or Z is the same or different groups CO, NH, O or S or a linker grouping capable of reacting with SH, OH, NH or $NH_2$;
and wherein the effector molecule E
is a cationic or anionic peptide or peptide derivative or a spermine or spermidine derivative or a glycosaminoglycane or a non-peptidic oligo/polycation or -anion; wherein
m and n are independently of each other 0, 1 or 2; wherein
p preferably is 3 to 20; and wherein
1 is 1 to 5, preferably 1.

**2.** The combination according to claim 1, wherein the amphiphilic polymer of the copolymer is a polyalkylene oxide.

**3.** The combination according to claim 2, wherein the amphiphilic polymer of the copolymer is a polyalkylene glycol.

**4.** The combination according to any one of claims 1 to 3, wherein X or E in the copolymer is a charged peptide or peptide derivative.

**5.** The combination according to any one of claims 1 to 4, wherein a ligand for a higher eukaryotic cell is coupled to the copolymer.

**6.** The combination according to any one of claims 1 to 5, wherein the nucleic acid molecule(s) is (are) condensed with organic polycation or cationic lipid molecules and the complex formed thereby has one or more charged copolymers of the general formula I bound to its surface via ionic interaction.

**7.** The combination according to any one of claims 1 to 6, containing a therapeutically effective nucleic acid molecule.

**8.** The combination according to any one of claims 1 to 7, wherein the carrier consists of a biologically non-resorbable material.

**9.** The combination according to any one of claims 1 to 7, wherein the carrier consists of a biologically resorbable material.

**10.** The combination according to claim 9, wherein the biologically resorbable material is collagen.

**11.** The combination according to claim 10, wherein the carrier is a collagen sponge.

12. The combination according to any one of claims 1 to 7, wherein the carrier is a carrier which is obtainable by cross-linking a copolymer as defined in claim 1.

13. Use of a combination according to any one of claims 1 to 12 for the transfer of a nucleic acid into cells.

14. A pharmaceutical composition containing a combination according to any one of claims 1 to 12.

15. A kit containing a carrier and a copolymer or a complex as defined in claim 1.

## Revendications

1. Combinaison formée d'un support et d'un complexe comprenant une ou plusieurs molécules d'acide nucléique et un ou plusieurs copolymères chargés de formule générale I

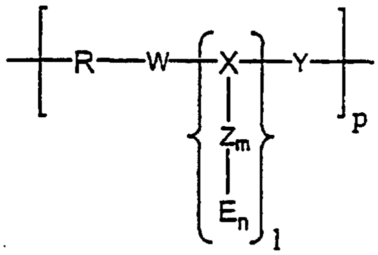

dans laquelle R est un polymère amphiphile ou un dérivé homo- ou hétéro-bifonctionnel de celui-ci,
et dans laquelle X

i) est un acide aminé ou un dérivé d'acide aminé, un peptide ou un dérivé de peptide ou la spermine ou un dérivé de la spermidine ; ou
ii) dans laquelle X est,

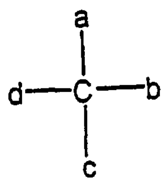

où
a représente H, ou un alkyle en $C_1$-$C_6$ éventuellement substitué par un halogène ou un dialkylamino ; et où
b, c et d identiques ou différents, représente un alkylène en $C_1$-$C_6$ éventuellement substitué par un halogène ou un dialkylamino ; ou
iii) dans laquelle X est

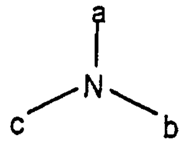

où
a représente H ou un alkyle en $C_1$-$C_6$ éventuellement substitué par un halogène ou un dialkylamino, et où
b et c identiques ou différents représentent un alkylène en $C_1$-$C_6$ éventuellement substitué par un halogène ou un dialkylamino ; ou
iv) dans laquelle X est un composé aromatique substitué avec trois groupements fonctionnels $W_1Y_1Z_1$, où W, Y et Z ont les significations mentionnées ci-dessous ;

où
W, Y ou Z identiques ou différents sont des restes CO, NH, O ou S ou un groupe de liaisons approprié pour réagir avec SH, OH, NH ou $NH_2$ ;
et dans laquelle la molécule effectrice E
est un peptide ou dérivé de peptide cationique ou anionique ou la spermine ou un dérivé de la spermidine ou un glycosaminoglycane ou un oligo/poly-cation ou -anion non peptidique ; où
m et n sont indépendamment l'un de l'autre 0, 1 ou 2 ; où
p est de préférence 3 à 20 ; et où
I est de 1 à 5, de préférence 1.

**2.** Combinaison selon la revendication 1, dans laquelle le polymère amphiphile du copolymère est un polyalkylènoxyde.

**3.** Combinaison selon la revendication 2, dans laquelle le polymère amphiphile du copolymère est un polyalkylèneglycol.

**4.** Combinaison selon l'une quelconque des revendications 1 à 3, dans laquelle X ou E dans le copolymère est un peptide ou dérivé de peptide chargé.

**5.** Combinaison selon l'une quelconque des revendications 1 à 4, dans laquelle un ligand pour une cellule eucaryotique supérieure est couplé au copolymère.

**6.** Combinaison selon l'une quelconque des revendications 1 à 5, dans laquelle la (les) molécule(s) d'acide nucléique est (sont) condensée(s) avec une molécule lipidique polycationique ou cationique organique, et le complexe ainsi obtenu a lié à sa surface un ou plusieurs copolymères chargés de formule générale I par effet d'échange ionique.

**7.** Combinaison selon l'une quelconque des revendications 1 à 6, comprenant une molécule d'acide nucléique à activité thérapeutique.

**8.** Combinaison selon l'une quelconque des revendications 1 à 7, dans laquelle le support consiste en un matériau biologique non résorbable.

**9.** Combinaison selon l'une quelconque des revendications 1 à 7, dans laquelle le support consiste en un matériau biologique résorbable.

**10.** Combinaison selon la revendication 9, dans laquelle le matériau biologique résorbable est du collagène.

**11.** Combinaison selon la revendication 10, dans laquelle le support est une éponge de collagène.

**12.** Combinaison selon l'une quelconque des revendications 1 à 7, dans laquelle le support est un support, qui peut être obtenu par réticulation croisée d'un copolymère tel que défini à la revendication 1.

**13.** Utilisation d'une combinaison selon l'une quelconque des revendications 1 à 12 pour le transfert d'un acide nucléique dans des cellules.

**14.** Médicament contenant une combinaison selon l'une quelconque des revendications 1 à 12.

**15.** Kit comprenant un support et un copolymère ou un complexe tel que défini à la revendication 1.

**Fig. 1**

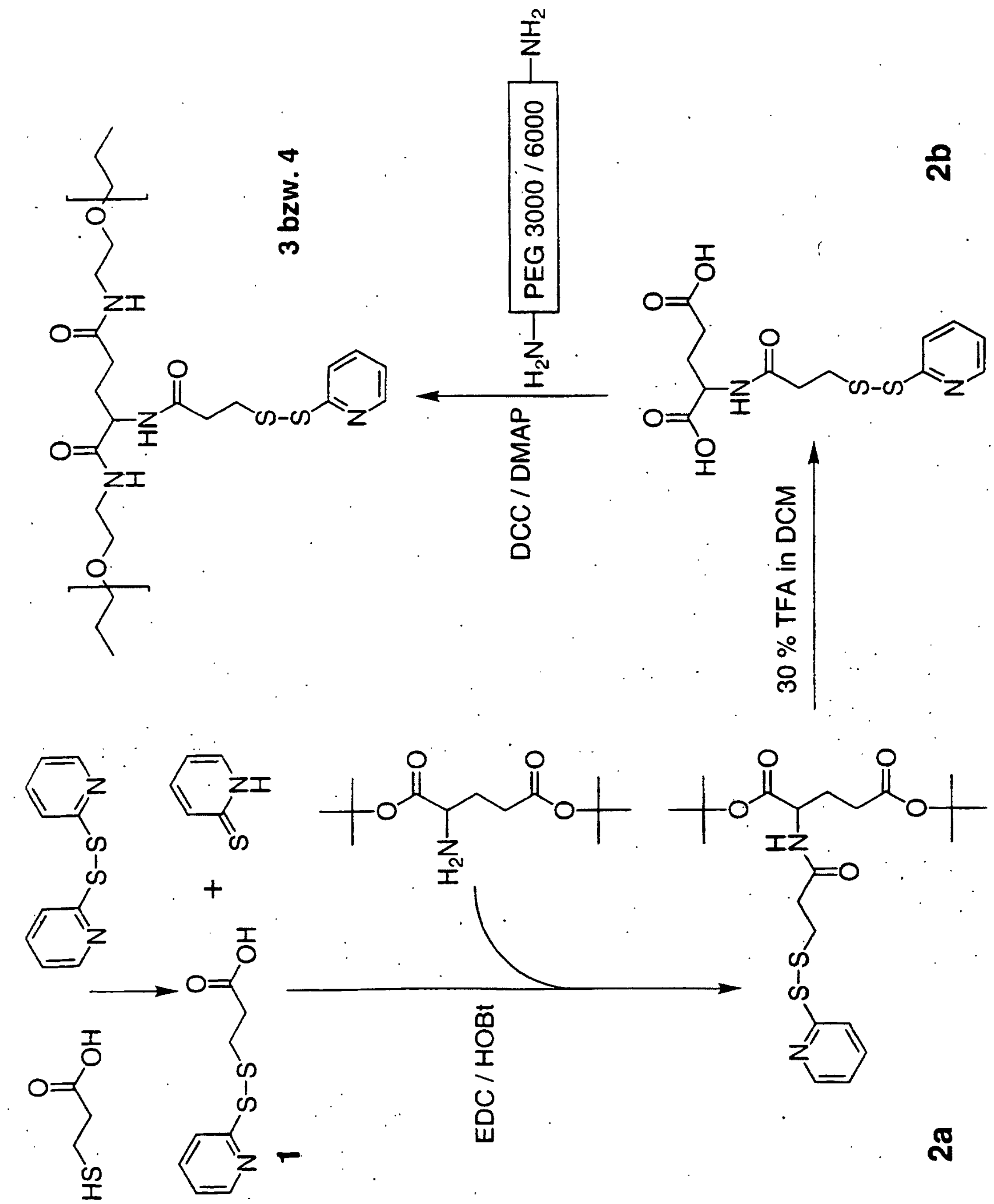


3 bzw. 4

2b

1

2a

**Fig. 2**

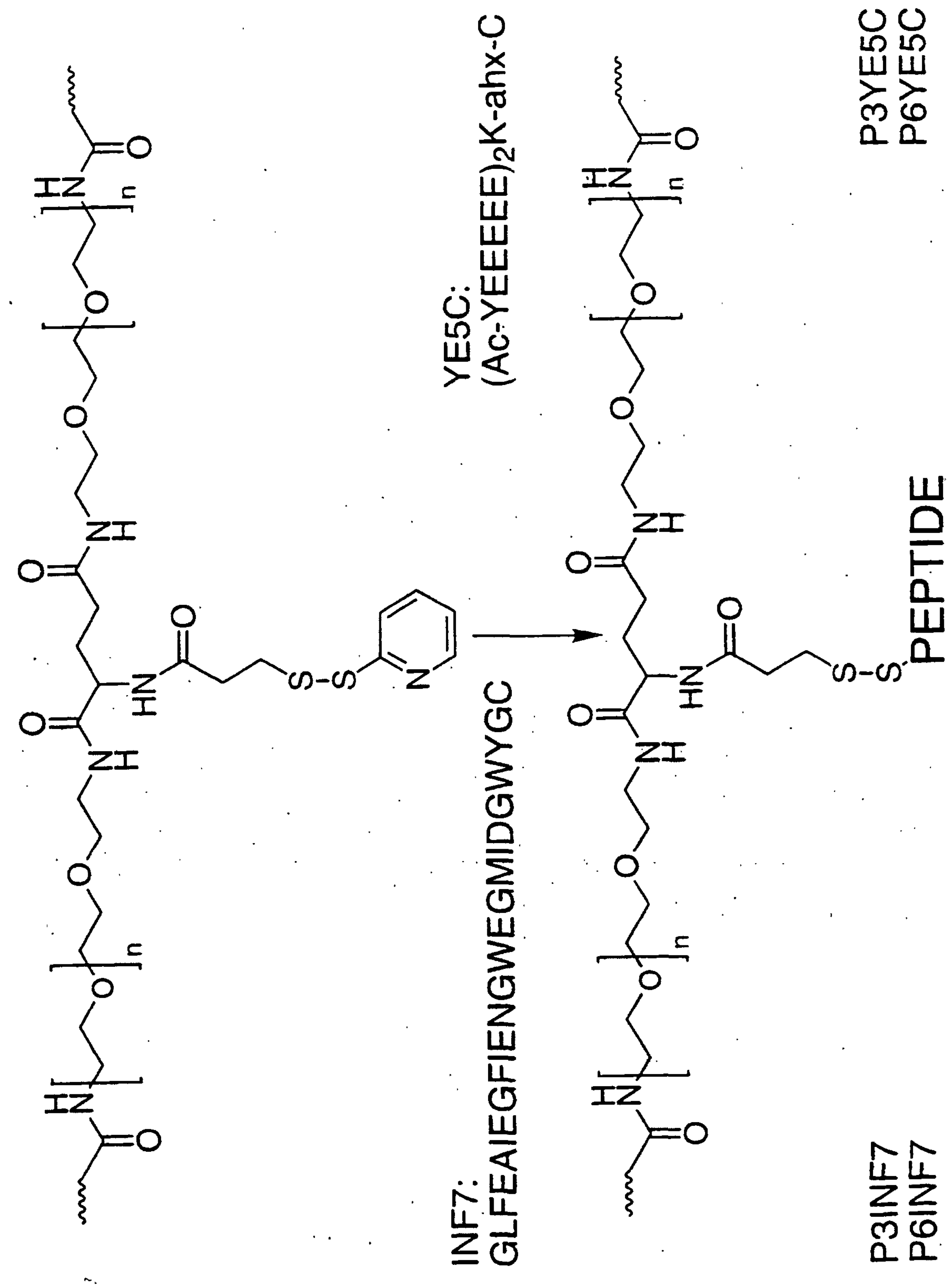
YE5C:
(Ac-YEEEEE)$_2$K-ahx-C
INF7:
GLFEAIEGFIENGWEGMIDGWYGC
PEPTIDE
P3YE5C
P6YE5C
P3INF7
P6INF7

# Fig. 3

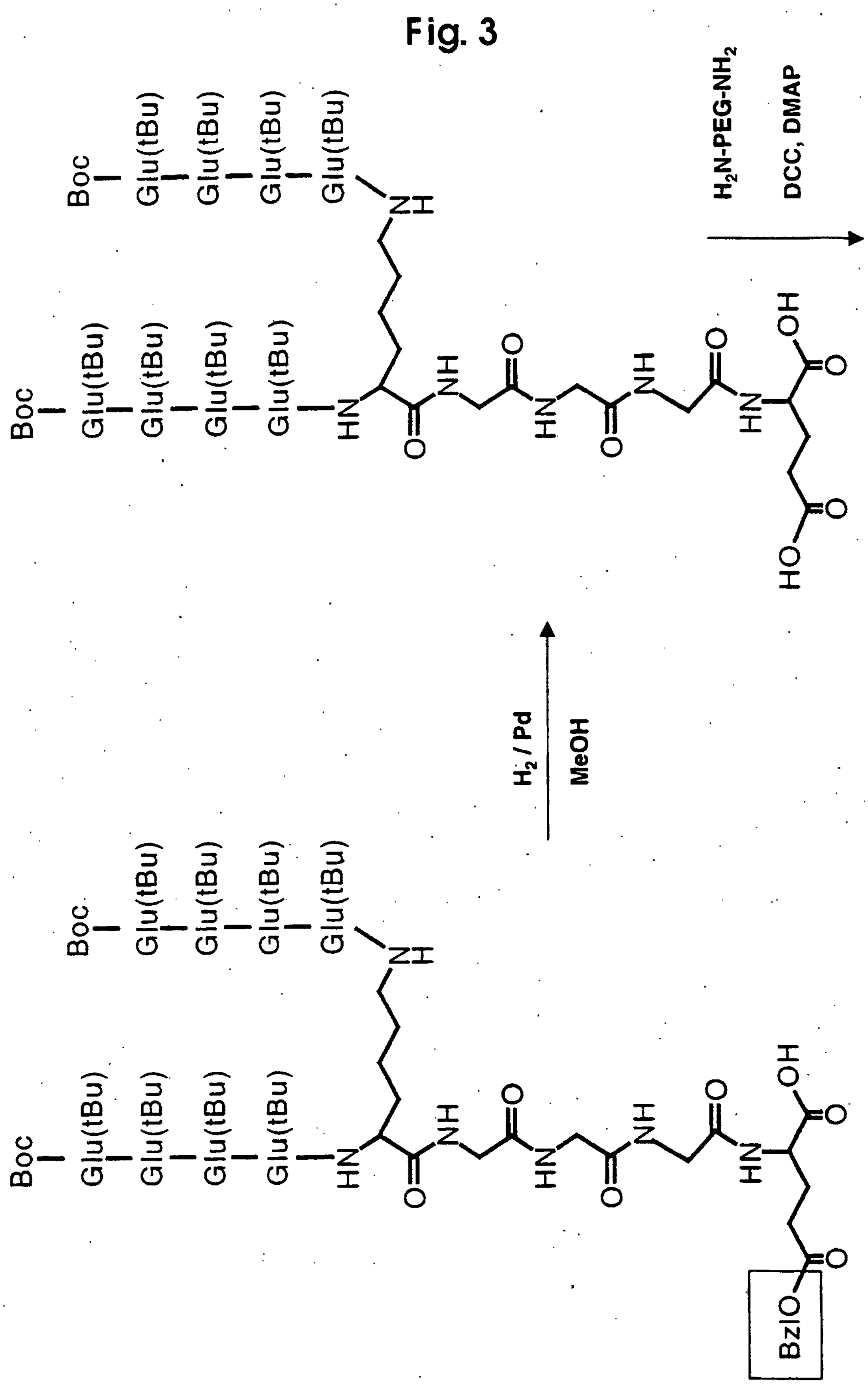

Fig. 3 Fortsetzung

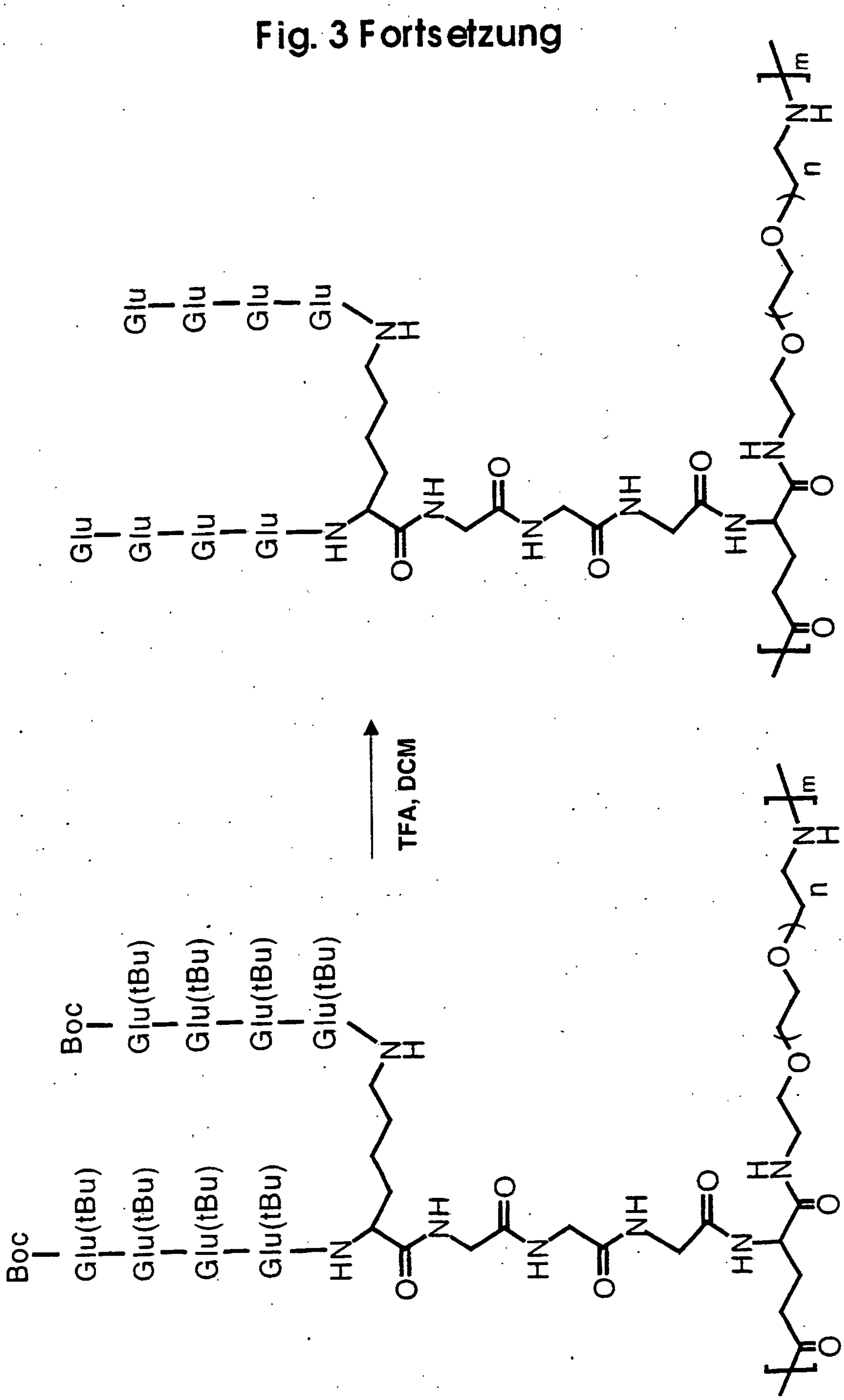

Fig. 4/1

A

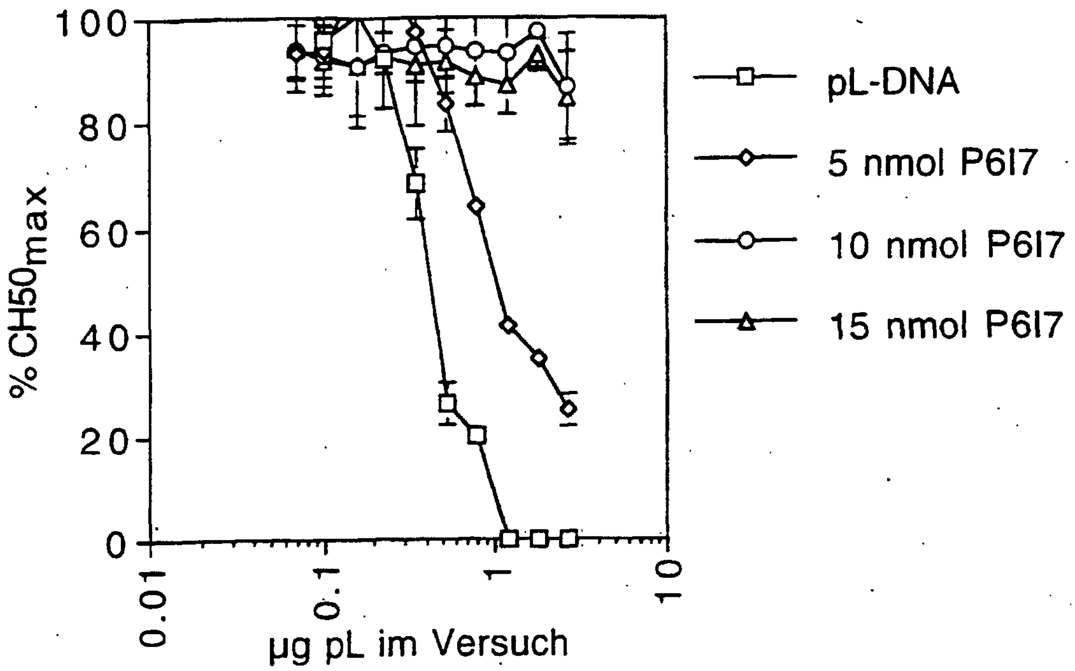

100
80
60
40
20
0
% CH50max
0.01
0.1
1
10
µg pL im Versuch
pL-DNA
5 nmol P6I7
10 nmol P6I7
15 nmol P6I7


B

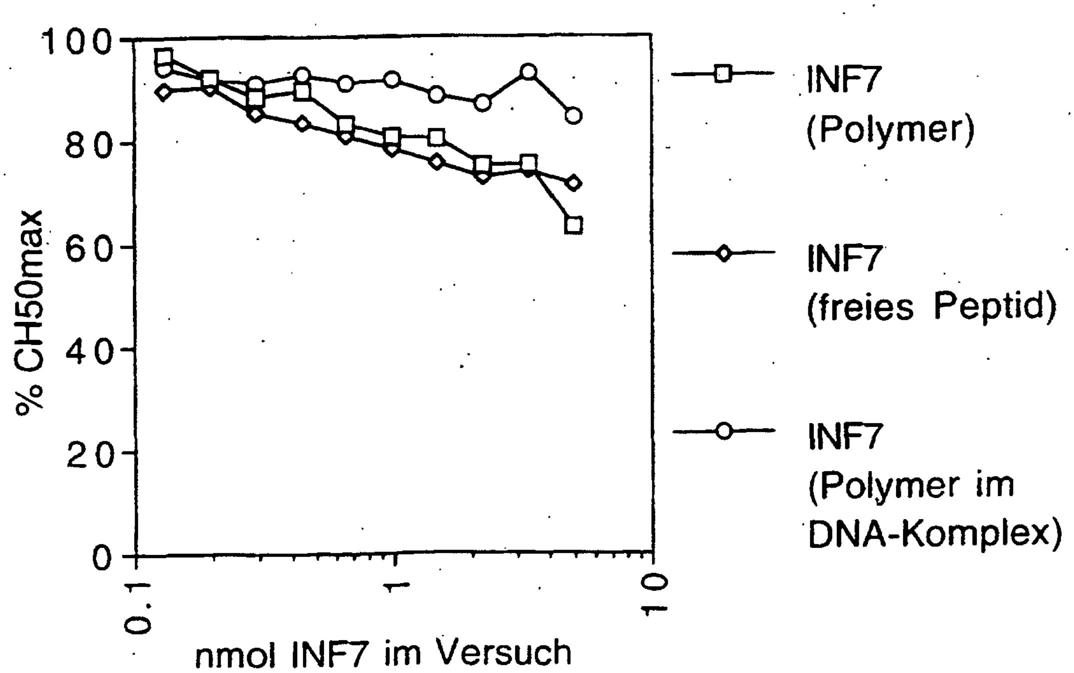

100
80
60
40
20
0
% CH50max
0.1
1
10
nmol INF7 im Versuch
INF7 (Polymer)
INF7 (freies Peptid)
INF7 (Polymer im DNA-Komplex)

Fig. 4/2

C

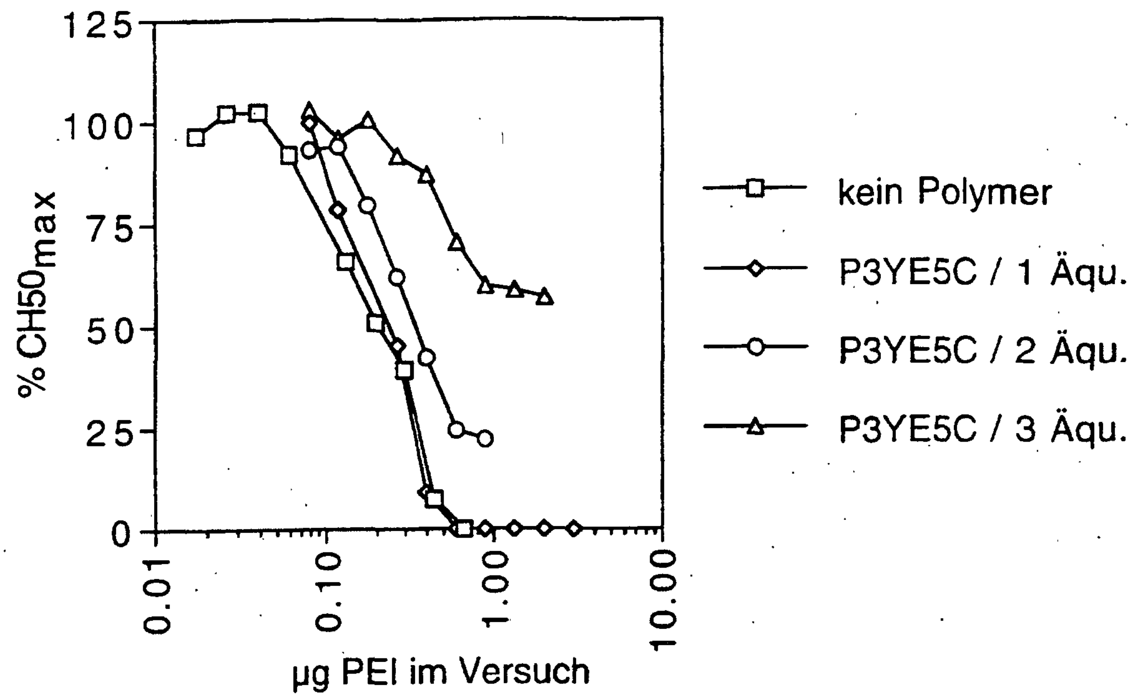
125
100
75
50
25
0
% CH50max
0.01
0.10
1.00
10.00
µg PEI im Versuch
kein Polymer
P3YE5C / 1 Äqu.
P3YE5C / 2 Äqu.
P3YE5C / 3 Äqu.

D

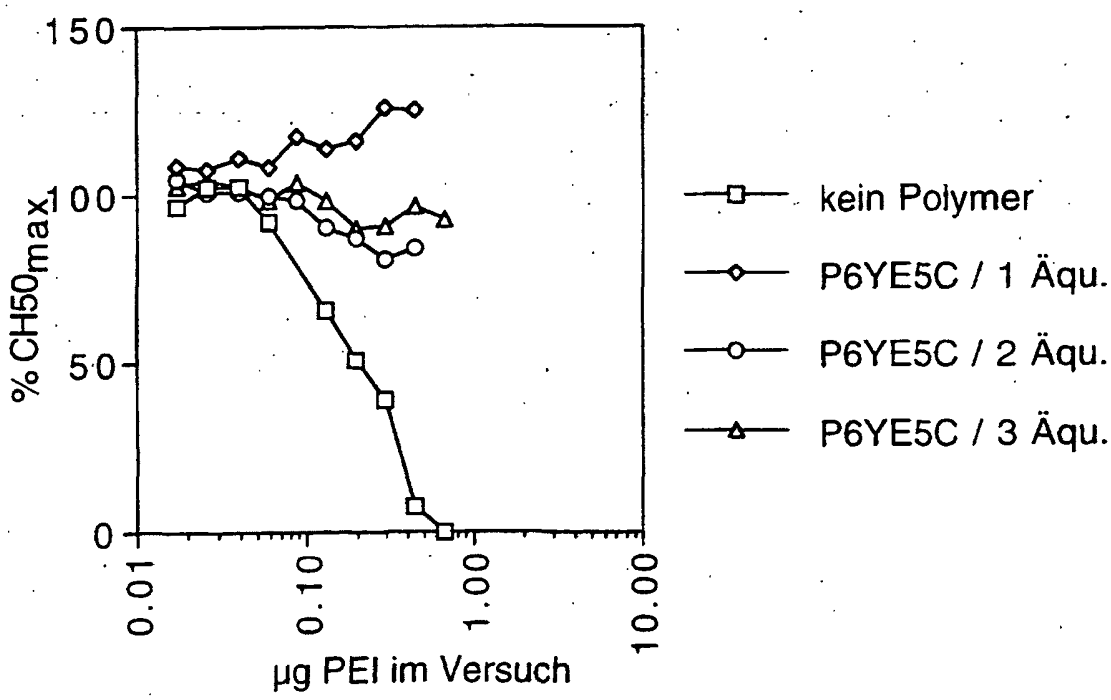
150
100
50
0
% CH50max
0.01
0.10
1.00
10.00
µg PEI im Versuch
kein Polymer
P6YE5C / 1 Äqu.
P6YE5C / 2 Äqu.
P6YE5C / 3 Äqu.

Fig. 5

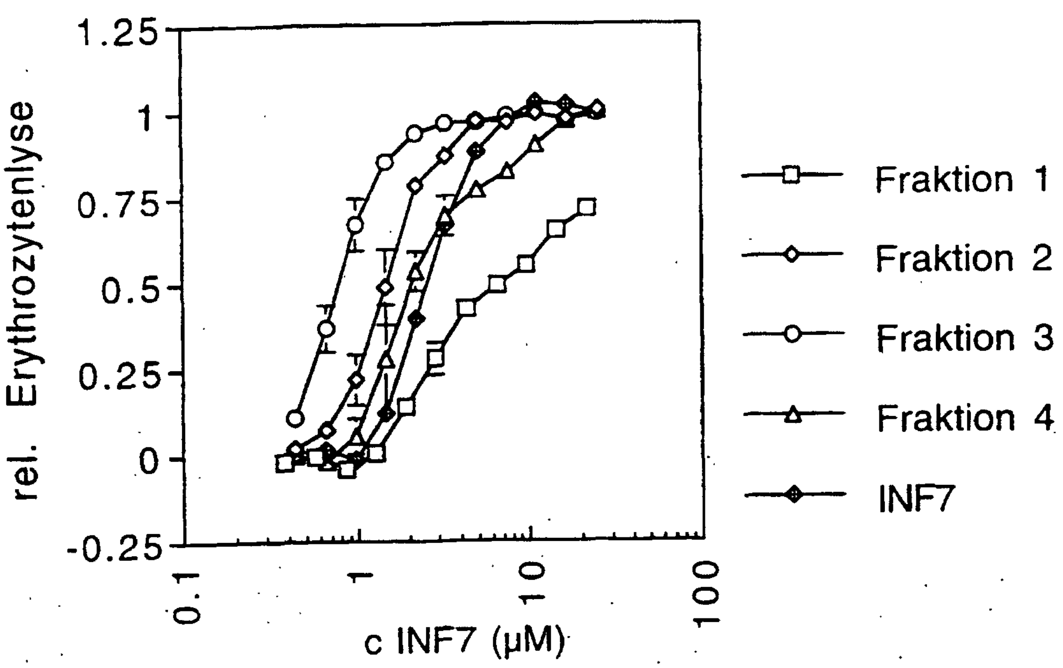
1.25
1
0.75
0.5
0.25
0
-0.25
rel. Erythrozytenlyse
0.1
1
10
100
c INF7 (µM)
Fraktion 1
Fraktion 2
Fraktion 3
Fraktion 4
INF7

Fig. 6c

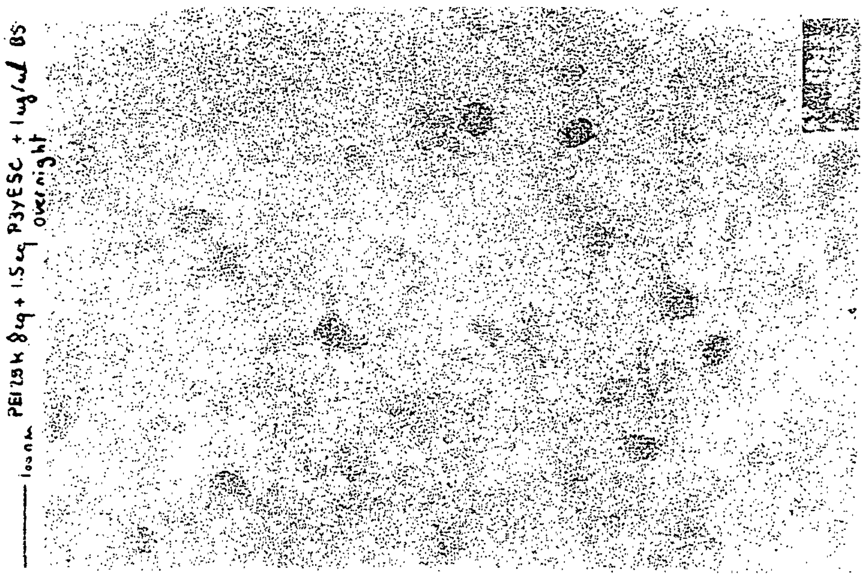


Fig. 6b

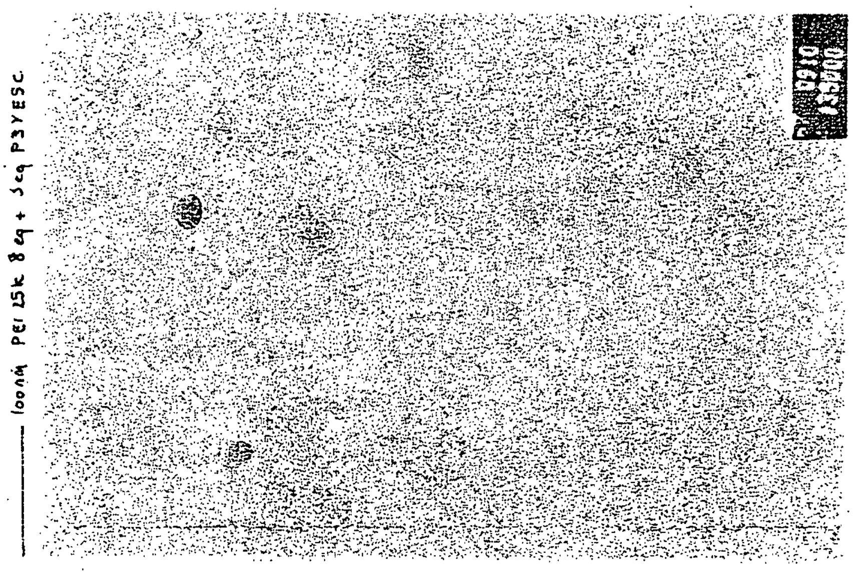


Fig. 6a

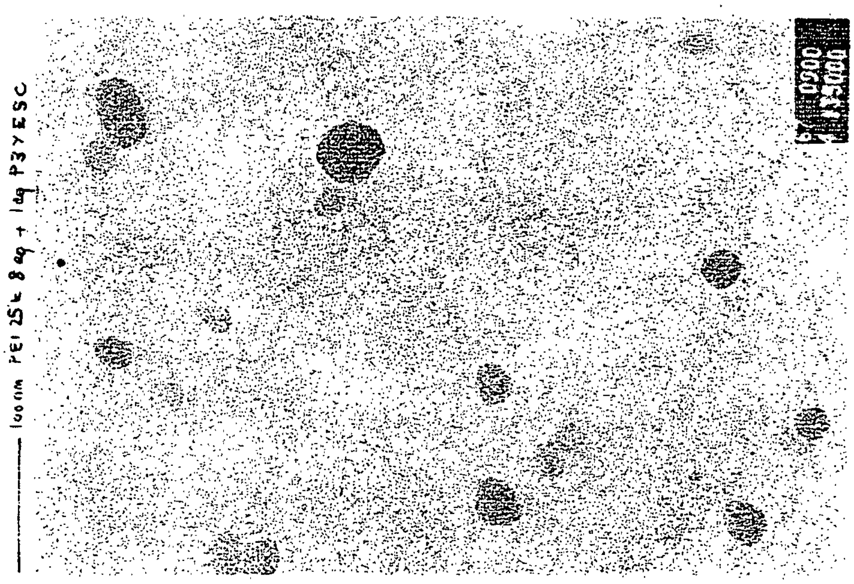

Fig. 7

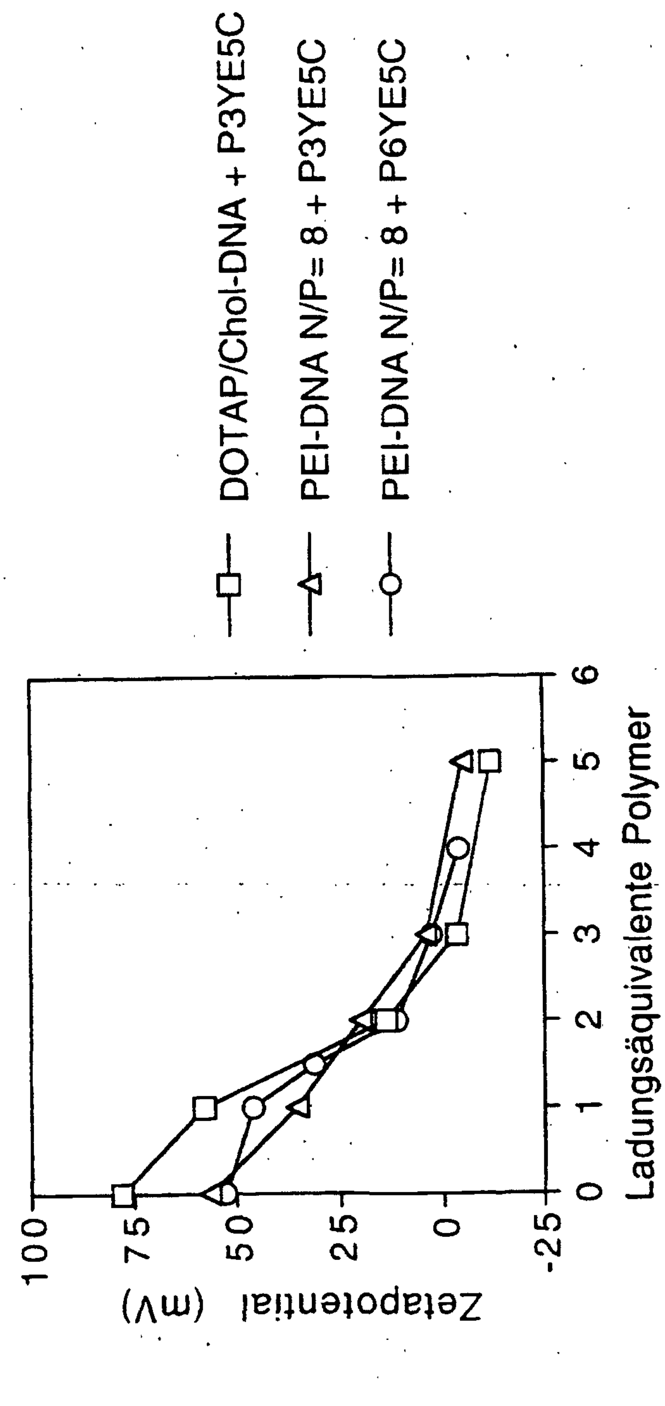
DOTAP/Chol-DNA + P3YE5C
PEI-DNA N/P= 8 + P3YE5C
PEI-DNA N/P= 8 + P6YE5C
Zetapotential (mV)
100
75
50
25
0
-25
0
1
2
3
4
5
6
Ladungsäquivalente Polymer

**Fig. 8**

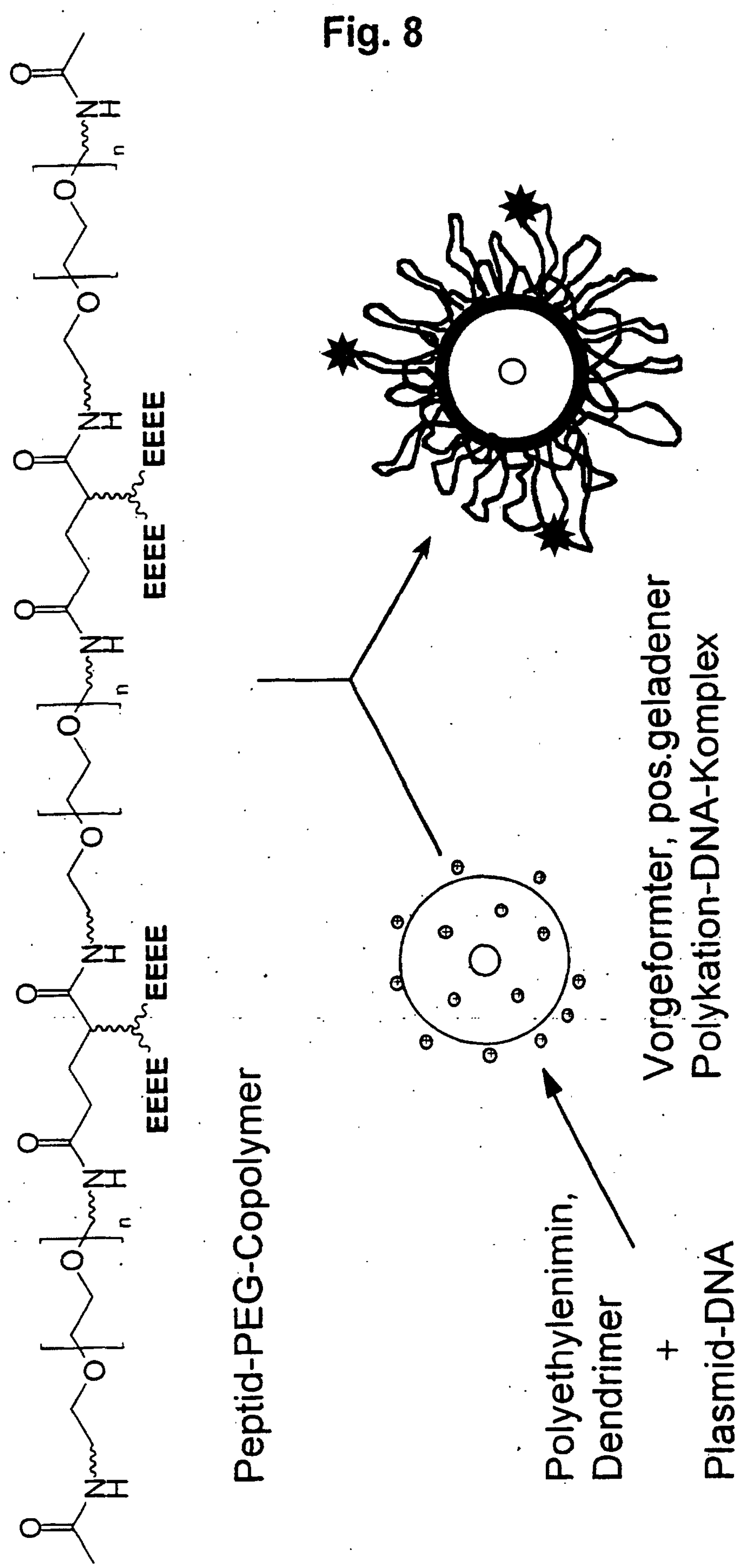
Peptid-PEG-Copolymer
Polyethylenimin, Dendrimer + Plasmid-DNA
Vorgeformter, pos.geladener Polykation-DNA-Komplex

Fig. 9

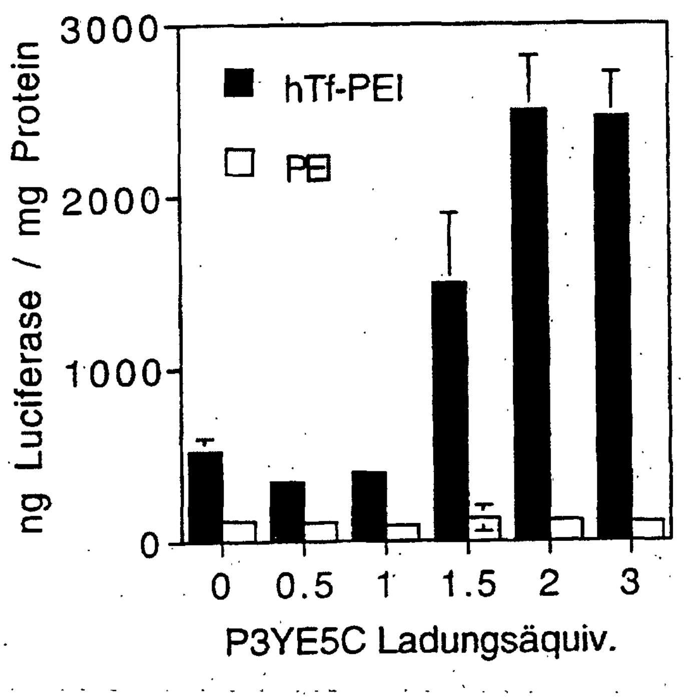
3000
2000
1000
0
ng Luciferase / mg Protein
hTf-PEI
PEI
0
0.5
1
1.5
2
3
P3YE5C Ladungsäquiv.

Fig. 10

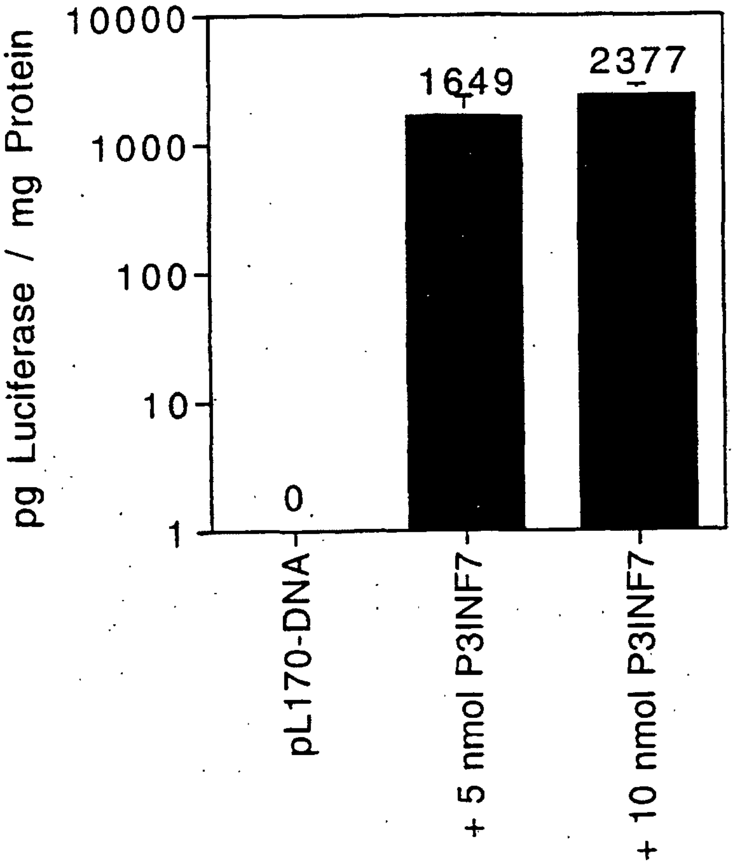
pg Luciferase / mg Protein
10000
1000
100
10
1
0
1649
2377
pL170-DNA
+ 5 nmol P3INF7
+ 10 nmol P3INF7

Fig. 11

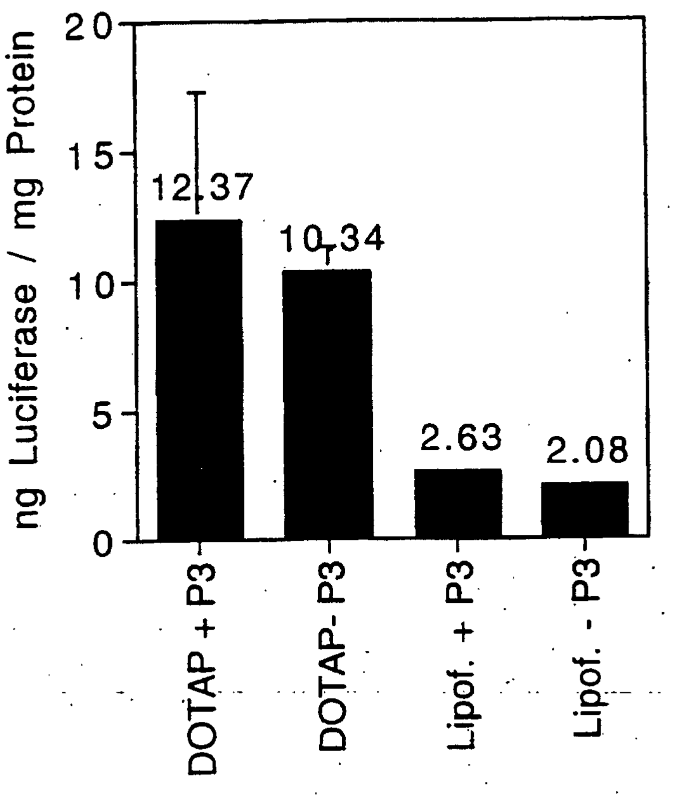
ng Luciferase / mg Protein
20
15
10
5
0
12.37
10.34
2.63
2.08
DOTAP + P3
DOTAP- P3
Lipof. + P3
Lipof. - P3

Fig. 12

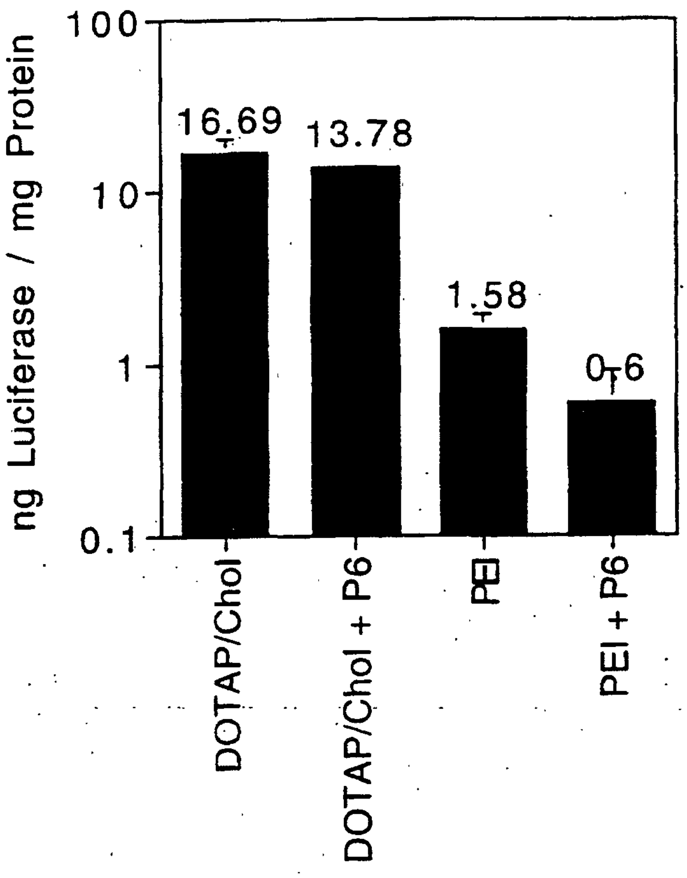
100
10
1
0.1
ng Luciferase / mg Protein
16.69
13.78
1.58
0.6
DOTAP/Chol
DOTAP/Chol + P6
PEI
PEI + P6

Fig. 13

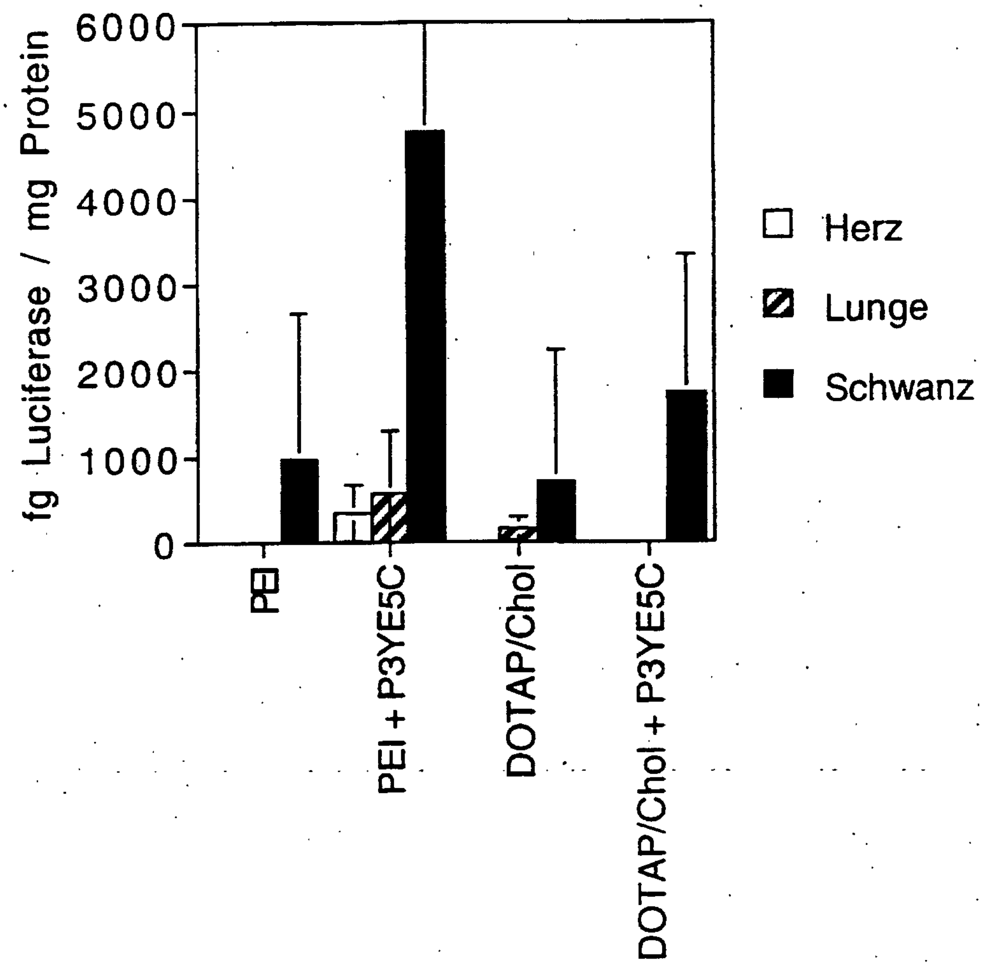
fg Luciferase / mg Protein
6000
5000
4000
3000
2000
1000
0
PEI
PEI + P3YE5C
DOTAP/Chol
DOTAP/Chol + P3YE5C
Herz
Lunge
Schwanz

Fig. 14A

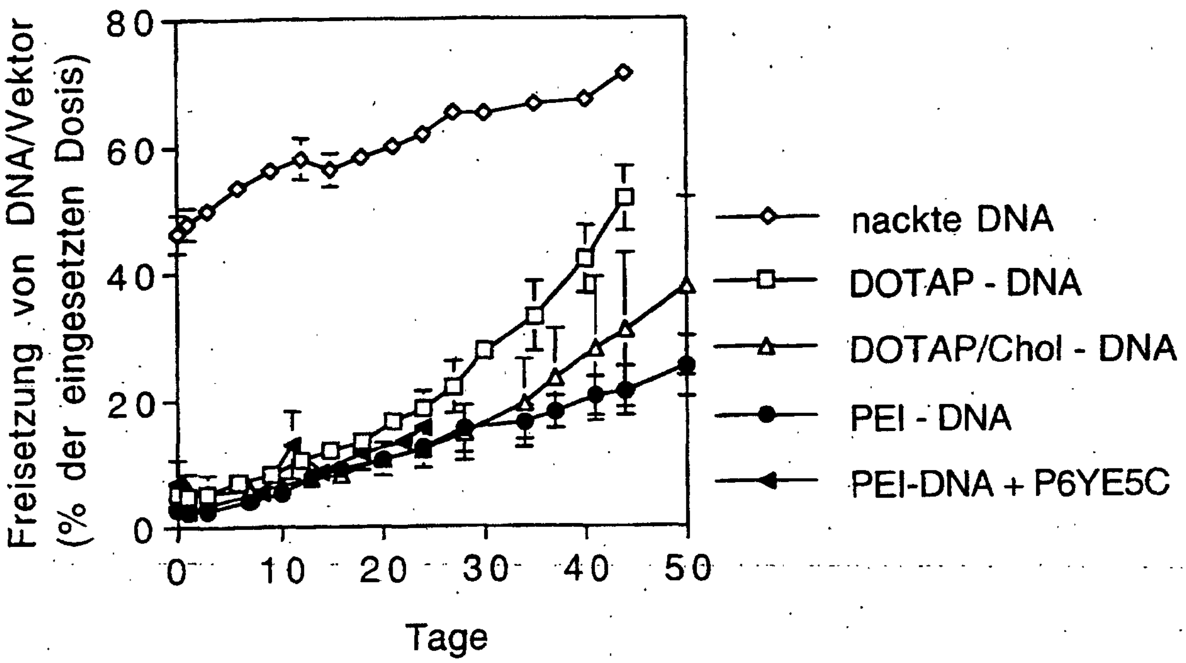
Freisetzung von DNA/Vektor
(% der eingesetzten Dosis)
80
60
40
20
0
0
10
20
30
40
50
Tage
nackte DNA
DOTAP - DNA
DOTAP/Chol - DNA
PEI - DNA
PEI-DNA + P6YE5C

**Fig. 14 Fortsetzung**

B

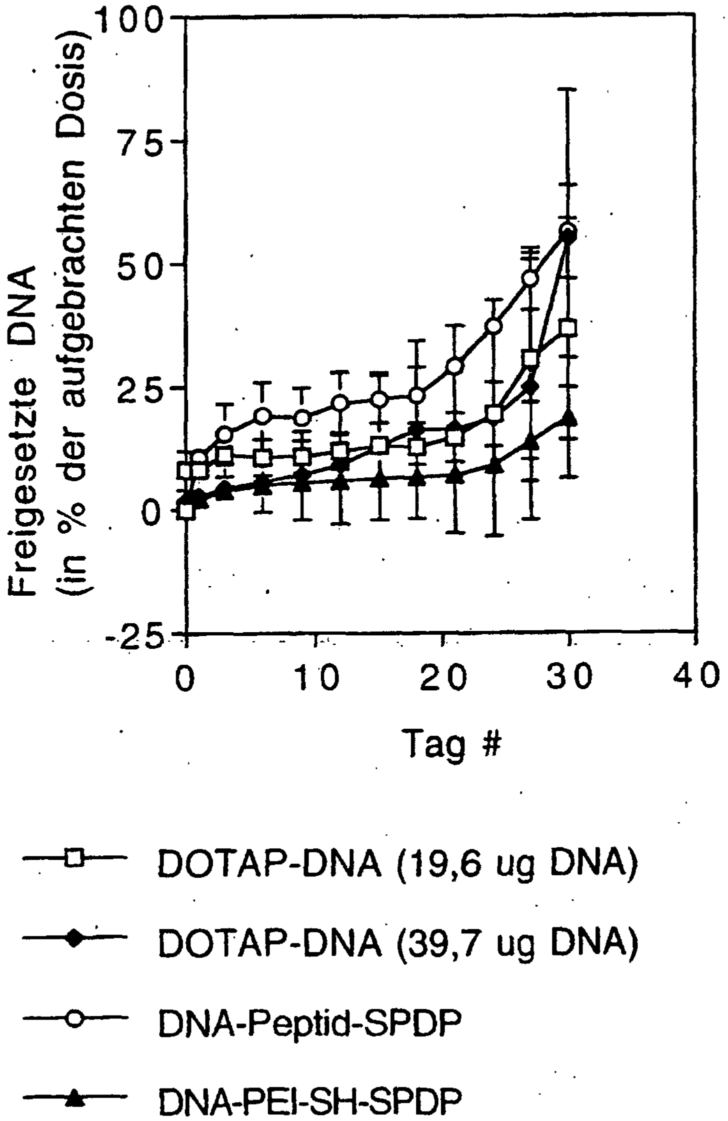

Fig. 15

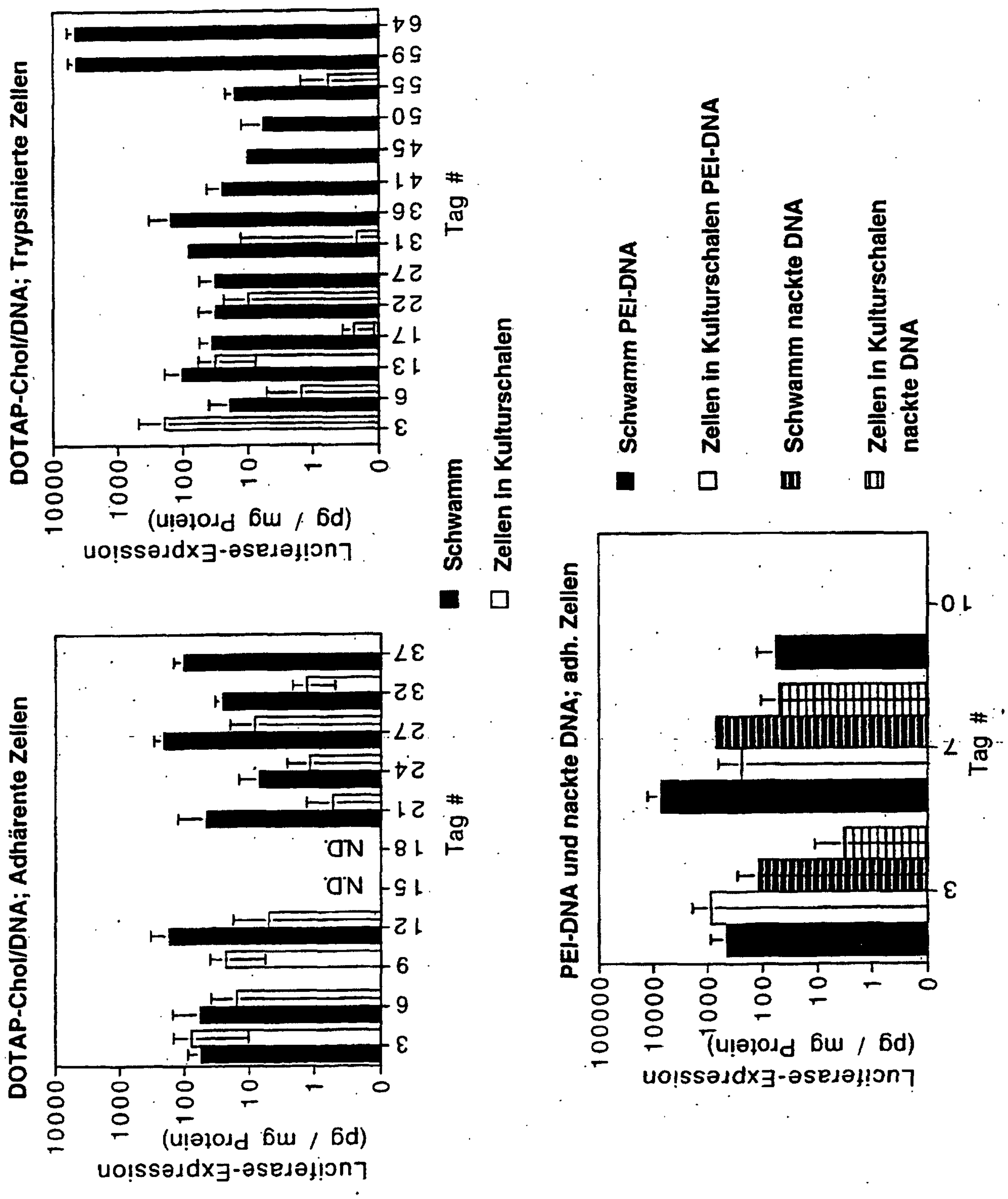
DOTAP-Chol/DNA; Adhärente Zellen
DOTAP-Chol/DNA; Trypsinierte Zellen
PEI-DNA und nackte DNA; adh. Zellen
Luciferase-Expression (pg / mg Protein)
Tag #
N.D.
Schwamm
Zellen in Kulturschalen
Schwamm PEI-DNA
Zellen in Kulturschalen PEI-DNA
Schwamm nackte DNA
Zellen in Kulturschalen nackte DNA

Fig. 16

A. PEI-DNA; N/P = 8

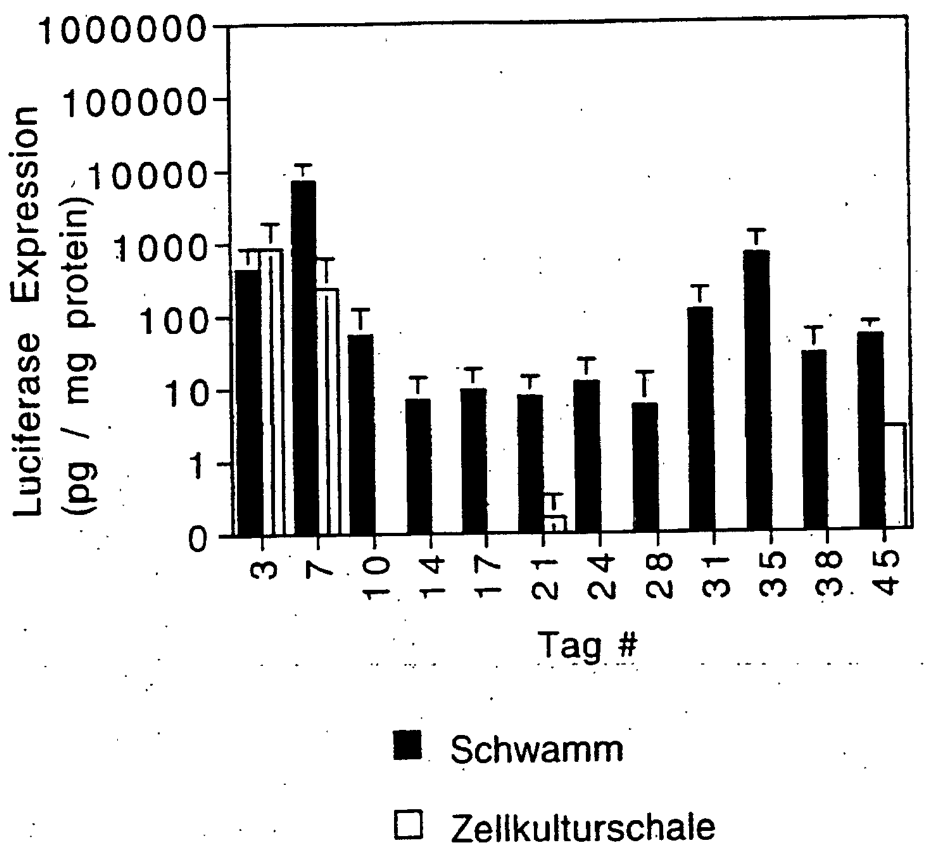

Fig. 16 Fortsetzung

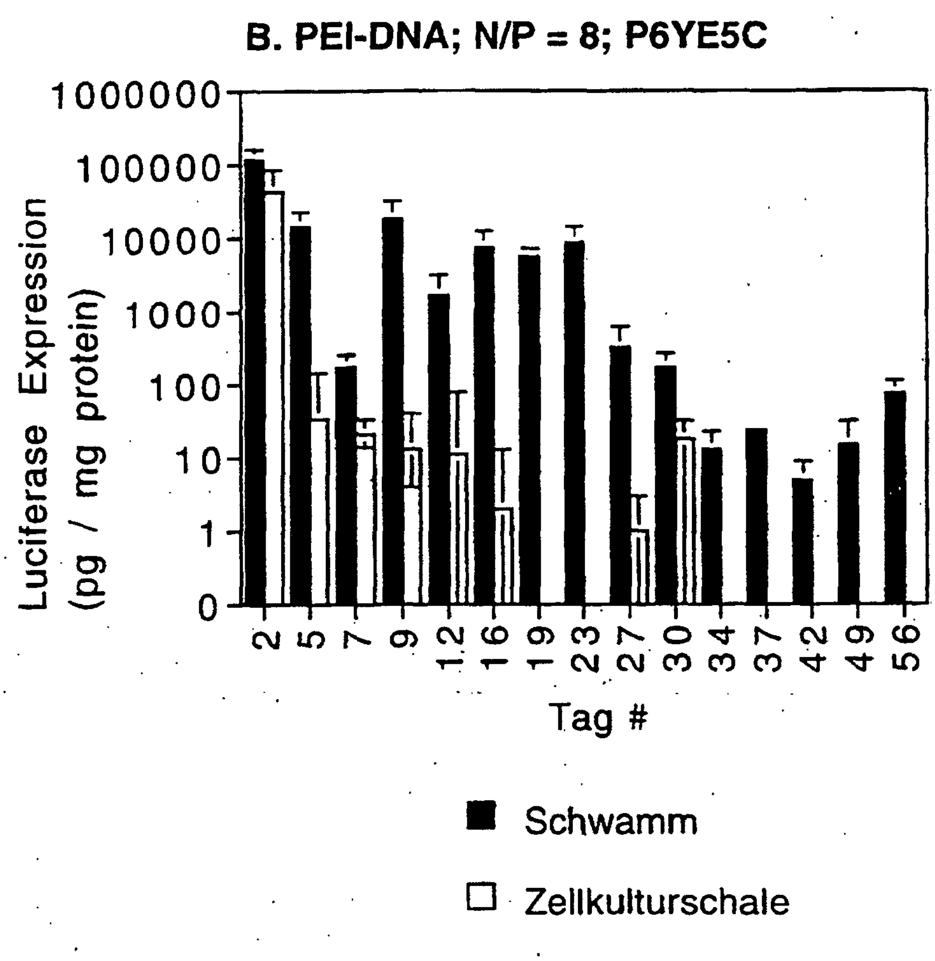

Fig. 16 Fortsetzung

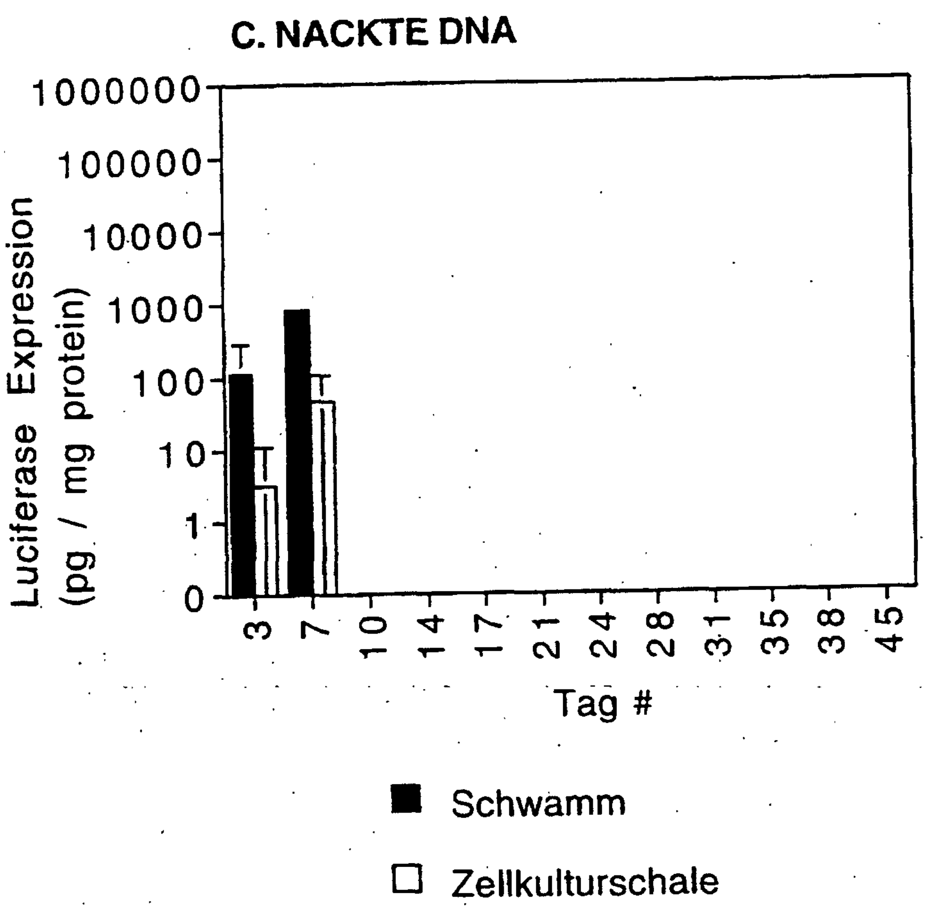

## Fig. 16 Fortsetzung